# EUROPEAN PATENT SPECIFICATION

(11) **EP 4 472 505 B1**
(45) Date of publication and mention of the grant of the patent: **05.08.2026**
(21) Application number: 23710508.5
(22) Date of filing: 06.02.2023
(51) Int. Cl.: A61B 5/145

(54) **SYSTEM AND METHOD FOR AN ANALYTE SENSOR**
SYSTEM UND VERFAHREN FÜR EINEN ANALYTSENSOR
SYSTÈME ET PROCÉDÉ ASSOCIÉS À UN CAPTEUR D'ANALYTE

(30) Priority: 04.02.2022 US 202263306872 P
(43) Date of publication of application: 11.12.2024
(73) Proprietor: Abbott Diabetes Care Inc., Alameda, CA 94502 (US)
(72) Inventor: VOIT, Peter, M., Dublin, CA 94568 (US); COLE, Jean-Pierre, Tracy, CA 95304 (US); MEYER, Thomas, Fremont, CA 94539 (US); LOVE, Michael, Pleasanton, CA 94566 (US)
(74) Representative: Mathys & Squire
(86) International application number: PCT/US2023/012441
(87) International publication number: WO 2023/150363

(56) References cited:
- WO-A1-2016/133841
- CN-U- 209 218 442
- US-A1- 2003 100 821
- US-A1- 2012 018 302
- US-A1- 2018 130 683
- US-A1- 2020 029 902
- US-A1- 2021 177 315

## Description

### FIELD

The subject matter described herein relates generally to systems, devices, and methods for analyte sensors. For example, methods for assembling a sensor subassembly, an on-body sensor puck assembly, and an applicator assembly are disclosed. A sensor including a in vivo portion, an ex vivo portion, and a neck that interconnects the in vivo portion and the ex vivo portion and methods of configuring a sensor are also disclosed. A sensor including electronic components mounted directly thereon is also disclosed.

### BACKGROUND

The detection and/or monitoring of analyte levels, such as glucose, ketones, lactate, oxygen, hemoglobin AIC, or the like, can be vitally important to the health of an individual having diabetes. Patients suffering from diabetes mellitus can experience complications including loss of consciousness, cardiovascular disease, retinopathy, neuropathy, and nephropathy. Diabetics are generally required to monitor their glucose levels to ensure that they are being maintained within a clinically safe range, and may also use this information to determine if and/or when insulin is needed to reduce glucose levels in their bodies, or when additional glucose is needed to raise the level of glucose in their bodies.

Growing clinical data demonstrates a strong correlation between the frequency of glucose monitoring and glycemic control. Despite such correlation, however, many individuals diagnosed with a diabetic condition do not monitor their glucose levels as frequently as they should due to a combination of factors including convenience, testing discretion, pain associated with glucose testing, and cost.

To increase patient adherence to a plan of frequent glucose monitoring, in vivo analyte monitoring systems can be utilized, in which a sensor control device may be worn on the body of an individual who requires analyte monitoring. To increase comfort and convenience for the individual, the sensor control device may have a small form-factor, and can be assembled and applied by the individual with a sensor applicator. The application process includes inserting a sensor, such as a dermal sensor that senses a user's analyte level in a bodily fluid located in the dermal layer of the human body, using an applicator or insertion mechanism, such that the sensor comes into contact with a bodily fluid. The sensor control device may also be configured to transmit analyte data to another device, from which the individual or her health care provider ("HCP") can review the data and make therapy decisions.

While current sensors can be convenient for users, they can be made more comfortable, convenient, and portable by further reducing the size of the on-body unit. Furthermore, by reducing the size of the on-body unit, and/or by reducing the number of internal components, the manufacturing cost of the on-body unit can be reduced. Lower manufacturing costs can be one means of reducing replacement costs for a patient, since the on-body unit can be a disposable, one-time use unit which needs regular replacement. One limit to such miniaturization is the need for a sensor substrate for the locating electrodes for sensing analyte concentration and separate substrate for locating electronic components for providing electrical power, processing sensor data, and transmitting sensor data to a remote device. However, previous manufacturing technologies prevent such components from being mounted directly to the sensor substrate.

Thus, a need exists for a continuous analyte monitoring system which has a reduced size and is economical to manufacture.

WO2016/133841A1 describes an electrochemical sensor of a flexible, body-mountable analyte sensing device. The electrochemical sensor is disposed on a flexible sensor probe that is configured to penetrate the skin such that the electrochemical sensor disposed on the sensor probe can detect an analyte in interstitial fluid. The electrochemical sensor is made sensitive to the analyte by disposing a substance that selectively binds to reacts with, catalyzes a reaction of, or otherwise selectively interacts with the analyte. The substance is localized by crosslinking on the surface of an electrode and/or by being disposed in a polymer layer disposed on the electrode. The polymer layer can be a hydrogel. Further, a hydrogel layer can be formed on the sensor probe to protect elements of the sensor probe and to increase the biocompatibility of the sensor probe.

US2003/100821A1 describes an analyte monitor includes a sensor, a sensor control unit, and a display unit. The sensor has, for example, a substrate, a recessed channel formed in the substrate, and conductive material disposed in the recessed channel to form a working electrode. The sensor control unit typically has a housing adapted for placement on skin and is adapted to receive a portion of an electrochemical sensor. The sensor control unit also includes two or more conductive contacts disposed on the housing and configured for coupling to two or more contact pads on the sensor. A transmitter is disposed in the housing and coupled to the plurality of conductive contacts for transmitting data obtained using the sensor. The display unit has a receiver for receiving data transmitted by the transmitter of the sensor control unit and a display coupled to the receiver for displaying an indication of a level of an analyte. The analyte monitor may also be part of a drug delivery system to alter the level of the analyte based on the data obtained using the sensor.

US2012/018302A1 describes a biosensor unit having a substrate composed of a subcutaneously retained part that is retained under the skin and a base part that is placed on the skin surface. The biosensor unit comprises a sensor part detecting numerical information regarding a substance to be measured as electric signals, a signal amplifying part amplifying the electric signals, a CPU including a calculation part A/D converting the amplified electric signals and processing them to create transmittable data, a storage storing electric signals and data, a transmission part transmitting data to an external device through optical communication, and a battery part for drive. The sensor part is provided on the subcutaneously retained part and the transmission part is provided on the base part.

### SUMMARY OF THE INVENTION

The invention is defined by the appended claims. The purpose and advantages of the disclosed subject matter will be set forth in and apparent from the description that follows, as well as will be learned by practice of the disclosed subject matter. Additional advantages of the disclosed subject matter will be realized and attained by the methods and systems particularly pointed out in the written description and claims hereof, as well as from the appended drawings.

### SUMMARY OF THE DISCLOSURE

To achieve these and other advantages and in accordance with the purpose of the disclosed subject matter, as embodied and broadly described, the disclosed subject matter is directed to a system for measuring an analyte level including an analyte sensor having an in vivo portion configured to be positioned in contact with the interstitial fluid of the user and an ex vivo portion. The analyte sensor can include a first substrate, at least one working electrode, and a reference electrode. The at least one working electrode can be located on the in vivo portion and can sense signals associated with an analyte level in the interstitial fluid of a user. The ex vivo portion can include a plurality of electronic components mounted thereon, and at least one of the plurality of electronic components can be configured to receive the generated signals associated with the analyte level.

As embodied herein, the electronic components can be electrically coupled to at least one of the working electrodes and the reference electrode. The plurality of electronic components can be further configured to transmit the signals associated with the analyte level to a remote device having a display screen. As embodied herein, the electronic components can be mounted to the ex vivo portion using photonic soldering.

As embodied herein, the first substrate can be a flexible monolithic unit. The first substrate can be polyamide or polyethylene terephthalate. The electronic components can include one or more processors and one or more batteries. As embodied herein, the electronic components can include a Wi-Fi antenna, NFC antenna, Bluetooth antenna, BTLE antenna, or GPS antenna. As embodied herein, the one or more batteries can be a printed battery. As embodied herein, the analyte sensor further can include a second substrate with at least one antenna.

As embodied herein, the remote device can be a display device, a mobile phone, or a wrist-mounted device. As embodied herein, the analyte sensor can be configured to sense at least one of lactate, glucose, or ketone.

As embodied herein, the ex vivo portion can include a first layer. The first layer can include a gradient mix of materials, and the mix of materials can include fiberglass. As embodied herein, the first layer can be approximately 10% fiberglass and the in vivo portion can include PET. As embodied herein, the ex vivo portion can include at least a second layer. In some embodiments, each of the first layer and at least second layer can include a gradient mix of materials.

As embodied herein, the system can include a sensor control device for housing the analyte sensor and an applicator for delivering the analyte sensor, wherein the applicator can include a housing with a sensor carrier configured to secure the sensor control device within the interior of the applicator and an applicator cap removably coupled to the housing to seal the interior of the applicator.

The disclosed subject matter is also directed to a method of assembling a system for measuring an analyte level including providing an analyte sensor having an in vivo portion configured to be positioned in contact with the interstitial fluid of the user and an ex vivo portion. The analyte sensor can include a first substrate, at least one working electrode, and a reference electrode. The at least one working electrode and reference electrode can be located on the in vivo portion and can further generate signals associated with a analyte level. The electronic components can be mounted on the ex vivo portion and at least one of the electronic components can be configured to receive the generated signals associated with the analyte level.

As embodied herein, the first substrate can be flexible. The at least one working electrode and the reference electrode can be printed on a substrate. According to some embodiments, the method can include printing the working electrode on a first surface of the analyte sensor and printing the reference electrode on a second surface of the analyte sensor.

As embodied herein, the method can include connecting the electronic to the first surface of the analyte sensor using photonic soldering. As embodied herein, the method can include mounting the electronic components to a first surface photonic soldering. As embodied herein, the method can include masking a portion of the first substrate prior to photonic soldering. As embodied herein, the method can include coating the first substrate with a reflective coating prior to performing the photonic soldering. As embodied herein, during the photonic soldering process, the method can include using a vacuum to prevent the first substrate from warping.

As embodied herein, method can include sterilizing the analyte sensor. The analyte sensor can be sterilized using radiation sterilization, heat treatment, electronic-beam sterilization, gamma sterilization, x-ray sterilization, ethylene oxide sterilization, autoclave steam sterilization, chlorine dioxide gas sterilization, or hydrogen peroxide sterilization. Further, as embodied herein, the analyte sensor can be sterilized before mounting the electronic components to the ex vivo portion. As embodied herein, the analyte sensor can be sterilized after mounting the plurality of electronic components to the ex vivo portion.

### BRIEF DESCRIPTION OF THE FIGURES

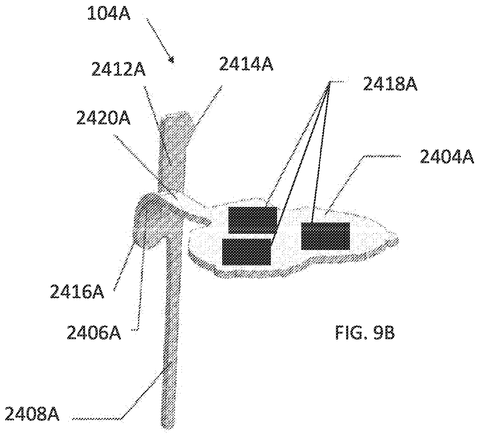

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be illustrated schematically rather than literally or precisely. Embodiments falling under the claimed invention are in particular described in fig. 9B, 11H and 40.
FIG. 1 is a system overview of a sensor applicator, reader device, monitoring system, network, and remote system.
FIG. 2A is a block diagram depicting an example embodiment of a reader device.
FIGS. 2B and 2C are block diagrams depicting example embodiments of sensor control devices.
FIG. 3A is a proximal perspective view depicting an example embodiment of a user preparing a tray for an assembly.
FIG. 3B is a side view depicting an example embodiment of a user preparing an applicator device for an assembly.
FIG. 3C is a proximal perspective view depicting an example embodiment of a user inserting an applicator device into a tray during an assembly.
FIG. 3D is a proximal perspective view depicting an example embodiment of a user removing an applicator device from a tray during an assembly.
FIG. 3E is a proximal perspective view depicting an example embodiment of a patient applying a sensor using an applicator device.
FIG. 3F is a proximal perspective view depicting an example embodiment of a patient with an applied sensor and a used applicator device.
FIG. 4A is a side view depicting an example embodiment of an applicator device coupled with a cap.
FIG. 4B is a side perspective view depicting an example embodiment of an applicator device and cap decoupled.
FIG. 4C is a perspective view depicting an example embodiment of a distal end of an applicator device and electronics housing.
FIG. 5 is a proximal perspective view depicting an example embodiment of a tray with sterilization lid coupled.
FIG. 6A is a proximal perspective cutaway view depicting an example embodiment of a tray with sensor delivery components.
FIG. 6B is a proximal perspective view depicting sensor delivery components.
FIGS. 7A to 7B are top and bottom perspective views, respectively, depicting an example embodiment of a sensor module.
FIGS. 8A and 8B are perspective and compressed views, respectively, depicting an example embodiment of a sensor connector.
FIGS. 9A and 9B are perspective views depicting example embodiments of a sensor.
FIGS. 10A and 10B are bottom and top perspective views, respectively, of an example embodiment of a sensor module assembly.
FIGS. 11A and 11B are close-up partial views of an example embodiment of a sensor module assembly.
FIGS. 11C-H are side views of example sensors, according to one or more embodiments of the disclosure.
FIGS. 12A and 12B are isometric and partially exploded isometric views of an example connector assembly, according to one or more embodiments.
FIG. 12C is an isometric bottom view of the connector of FIGS. 12A-12B.
FIGS. 12D and 12E are isometric and partially exploded isometric views of another example connector assembly, according to one or more embodiments.
FIG. 12F is an isometric bottom view of the connector of FIGS. 12D-12E.
FIG. 13A is a perspective view depicting an example embodiment of a sharp module.
FIG. 13B is a perspective view of another example embodiment of a sharp module.
FIGS. 13C and 13D are schematic views depicting the sharp module of FIG. 13B.
FIGS. 13E and 13F are a side schematic view and a top-down schematic view, respectively, of the sharp module of FIG. 13B, as assembled with a sensor module.
FIG. 13G is a perspective view of another example embodiment of a sharp module.
FIG. 13H is a side schematic view depicting the sharp module of FIG. 13G.
FIGS. 13I and 13J are a side cross-sectional view and a side view, respectively, of the sharp module of FIG. 13G, as assembled with a sensor module.
FIGS. 14A and 14B are isometric and side views, respectively, of another example sensor control device.
FIGS. 15A and 15B are exploded isometric top and bottom views, respectively of the sensor control device of FIGS. 14A-14B.
FIG. 16 is a cross-sectional side view of an assembled sealed subassembly, according to one or more embodiments.
FIGS. 17A-17C are progressive cross-sectional side views showing assembly of the sensor applicator with the sensor control device of FIGS. 14A-14B.
FIGS. 18A and 18B are perspective and top views, respectively, of the cap post of FIG. 17C, according to one or more additional embodiments.
FIG. 19 is a cross-sectional side view of the sensor control device of FIGS. 14A-14B.
FIGS. 20A and 20B are cross-sectional side views of the sensor applicator ready to deploy the sensor control device to a target monitoring location.
FIGS. 21A-21C are progressive cross-sectional side views showing assembly and disassembly of an example embodiment of the sensor applicator with the sensor control device of FIGS. 14A-14B.
FIG. 22A is an isometric bottom view of the housing, according to one or more embodiments.
FIG. 23A is an isometric bottom view of the housing with the sheath and other components at least partially positioned therein.
FIG. 24 is an enlarged cross-sectional side view of the sensor applicator with the sensor control device installed therein, according to one or more embodiments.
FIG. 25A is an isometric top view of the cap, according to one or more embodiments.
FIG. 25B is an enlarged cross-sectional view of the engagement between the cap and the housing, according to one or more embodiments.
FIGS. 26A and 26B are isometric views of the sensor cap and the collar, respectively, according to one or more embodiments.
FIGS. 27A and 27B are side and isometric views, respectively, of an example sensor control device, according to one or more embodiments of the present disclosure.
FIGS. 28A and 28B are exploded, isometric top and bottom views, respectively, of the sensor control device of FIG. 2, according to one or more embodiments.
FIG. 29 is a cross-sectional side view of the sensor control device of FIGS. 27A-27B and 28A-28B, according to one or more embodiments.
FIG. 29A is an exploded isometric view of a portion of another embodiment of the sensor control device of FIGS. 27A-27B and 28A-28B.
FIG. 30A is an isometric bottom view of the mount of FIGS. 27A-27B and 28A-28B.
FIG. 30B is an isometric top view of the sensor cap of FIGS. 27A-27B and 28A-28B.
FIGS. 31A and 31B are side and cross-sectional side views, respectively, of an example sensor applicator, according to one or more embodiments.
FIGS. 32A and 32B are perspective and top views, respectively, of the cap post of FIG. 31B, according to one or more embodiments.
FIG. 33 is a cross-sectional side view of the sensor control device positioned within the applicator cap, according to one or more embodiments.
FIG. 34 is a cross-sectional view of a sensor control device showing example interaction between the sensor and the sharp.
FIGS. 35A-35F illustrate cross-sectional views depicting an example embodiment of an applicator during a stage of deployment.
FIGS. 36A-36H illustrate steps of a process for assembling a sensor subassembly.
FIGS. 37A-37J illustrate steps of a process for assembling a sensor control device.
FIGS. 38A-38K illustrate steps of a process for assembling an applicator.
FIG. 39 is a cross-sectional view of a sensor applicator of FIG. 10A and an external sterilization assembly.
FIG. 40 is an exploded view of an exemplary sensor control device with a monolithically integrated sensor and PCB, according to one or more embodiments.

### DETAILED DESCRIPTION

Before the present subject matter is described in detail, it is to be understood that this disclosure is not limited to the particular embodiments described, as such may, of course, vary. It is also to be understood that the terminology used herein is for the purpose of describing particular embodiments only, and is not intended to be limiting, since the scope of the present disclosure will be limited only by the appended claims.

As used herein and in the appended claims, the singular forms "a," "an," and "the" include plural referents unless the context clearly dictates otherwise.

The publications discussed herein are provided solely for their disclosure prior to the filing date of the present application. Nothing herein is to be construed as an admission that the present disclosure is not entitled to antedate such publication by virtue of prior disclosure. Further, the dates of publication provided may be different from the actual publication dates which may need to be independently confirmed.

Generally, embodiments of the present disclosure include systems, devices, and methods for the use of analyte sensor insertion applicators for use with in vivo analyte monitoring systems. An applicator can be provided to the user in a sterile package with an electronics housing of the sensor control device contained therein. According to some embodiments, a structure separate from the applicator, such as a container, can also be provided to the user as a sterile package with a sensor module and a sharp module contained therein. The user can couple the sensor module to the electronics housing, and can couple the sharp to the applicator with an assembly process that involves the insertion of the applicator into the container in a specified manner. In other embodiments, the applicator, sensor control device, sensor module, and sharp module can be provided in a single package. The applicator can be used to position the sensor control device on a human body with a sensor in contact with the wearer's bodily fluid. The embodiments provided herein are improvements to reduce the likelihood that a sensor is improperly inserted or damaged, or elicits an adverse physiological response. Other improvements and advantages are provided as well. The various configurations of these devices are described in detail by way of the embodiments which are only examples.

Furthermore, many embodiments include in vivo analyte sensors structurally configured so that at least a portion of the sensor is, or can be, positioned in the body of a user to obtain information about at least one analyte of the body. It should be noted, however, that the embodiments disclosed herein can be used with in vivo analyte monitoring systems that incorporate in vitro capability, as well as purely in vitro or ex vivo analyte monitoring systems, including systems that are entirely non-invasive.

Furthermore, for each and every embodiment of a method disclosed herein, systems and devices capable of performing each of those embodiments are covered within the scope of the present disclosure. For example, embodiments of sensor control devices are disclosed and these devices can have one or more sensors, analyte monitoring circuits (e.g., an analog circuit), memories (e.g., for storing instructions), power sources, communication circuits, transmitters, receivers, processors and/or controllers (e.g., for executing instructions) that can perform any and all method steps or facilitate the execution of any and all method steps. These sensor control device embodiments can be used and can be capable of use to implement those steps performed by a sensor control device from any and all of the methods described herein.

As mentioned, a number of embodiments of systems, devices, and methods are described herein that provide for the improved assembly and use of dermal sensor insertion devices for use with in vivo analyte monitoring systems. In particular, several embodiments of the present disclosure are designed to improve the method of sensor insertion with respect to in vivo analyte monitoring systems and, in particular, to prevent the premature retraction of an insertion sharp during a sensor insertion process. Some embodiments, for example, include a dermal sensor insertion mechanism with an increased firing velocity and a delayed sharp retraction. In other embodiments, the sharp retraction mechanism can be motion-actuated such that the sharp is not retracted until the user pulls the applicator away from the skin. Consequently, these embodiments can reduce the likelihood of prematurely withdrawing an insertion sharp during a sensor insertion process; decrease the likelihood of improper sensor insertion; and decrease the likelihood of damaging a sensor during the sensor insertion process, to name a few advantages. Several embodiments of the present disclosure also provide for improved insertion sharp modules to account for the small scale of dermal sensors and the relatively shallow insertion path present in a subject's dermal layer. In addition, several embodiments of the present disclosure are designed to prevent undesirable axial and/or rotational movement of applicator components during sensor insertion. Accordingly, these embodiments can reduce the likelihood of instability of a positioned dermal sensor, irritation at the insertion site, damage to surrounding tissue, and breakage of capillary blood vessels resulting in fouling of the dermal fluid with blood, to name a few advantages. In addition, to mitigate inaccurate sensor readings which can be caused by trauma at the insertion site, several embodiments of the present disclosure can reduce the end-depth penetration of the needle relative to the sensor tip during insertion.

Before describing these aspects of the embodiments in detail, however, it is first desirable to describe examples of devices that can be present within, for example, an in vivo analyte monitoring system, as well as examples of their operation, all of which can be used with the embodiments described herein.

There are various types of in vivo analyte monitoring systems. "Continuous Analyte Monitoring" systems (or "Continuous Glucose Monitoring" systems), for example, can transmit data from a sensor control device to a reader device continuously without prompting, e.g., automatically according to a schedule. "Flash Analyte Monitoring" systems (or "Flash Glucose Monitoring" systems or simply "Flash" systems), as another example, can transfer data from a sensor control device in response to a scan or request for data by a reader device, such as with a Near Field Communication (NFC) or Radio Frequency Identification (RFID) protocol. In vivo analyte monitoring systems can also operate without the need for finger stick calibration.

In vivo analyte monitoring systems can be differentiated from "in vitro" systems that contact a biological sample outside of the body (or "ex vivo") and that typically include a meter device that has a port for receiving an analyte test strip carrying bodily fluid of the user, which can be analyzed to determine the user's blood sugar level.

In vivo monitoring systems can include a sensor that, while positioned in vivo, makes contact with the bodily fluid of the user and senses the analyte levels contained therein. The sensor can be part of the sensor control device that resides on the body of the user and contains the electronics and power supply that enable and control the analyte sensing. The sensor control device, and variations thereof, can also be referred to as a "sensor control unit," an "on-body electronics" device or unit, an "on-body" device or unit, or a "sensor data communication" device or unit, to name a few.

In vivo monitoring systems can also include a device that receives sensed analyte data from the sensor control device and processes and/or displays that sensed analyte data, in any number of forms, to the user. This device, and variations thereof, can be referred to as a "handheld reader device," "reader device" (or simply a "reader"), "handheld electronics" (or simply a "handheld"), a "portable data processing" device or unit, a "data receiver," a "receiver" device or unit (or simply a "receiver"), or a "remote" device or unit, to name a few. Other devices such as personal computers have also been utilized with or incorporated into in vivo and in vitro monitoring systems.

### Example Embodiment of In Vivo Analyte Monitoring System

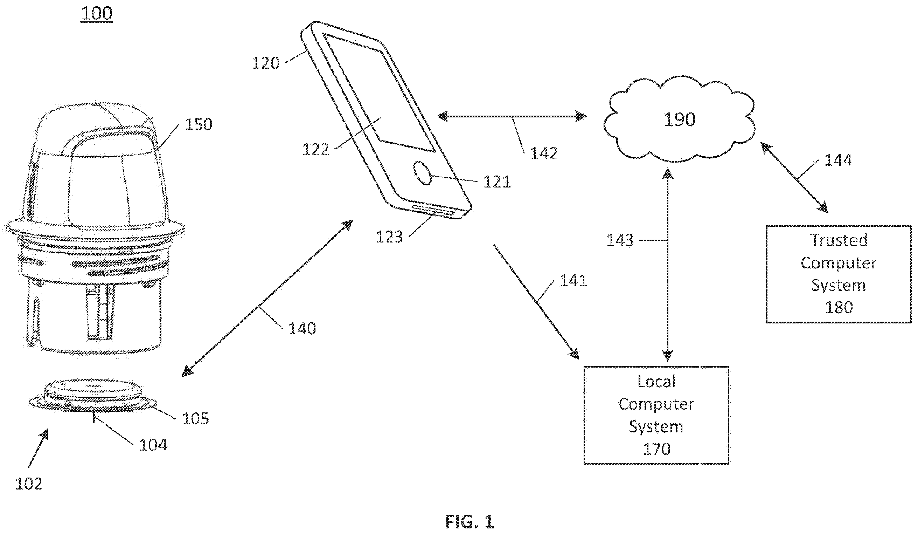

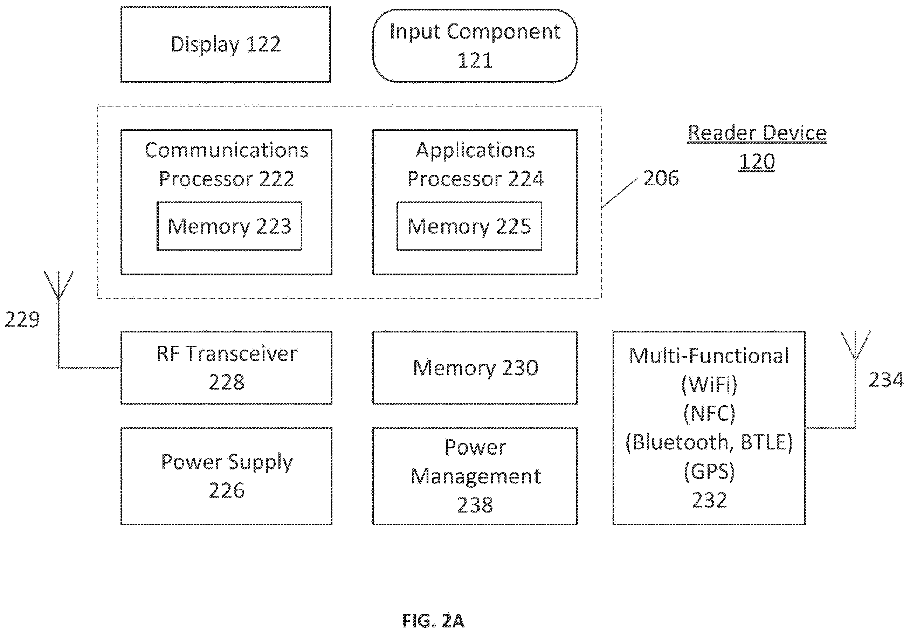

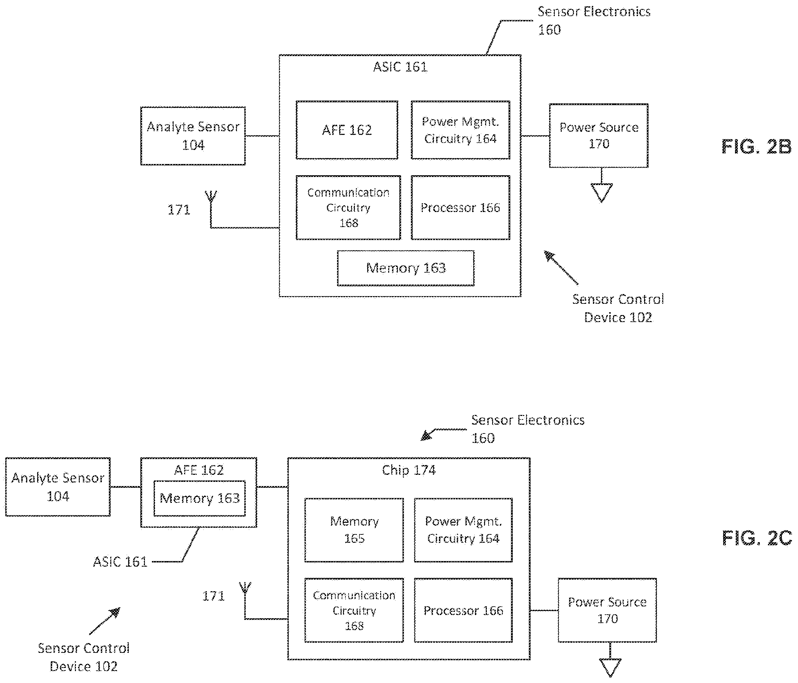

FIG. 1 is a conceptual diagram depicting an example embodiment of an analyte monitoring system 100 that includes a sensor applicator 150, a sensor control device 102, and a reader device 120. Here, sensor applicator 150 can be used to deliver sensor control device 102 to a monitoring location on a user's skin where a sensor 104 is maintained in position for a period of time by an adhesive patch 105. Sensor control device 102 is further described in FIGS. 2B and 2C, and can communicate with reader device 120 via a communication path 140 using a wired or wireless technique. Example wireless protocols include Bluetooth, Bluetooth Low Energy (BLE, BTLE, Bluetooth SMART, etc.), Near Field Communication (NFC) and others. Users can monitor applications installed in memory on reader device 120 using screen 122 and input 121 and the device battery can be recharged using power port 123. More detail about reader device 120 is set forth with respect to FIG. 2A below. Reader device 120 can communicate with local computer system 170 via a communication path 141 using a wired or wireless technique. Local computer system 170 can include one or more of a laptop, desktop, tablet, phablet, smartphone, set-top box, video game console, or other computing device and wireless communication can include any of a number of applicable wireless networking protocols including Bluetooth, Bluetooth Low Energy (BTLE), Wi-Fi or others. Local computer system 170 can communicate via communications path 143 with a network 190 similar to how reader device 120 can communicate via a communications path 142 with network 190, by wired or wireless technique as described previously. Network 190 can be any of a number of networks, such as private networks and public networks, local area or wide area networks, and so forth. A trusted computer system 180 can include a server and can provide authentication services and secured data storage and can communicate via communications path 144 with network 190 by wired or wireless technique.

### Example Embodiment of Reader Device

FIG. 2A is a block diagram depicting an example embodiment of a reader device configured as a smartphone. Here, reader device 120 can include a display 122, input component 121, and a processing core 206 including a communications processor 222 coupled with memory 223 and an applications processor 224 coupled with memory 225. Also included can be separate memory 230, RF transceiver 228 with antenna 229, and power supply 226 with power management module 238. Further included can be a multifunctional transceiver 232 which can communicate over Wi-Fi, NFC, Bluetooth, BTLE, and GPS with one or more antennas 234. As understood by one of skill in the art, these components are electrically and communicatively coupled in a manner to make a functional device.

### Example Embodiments of Sensor Control Device

FIGS. 2B and 2C are block diagrams depicting example embodiments of sensor control device 102 having analyte sensor 104 and sensor electronics 160 (including analyte monitoring circuitry) that can have the majority of the processing capability for rendering end-result data suitable for display to the user. In FIG. 2B, a single semiconductor chip 161 is depicted that can be a custom application specific integrated circuit (ASIC). Shown within ASIC 161 are certain high-level functional units, including an analog front end (AFE) 162, power management (or control) circuitry 164, processor 166, and communication circuitry 168 (which can be implemented as a transmitter, receiver, transceiver, passive circuit, or otherwise according to the communication protocol). In this embodiment, both AFE 162 and processor 166 are used as analyte monitoring circuitry, but in other embodiments either circuit can perform the analyte monitoring function. Processor 166 can include one or more processors, microprocessors, controllers, and/or microcontrollers, each of which can be a discrete chip or distributed amongst (and a portion of) a number of different chips.

A memory 163 is also included within ASIC 161 and can be shared by the various functional units present within ASIC 161, or can be distributed amongst two or more of them. Memory 163 can also be a separate chip. Memory 163 can be volatile and/or nonvolatile memory. In this embodiment, ASIC 161 is coupled with power source 170, which can be a coin cell battery, or the like. AFE 162 interfaces with in vivo analyte sensor 104 and receives measurement data therefrom and outputs the data to processor 166 in digital form, which in turn processes the data to arrive at the end-result glucose discrete and trend values, etc. This data can then be provided to communication circuitry 168 for sending, by way of one or more antennas 171, to reader device 120 (not shown), for example, where minimal further processing is needed by the resident software application to display the data.

FIG. 2C is similar to FIG. 2B but instead includes two discrete semiconductor chips 162 and 174, which can be packaged together or separately. Here, AFE 162 is resident on ASIC 161. Processor 166 is integrated with power management circuitry 164 and communication circuitry 168 on chip 174. AFE 162 includes memory 163 and chip 174 includes memory 165, which can be isolated or distributed within. In one example embodiment, AFE 162 is combined with power management circuitry 164 and processor 166 on one chip, while communication circuitry 168 is on a separate chip. In another example embodiment, both AFE 162 and communication circuitry 168 are on one chip, and processor 166 and power management circuitry 164 are on another chip. It should be noted that other chip combinations are possible, including three or more chips, each bearing responsibility for the separate functions described, or sharing one or more functions for fail-safe redundancy.

### Example Embodiment of Assembly Process for Sensor Control Device

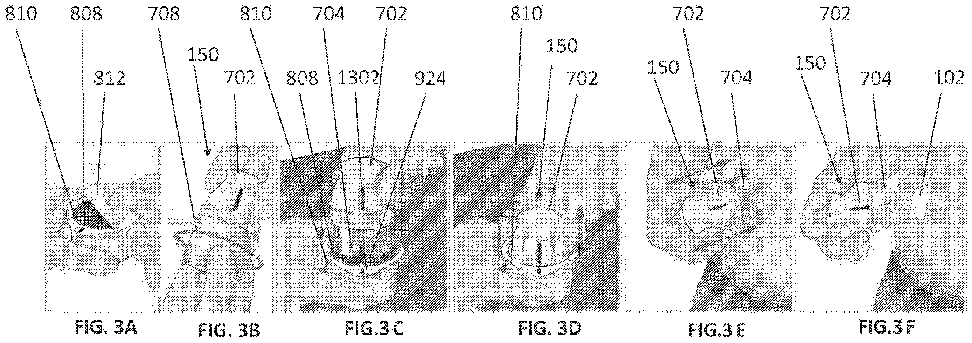

The components of sensor control device 102 can be acquired by a user in multiple packages requiring final assembly by the user before delivery to an appropriate user location. FIGS. 3A-3D depict an example embodiment of an assembly process for sensor control device 102 by a user, including preparation of separate components before coupling the components in order to ready the sensor for delivery. FIGS. 3E-3F depict an example embodiment of delivery of sensor control device 102 to an appropriate user location by selecting the appropriate delivery location and applying device 102 to the location.

FIG. 3A is a proximal perspective view depicting an example embodiment of a user preparing a container 810, configured here as a tray (although other packages can be used), for an assembly process. The user can accomplish this preparation by removing lid 812 from tray 810 to expose platform 808, for instance by peeling a non-adhered portion of lid 812 away from tray 810 such that adhered portions of lid 812 are removed. Removal of lid 812 can be appropriate in various embodiments so long as platform 808 is adequately exposed within tray 810. Lid 812 can then be placed aside.

FIG. 3B is a side view depicting an example embodiment of a user preparing an applicator device 150 for assembly. Applicator device 150 can be provided in a sterile package sealed by a cap 708. Preparation of applicator device 150 can include uncoupling housing 702 from cap 708 to expose sheath 704 (FIG. 3C). This can be accomplished by unscrewing (or otherwise uncoupling) cap 708 from housing 702. Cap 708 can then be placed aside.

FIG. 3C is a proximal perspective view depicting an example embodiment of a user inserting an applicator device 150 into a tray 810 during an assembly. Initially, the user can insert sheath 704 into platform 808 inside tray 810 after aligning housing orienting feature 1302 (or slot or recess) and tray orienting feature 924 (an abutment or detent). Inserting sheath 704 into platform 808 temporarily unlocks sheath 704 relative to housing 702 and also temporarily unlocks platform 808 relative to tray 810. At this stage, removal of applicator device 150 from tray 810 will result in the same state prior to initial insertion of applicator device 150 into tray 810 (i.e., the process can be reversed or aborted at this point and then repeated without consequence).

Sheath 704 can maintain position within platform 808 with respect to housing 702 while housing 702 is distally advanced, coupling with platform 808 to distally advance platform 808 with respect to tray 810. This step unlocks and collapses platform 808 within tray 810. Sheath 704 can contact and disengage locking features (not shown) within tray 810 that unlock sheath 704 with respect to housing 702 and prevent sheath 704 from moving (relatively) while housing 702 continues to distally advance platform 808. At the end of advancement of housing 702 and platform 808, sheath 704 is permanently unlocked relative to housing 702. A sharp and sensor (not shown) within tray 810 can be coupled with an electronics housing (not shown) within housing 702 at the end of the distal advancement of housing 702. Operation and interaction of the applicator device 150 and tray 810 are further described below.

FIG. 3D is a proximal perspective view depicting an example embodiment of a user removing an applicator device 150 from a tray 810 during an assembly. A user can remove applicator 150 from tray 810 by proximally advancing housing 702 with respect to tray 810 or other motions having the same end effect of uncoupling applicator 150 and tray 810. The applicator device 150 is removed with sensor control device 102 (not shown) fully assembled (sharp, sensor, electronics) therein and positioned for delivery.

FIG. 3E is a proximal perspective view depicting an example embodiment of a patient applying sensor control device 102 using applicator device 150 to a target area of skin, for instance, on an abdomen or other appropriate location. Advancing housing 702 distally collapses sheath 704 within housing 702 and applies the sensor to the target location such that an adhesive layer on the bottom side of sensor control device 102 adheres to the skin. The sharp is automatically retracted when housing 702 is fully advanced, while the sensor (not shown) is left in position to measure analyte levels.

FIG. 3F is a proximal perspective view depicting an example embodiment of a patient with sensor control device 102 in an applied position. The user can then remove applicator 150 from the application site.

System 100, described with respect to FIGS. 3A-3F and elsewhere herein, can provide a reduced or eliminated chance of accidental breakage, permanent deformation, or incorrect assembly of applicator components compared to prior art systems. Since applicator housing 702 directly engages platform 808 while sheath 704 unlocks, rather than indirect engagement via sheath 704, relative angularity between sheath 704 and housing 702 will not result in breakage or permanent deformation of the arms or other components. The potential for relatively high forces (such as in conventional devices) during assembly will be reduced, which in turn reduces the chance of unsuccessful user assembly.

### Example Embodiment of Sensor Applicator Device

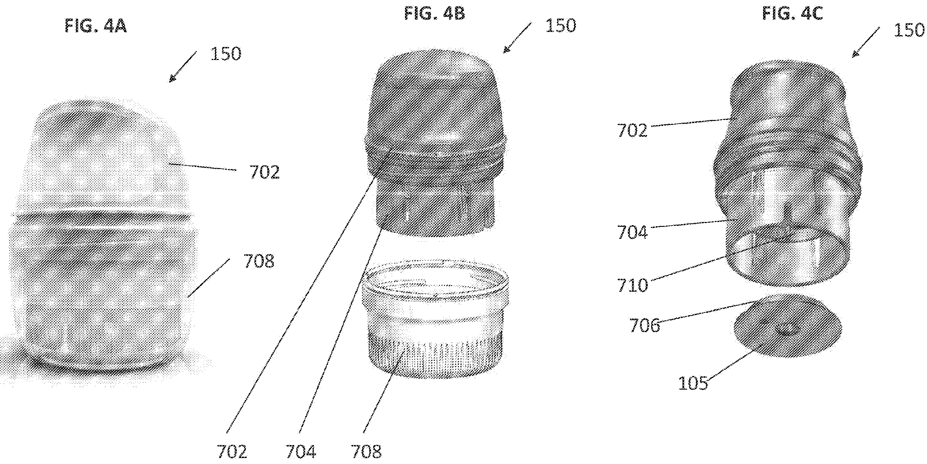

FIG. 4A is a side view depicting an example embodiment of an applicator device 150 coupled with screw cap 708. This is an example of how applicator 150 is shipped to and received by a user, prior to assembly by the user with a sensor. FIG. 4B is a side perspective view depicting applicator 150 and cap 708 after being decoupled. FIG. 4C is a perspective view depicting an example embodiment of a distal end of an applicator device 150 with electronics housing 706 and adhesive patch 105 removed from the position they would have retained within sensor electronics carrier 710 of sheath 704, when cap 708 is in place.

### Example Embodiment of Tray and Sensor Module Assembly

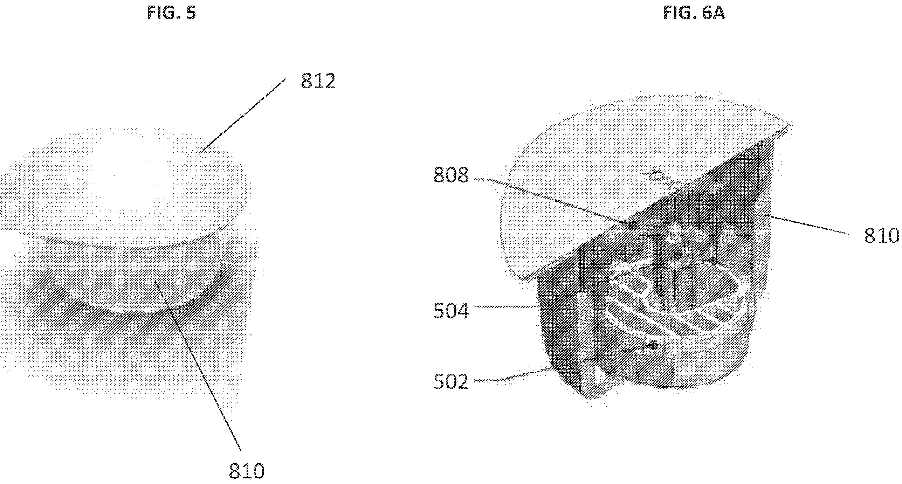

FIG. 5 is a proximal perspective view depicting an example embodiment of a tray 810 with sterilization lid 812 removably coupled thereto, which may be representative of how the package is shipped to and received by a user prior to assembly.

FIG. 6A is a proximal perspective cutaway view depicting sensor delivery components within tray 810. Platform 808 is slidably coupled within tray 810. Desiccant 502 is stationary with respect to tray 810. Sensor module 504 is mounted within tray 810.

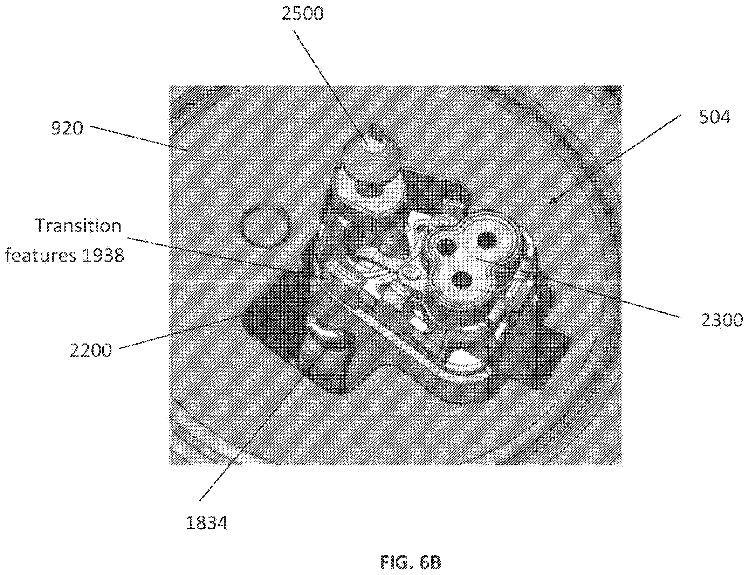

FIG. 6B is a proximal perspective view depicting sensor module 504 in greater detail. Here, retention arm extensions 1834 of platform 808 releasably secure sensor module 504 in position. Module 2200 is coupled with connector 2300, sharp module 2500 and sensor (not shown) such that during assembly they can be removed together as sensor module 504.

### Example Embodiments of Sensor Modules

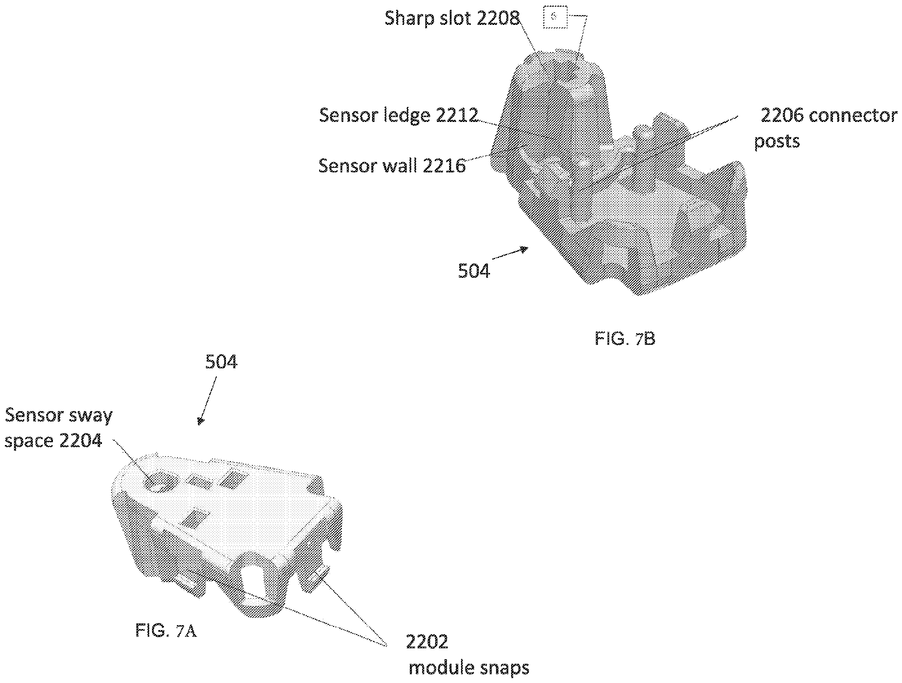

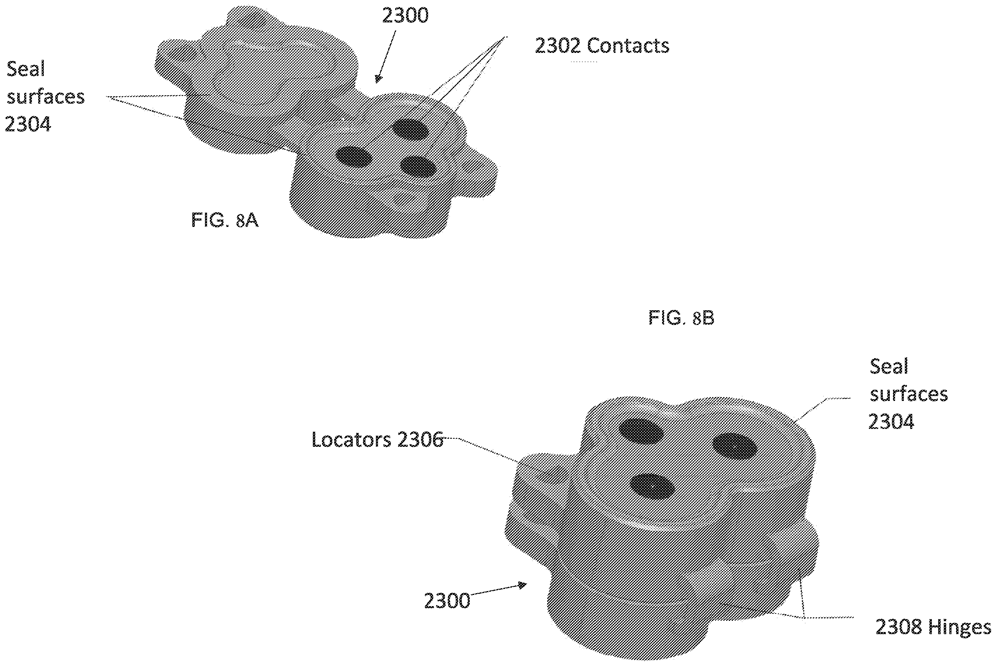

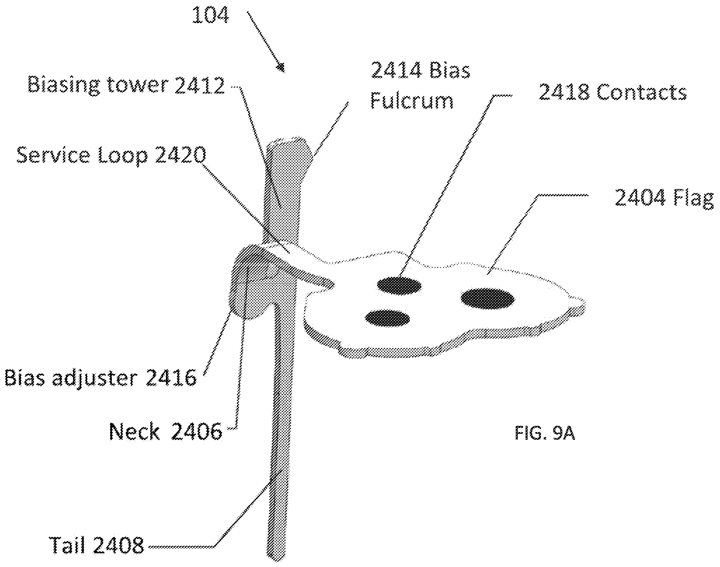

FIGS. 7A and 7B are a top perspective view and a bottom perspective view, respectively, depicting an example embodiment of sensor module 504. Module 504 can hold a connector 2300 (FIGS. 8A and 8B) and a sensor 104 (FIG. 9A). Module 504 is capable of being securely coupled with electronics housing 706. One or more deflectable arms or module snaps 2202 can snap into the corresponding features 2010 of housing 706. A sharp slot 2208 can provide a location for sharp tip 2502 to pass through and sharp shaft 2504 to temporarily reside. A sensor ledge 2212 can define a sensor position in a horizontal plane, prevent a sensor from lifting connector 2300 off of posts and maintain sensor 104 parallel to a plane of connector seals. It can also define sensor bend geometry and minimum bend radius. It can limit sensor travel in a vertical direction and prevent a tower from protruding above an electronics housing surface and define a sensor in vivo portion length below a patch surface. A sensor wall 2216 can constrain a sensor and define a sensor bend geometry and minimum bend radius.

FIGS. 8A and 8B are perspective views depicting an example embodiment of connector 2300 in an open state and a closed state, respectively. Connector 2300 can be made of silicone rubber that encapsulates compliant carbon impregnated polymer modules that serve as electrical conductive contacts 2302 between sensor 104 and electrical circuitry contacts for the electronics within housing 706. The connector can also serve as a moisture barrier for sensor 104 when assembled in a compressed state after transfer from a container to an applicator and after application to a user's skin. A plurality of seal surfaces 2304 can provide a watertight seal for electrical contacts and sensor contacts. One or more hinges 2208 can connect two distal and proximal portions of connector 2300.

FIG. 9A is a perspective view depicting an example embodiment of sensor 104. A neck 2406 can be a zone which allows folding of the sensor, for example ninety degrees. A membrane on in vivo portion 2408 can cover an active analyte sensing element of the sensor 104. In vivo portion 2408 can be the portion of sensor 104 that resides under a user's skin after insertion. A ex vivo portion 2404 can contain contacts and a sealing surface. A biasing tower 2412 can be a tab that biases the in vivo portion 2408 into sharp slot 2208. A bias fulcrum 2414 can be an offshoot of biasing tower 2412 that contacts an inner surface of a needle to bias a in vivo portion into a slot. A bias adjuster 2416 can reduce a localized bending of a in vivo portion connection and prevent sensor trace damage. Contacts 2418 can electrically couple the active portion of the sensor to connector 2300. A service loop 2420 can translate an electrical path from a vertical direction ninety degrees and engage with sensor ledge 2212 (FIG. 7B).

Referring again to FIG. 9A, the sensor 104 can be configured with a neck 2406, interconnecting the ex vivo portion 2404 and the in vivo portion 2408, that allows bending of the sensor 104 between the ex vivo portion 2404 and the in vivo portion 2408. In one example, the neck 2406 can be bent about ninety degrees to facilitate the contacts 2418 of the ex vivo portion 2404 making contact with a sensor ledge 2212 (FIG. 7B). The sensor 104, however, can be manufactured, and in some embodiments even shipped, or stored, in a relatively flat configuration where there is substantially no bend in the neck 2406 of the sensor 104, such that the ex vivo portion 2404, neck 2406, and in vivo portion 2408 can form a substantially planar surface. To configure the sensor 104 in the illustrated embodiment, the neck 2406 must be bent. However, bending the neck 2406 subjects the sensor 104 generally, and the neck 2404 in particular, to stresses that may weaken or damage the sensor 104, cause microfactures, or otherwise reduce its efficiency and efficacy. Techniques described herein below can ensure that the neck 2404 can be bent to a desired angle while reducing damage to the sensor 104 and its constituent parts.

One exemplary technique to reduce damage caused by bending the neck 2406 of the sensor 104 is to apply a sufficient amount of heat for a sufficient amount of time in temporal proximity to the time when the neck 2406 will be bent. These factors of the degree of heat, the length of time of exposure, and the nearness of the application of heat to the time when the bend is conducted, can be determined based on the type of material comprising the sensor 104 generally and the neck 2406 in particular with suitable examples provided below. Care must be taken, for example, to avoid damaging the contacts 2418 and the membrane covering the in vivo portion 2408.

The application of heat can be controlled by the manufacturing components used to bend the neck 2406. In one embodiment, the neck 2406 can be bent, or folded, by heating a portion of the neck 2406 of the sensor 104 to a predetermined temperature and bending the neck 2406 of the sensor 104 to form an angle between the in vivo portion 2408 of the sensor 104 and the ex vivo portion 2404 of the sensor. As mentioned, the predetermined temperature and length of heating can be determined based on properties of one or more of the materials comprising the neck 2406 of the sensor 104. The temperature and length of heating can be chosen based on being sufficient to improve malleability of the neck 2406 of the sensor 104 without damaging the rest of the sensor. Heating the neck 2406 of the sensor 104 can include heating only a region of the neck 2406 of the sensor 104, heating substantially all of the neck 2406, or heating one or more other components of the sensor 104.

The heating and bending can be performed by one or more heating and bending apparatuses. For example, the sensor 104 can be inserted in to a first configuration of a heading-bending apparatus that includes separate, dedicated components for heating the neck 2406 and bending the neck 2406. Configuring the sensor 104, then, includes heating the neck 2406 with the first component for heating the neck 2406 before passing the sensor 104 to the second component for bending the neck 2406 to the desired angle. Heating the neck 2406 can be performed by a heating element of a heating apparatus. The heating element can be raised to a desired temperature and can be made to contact, or be brought into close proximity with, the designated portion of the neck 2406 for a set period of time, causing the temperature of the neck 2406 to rise. Additionally, the local temperature around the sensor 104 can be raised to indirectly heat the neck 2406 without contacting the neck 2406 with a heating element directly.

Additionally, the heating and bending can be performed by a unified heated-bending apparatus where the necessary components to the heat the neck 2406 are integrated into the components to bend the neck 2406. Heat, therefore, can be applied during the bending in addition to before or after the bending process is complete. The degree of heat, e.g., the temperature being applied to the neck 2406 can remain consistent during the heating and/or heated-bending process by ensuring that the temperature of the heating element remains substantially consistent and that the distance between the heating element and the neck 2406 remains substantially consistent. Alternatively, the temperature of the neck 2406 can be caused to vary during the bending process. For example, the temperature of the next can be raised to a set threshold temperature, allowed to fall to a set threshold before bending is applied, and can be raised again after the bending process (e.g., to avoid microfractures). Where the heating element is integrated into the bending apparatus, the process can involve increasing or decreasing the temperature of the neck 2406 while the neck 2406 is being bent.

In addition, after bending the neck 2406 to form the desired angle, a step in manufacturing or manipulating the sensor 104 can include verifying the integrity of the sensor 104 after the bending by checking the neck 2406 for microfractures. If the number or intensity of microfractures exceeds a predetermined threshold, the sensor can be discarded. Other integrity checks can include check the sensitive components of the sensor 104 to ensure that they remain in a form that is consistent with their intended functions and have not been compromised by the bending process.

Referring to FIG. 9B, sensor 104A can include each of the features of sensor 104 including neck 2406A, biasing tower 2412A, bias adjuster 2416A, bias fulcrum 2414A, and service loop 2420A. Sensor 104A can also include ex vivo portion 2404A and in vivo portion 2408A. Sensor 104A can further include electronic components 2418A directly mounted to ex vivo portion 2404A, for example, by using photonic soldering, as described in greater detail below. Therefore, the need for contacts 2418 can be eliminated in sensor 104A.

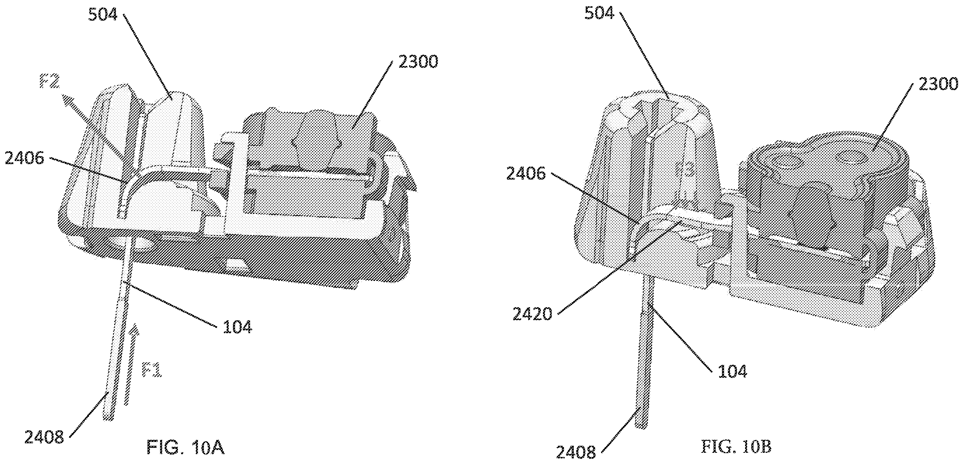

FIGS. 10A and 10B are bottom and top perspective views, respectively, depicting an example embodiment of a sensor module assembly comprising sensor module 504, connector 2300, and sensor 104. According to one aspect of the aforementioned embodiments, during or after insertion, sensor 104 can be subject to axial forces pushing up in a proximal direction against sensor 104 and into the sensor module 105, as shown by force, F1, of FIG. 10A. According to some embodiments, this can result in an adverse force, F2, being applied to neck 2406 of sensor 104 and, consequently, result in adverse forces, F3, being translated to service loop 2420 of sensor 104. In some embodiments, for example, axial forces, F1, can occur as a result of a sensor insertion mechanism in which the sensor is designed to push itself through the tissue, a sharp retraction mechanism during insertion, or due to a physiological reaction created by tissue surrounding sensor 104 (e.g., after insertion).

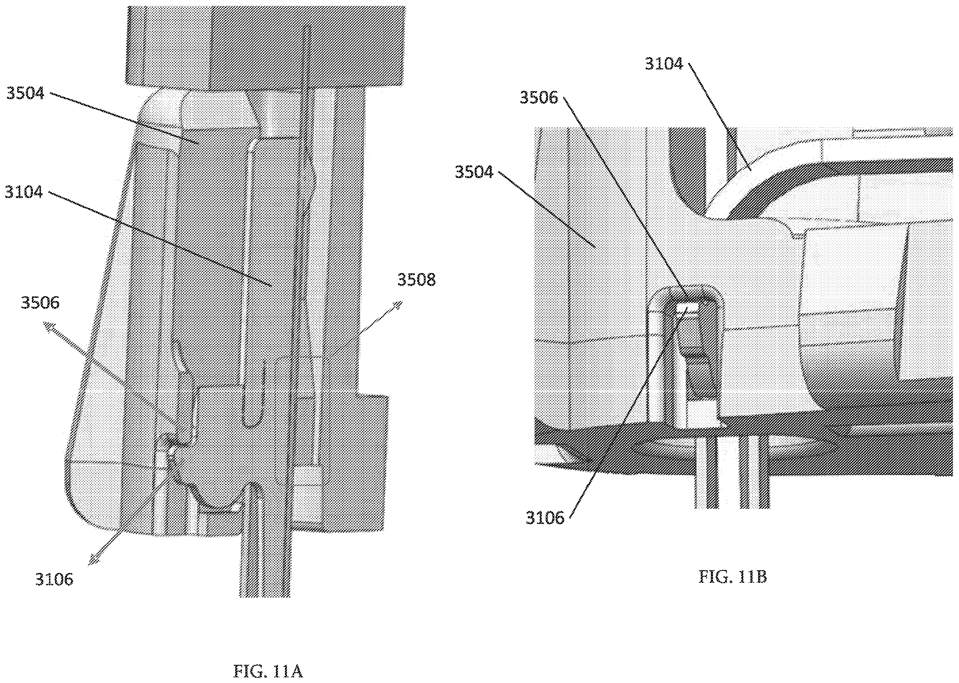

FIGS. 11A and 11B are close-up partial views of an example embodiment of a sensor module assembly having certain axial stiffening features. In a general sense, the embodiments described herein are directed to mitigating the effects of axial forces on the sensor as a result of insertion and/or retraction mechanisms, or from a physiological reaction to the sensor in the body. As can be seen in FIGS. 11A and 11B, according to one aspect of the embodiments, sensor 3104 comprises a proximal portion having a hook feature 3106 configured to engage a catch feature 3506 of the sensor module 3504. In some embodiments, sensor module 3504 can also include a clearance area 3508 to allow a distal portion of sensor 3104 to swing backwards during assembly to allow for the assembly of the hook feature 3106 of sensor 3104 over and into the catch feature 3506 of sensor module 3504.

According to another aspect of the embodiments, the hook and catch features 3106, 3506 operate in the following manner. Sensor 3104 includes a proximal sensor portion, coupled to sensor module 3504, as described above, and a distal sensor portion that is positioned beneath a skin surface in contact with a bodily fluid. As seen in FIGS. 11A and 11B, the proximal sensor portion includes a hook feature 3106 adjacent to the catch feature 3506 of sensor module 3504. During or after sensor insertion, one or more forces are exerted in a proximal direction along a longitudinal axis of sensor 3104. In response to the one or more forces, hook feature 3106 engages catch feature 3506 to prevent displacement of sensor 3104 in a proximal direction along the longitudinal axis.

According to another aspect of the embodiments, sensor 3104 can be assembled with sensor module 3504 in the following manner. Sensor 3104 is loaded into sensor module 3504 by displacing the proximal sensor portion in a lateral direction to bring the hook feature 3106 in proximity to the catch feature 3506 of sensor module 3504. More specifically, displacing the proximal sensor portion in a lateral direction causes the proximal sensor portion to move into clearance area 3508 of sensor module 3504.

Although FIGS. 11A and 11B depict hook feature 3106 as a part of sensor 3104, and catch feature 3506 as a part of sensor module 3504, those of skill in the art will appreciate that hook feature 3106 can instead be a part of sensor module 3504, and, likewise, catch feature 3506 can instead be a part of sensor 3106. Similarly, those of skill in the art will also recognize that other mechanisms (e.g., detent, latch, fastener, screw, etc.) implemented on sensor 3104 and sensor module 3504 to prevent axial displacement of sensor 3104 are possible and within the scope of the present disclosure.

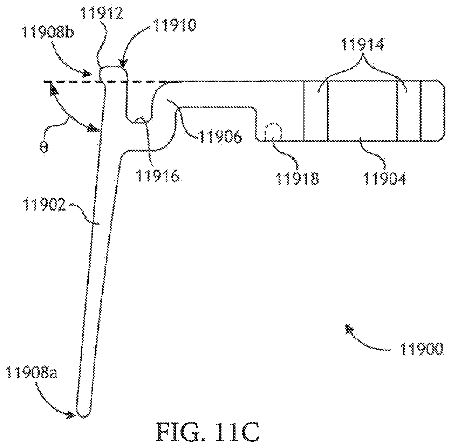

FIG. 11C is a side view of an example sensor 11900, according to one or more embodiments of the disclosure. The sensor 11900 may be similar in some respects to any of the sensors described herein and, therefore, may be used in an analyte monitoring system to detect specific analyte concentrations. As illustrated, the sensor 11900 includes a in vivo portion 11902, a ex vivo portion 11904, and a neck 11906 that interconnects the in vivo portion 11902 and the ex vivo portion 11904. The in vivo portion 11902 includes an enzyme or other chemistry or biologic and, in some embodiments, a membrane may cover the chemistry. In use, the in vivo portion 11902 is transcutaneously received beneath a user's skin, and the chemistry included thereon helps facilitate analyte monitoring in the presence of bodily fluids.

The in vivo portion 11902 may be received within a hollow or recessed portion of a sharp (not shown) to at least partially circumscribe the in vivo portion 11902 of the sensor 11900. As illustrated, the in vivo portion 11902 may extend at an angle Q offset from horizontal. In some embodiments, the angle Q may be about 85°. Accordingly, in contrast to other sensor in vivo portions, the in vivo portion 11902 may not extend perpendicularly from the ex vivo portion 11904, but instead at an angle offset from perpendicular. This may prove advantageous in helping maintain the in vivo portion 11902 within the keep the recessed portion of the sharp.

The in vivo portion 11902 includes a first or bottom end 11908a and a second or top end 11908b opposite the top end 11908a. A tower 11910 may be provided at or near the top end 11908b and may extend vertically upward from the location where the neck 11906 interconnects the in vivo portion 11902 to the ex vivo portion 11904. During operation, if the sharp moves laterally, the tower 11910 will help pivot the in vivo portion 11902 toward the sharp and otherwise stay within the recessed portion of the sharp. Moreover, in some embodiments, the tower 11910 may provide or otherwise define a protrusion 11912 that extends laterally therefrom. When the sensor 11900 is mated with the sharp and the in vivo portion 11902 extends within the recessed portion of the sharp, the protrusion 11912 may engage the inner surface of the recessed portion. In operation, the protrusion 11912 may help keep the in vivo portion 11902 within the recessed portion.

The ex vivo portion 11904 may comprise a generally planar surface having one or more sensor contacts 11914 arranged thereon. The sensor contact(s) 11914 may be configured to align with a corresponding number of compliant carbon impregnated polymer modules encapsulated within a connector.

In some embodiments, as illustrated, the neck 11906 may provide or otherwise define a dip or bend 11916 extending between the ex vivo portion 11904 and the in vivo portion 11902. The bend 11916 may prove advantageous in adding flexibility to the sensor 11900 and helping prevent bending of the neck 11906.

In some embodiments, a notch 11918 (shown in dashed lines) may optionally be defined in the ex vivo portion near the neck 11906. The notch 11918 may add flexibility and tolerance to the sensor 11900 as the sensor 11900 is mounted to the mount. More specifically, the notch 11918 may help take up interference forces that may occur as the sensor 11900 is mounted within the mount.

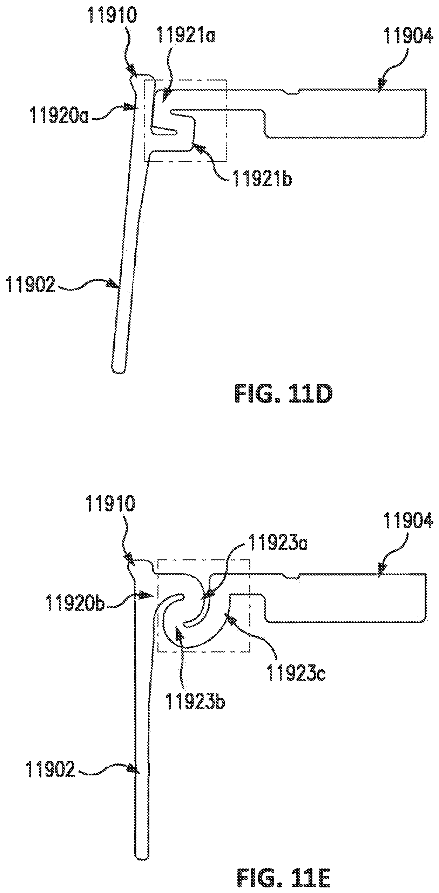

In some embodiments, as illustrated in FIGS. 11D-11G, the neck may comprise or otherwise define a non-linear configuration such as a dip or bend 11920a-11920d with a plurality of turns, e.g., 11921a, 11921b, extending between the ex vivo portion 11904 and the in vivo portion 11902. The bend 11920a-11920d can be advantageous in reducing in-place stiffness of the sensor 11900 by adding flexibility to the sensor 11900 in both a vertically-oriented and horizontally-oriented direction. The added flexibility can provide a multi-directional spring-like structure in the sensor 11900 that helps to limit deformation of the neck 11906 while ensuring that the in vivo portion 11902 and the ex vivo portion 11904 can remain in their expected or fixed positions. The spring-like structure also increases compliance of the sensor 11900 while reducing stress on the overall structure.

Generally, the sensor can be understood as including a in vivo portion, a ex vivo portion, and a neck aligned along a planar surface having a vertical axis and a horizontal axis. The spring-like structure can be formed by various orientations of turns in the bend of the neck of a sensor. Between the in vivo portion and the ex vivo portion, the neck can include at least two turns in relation to the vertical axis providing a spring-like structure. The at least two turns can provide, in relation to an axis of the planar surface shared by the in vivo portion, the ex vivo portion, and the neck, overlapping layers of the structure of the neck, where the neck itself remains unbroken. These overlapping turns make up the spring-like structure. In some embodiments, the overlapping layers of the neck are vertically-oriented. In some embodiments, the overlapping layers of the neck are horizontally-oriented.

FIG. 11D illustrates one embodiment of a sensor 11900 including a neck between the ex vivo portion 11904 and in vivo portion 11902 with a bend 11920a including turns 11921a and 11921b. In the illustrated embodiment, at least one turn 11921a abuts the top end of the in vivo portion or possibly the tower 11910 of the sensor 11900. This orientation can be advantageous in that it reduces the overall footprint of the sensor, even considering the additional material used to generate the bend 11920a. The arrangement can provide multiple overlapping, vertically-aligned horizontal layers between the turns.

FIG. 11E illustrates another embodiment of a sensor 11900 including a neck between the ex vivo portion 11904 and in vivo portion 11902 with a bend 11920b that generally forms a swirl pattern including at least turn turns 11923a, 11923b, and 11923c. In this embodiment, the turns again abut the top end of the in vivo portion or the tower 11910 of the sensor 11900. In addition to maintaining the overall footprint of the sensor, this orientation may provide for additional balancing of the horizontally-oriented and vertically-oriented stresses. The overlapping layers in this arrangement of turns are substantially balanced in along both the horizontal and vertical axes.

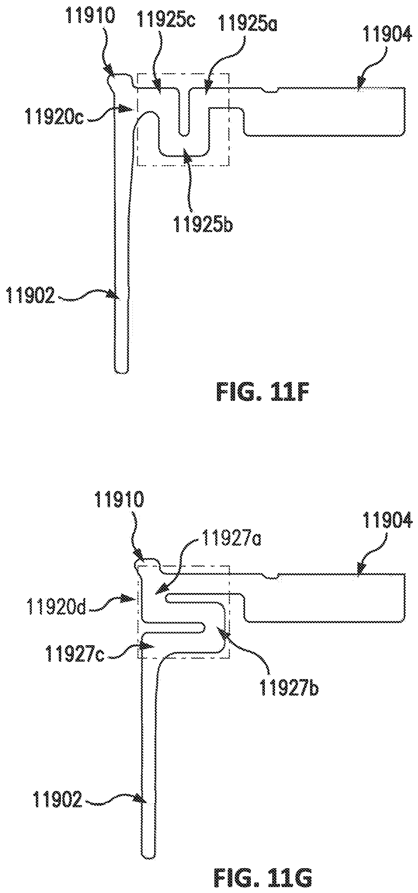

FIG. 11F illustrates another embodiment of a sensor 11900 including a neck between the ex vivo portion 11904 and in vivo portion 11902 with a bend 11920c including turns 11925a, 11925b, and 11925c. In the illustrated embodiment, the turn 11925c connects a region of the in vivo portion 11902 near the top end of the in vivo portion or the tower 11910 of the sensor to the rest of the bend 11920c. In addition to reducing the overall footprint of the sensor, this orientation can provide additional flexibility in the horizontally-oriented axis. The arrangement can provide multiple overlapping, horizontally-aligned vertical layers between the turns.

FIG. 11G illustrates another embodiment of a sensor 11900 including a neck between the ex vivo portion 11904 and in vivo portion 11902 with a bend 11920d including turn 11927a, 11927b, and 11927c. In the illustrated embodiment, the bend 11920d occurs primarily in the in vivo portion 11902 of the sensor, connecting the in vivo portion 11902 and the tower 11910, while the stretch of the sensor between the tower 11910 and the ex vivo portion 11904 is generally uninterrupted. The turn 11927a generally connects the tower 11910 to the rest of the bend 11920d, while the turn 11927c connects the in vivo portion 11902 to the rest of the bend 11920d. This orientation can provide additional flexibility in the vertically-oriented axis. The arrangement can provide multiple overlapping, horizontally-aligned vertical layers between the turns.

The turns of the neck can be formed by folding or bending the neck of the sensor from a larger neck structure, laser cutting the sensor from a sheet of the material or layers of material comprising the sensor, printing the sensor having the configuration with turns from a sheet of the material or layers of material of which the sensor is composed, stamping the sensor from a sheet of material or layers of material of which the sensor is composed, or other suitable manufacturing processes for providing precision bends in the neck.

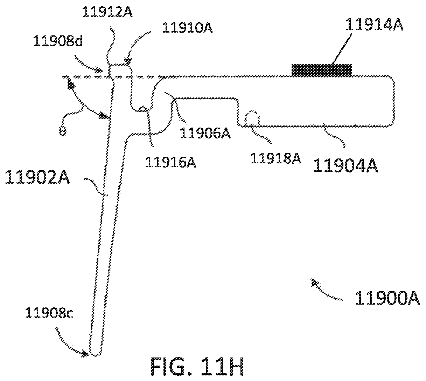

Referring to FIG. 11H, sensor 11900A can include each of the features of sensor 11900 including neck 11906A, bottom end 11908c, top end 11908d, tower 11910A, protrusion 11912A, dip or bend 11916A (or alternatively, dips or bends similar to 11920a-11920d as depicted in Figs. 11D-11G), and notch 11918A. Sensor 11900A can also include ex vivo portion 11904A and in vivo portion 11902A. Sensor 11900A can include electronic components 11914A directly mounted to ex vivo portion 2404A, for example, by using photonic soldering, as described in greater detail below. Therefore, the need for sensor contacts 11914 can be eliminated in sensor 11900A.

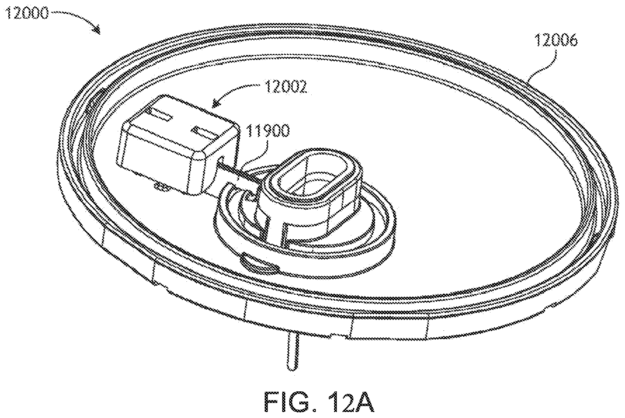

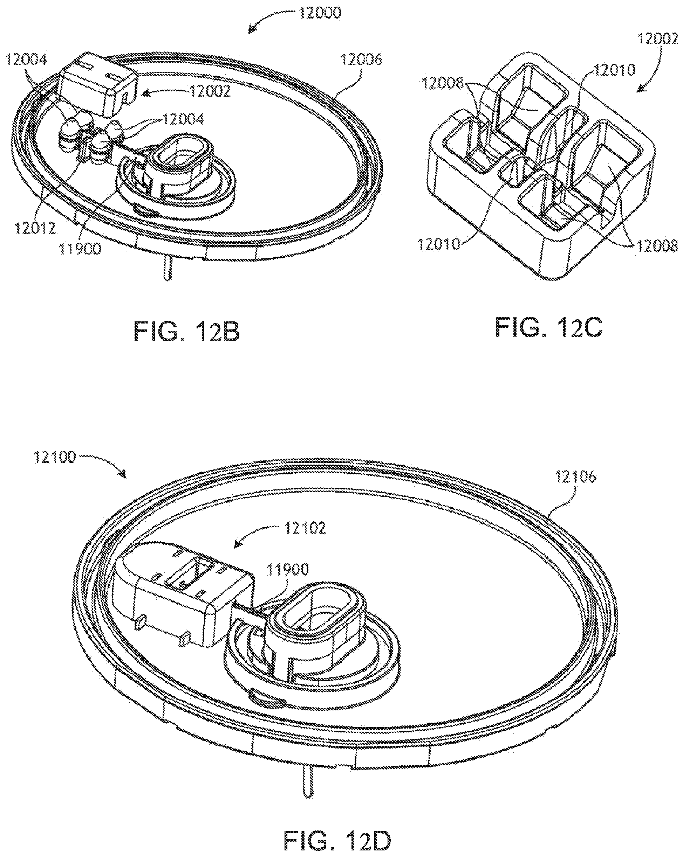

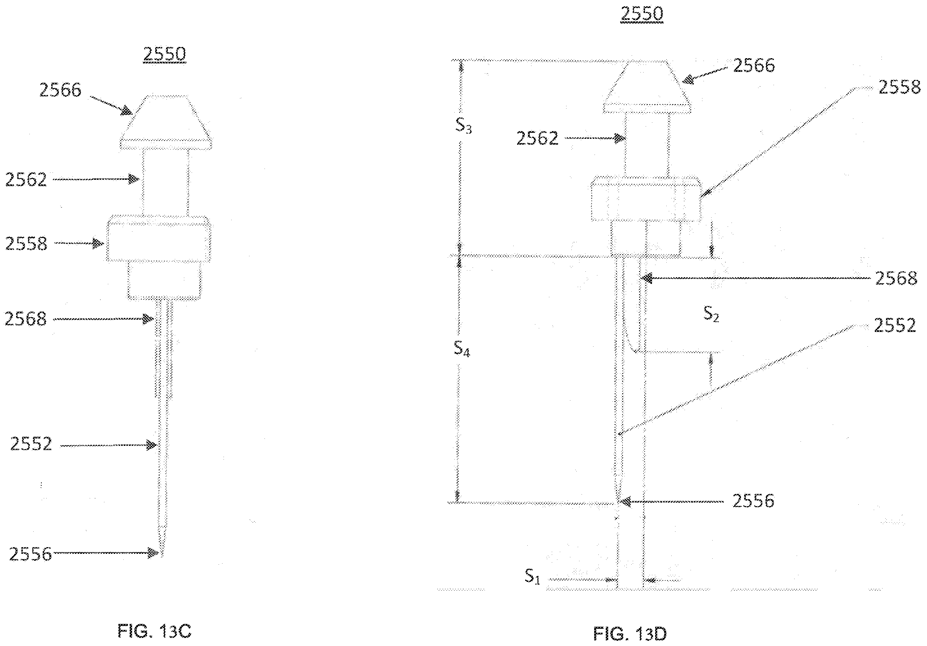

FIGS. 12A and 12B are isometric and partially exploded isometric views of an example connector assembly 12000, according to one or more embodiments. As illustrated, the connector assembly 12000 may include a connector 12002, and FIG. 13C is an isometric bottom view of the connector 12002. The connector 12002 may comprise an injection molded part used to help secure one or more compliant carbon impregnated polymer modules 12004 (four shown in FIG. 12B) to a mount 12006. More specifically, the connector 12002 may help secure the modules 12004 in place adjacent the sensor 11900 and in contact with the sensor contacts 11914 (FIG. 11C) provided on the ex vivo portion 11904 (FIG. 11C). The modules 12004 may be made of a conductive material to provide conductive communication between the sensor 11900 and corresponding circuitry contacts (not shown) provided within the mount 12006.

As best seen in FIG. 12C, the connector 12002 may define pockets 12008 sized to receive the modules 12004. Moreover, in some embodiments, the connector 12002 may further define one or more depressions 12010 configured to mate with one or more corresponding flanges 12012 (FIG. 12B) on the mount 12006. Mating the depressions 12010 with the flanges 12012 may secure the connector 12002 to the mount 12006 via an interference fit or the like. In other embodiments, the connector 12002 may be secured to the mount 12006 using an adhesive or via sonic welding.

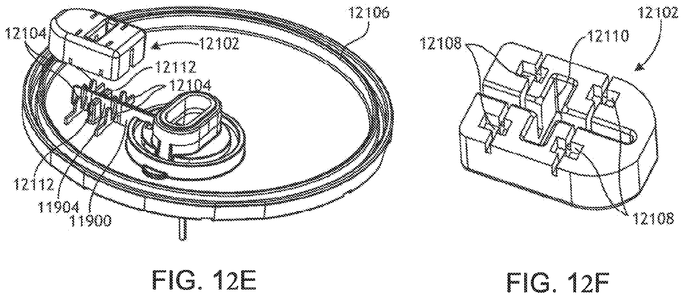

FIGS. 12D and 12E are isometric and partially exploded isometric views of another example connector assembly 12100, according to one or more embodiments. As illustrated, the connector assembly 12100 may include a connector 12102, and FIG. 12F is an isometric bottom view of the connector 12102. The connector 12102 may comprise an injection molded part used to help keep one or more compliant metal contacts 12104 (four shown in FIG. 12E) secured against the sensor 11900 on a mount 12106. More specifically, the connector 12102 may help secure the contacts 12104 in place adjacent the sensor 11900 and in contact with the sensor contacts 11914 (FIG. 11C) provided on the ex vivo portion 11904. The contacts 12104 may be made of a stamped conductive material that provides conductive communication between the sensor 11900 and corresponding circuitry contacts (not shown) provided within the mount 12106. In some embodiments, for example, the contacts 12104 may be soldered to a PCB (not shown) arranged within the mount 12106.

As best seen in FIG. 12F, the connector 12102 may define pockets 12108 sized to receive the contacts 12104. Moreover, in some embodiments, the connector 12102 may further define one or more depressions 12110 configured to mate with one or more corresponding flanges 12112 (FIG. 80B) on the mount 12006. Mating the depressions 12110 with the flanges 12112 may help secure the connector 12102 to the mount 12106 via an interference fit or the like. In other embodiments, the connector 12102 may be secured to the mount 12106 using an adhesive or via sonic welding.

### Example Embodiments of Sharp Modules

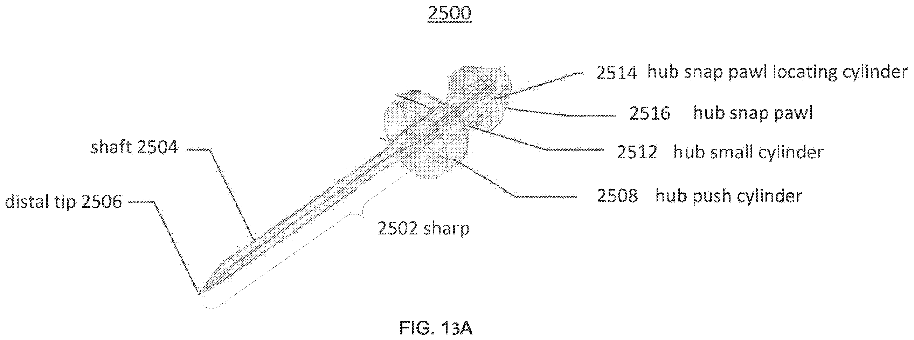

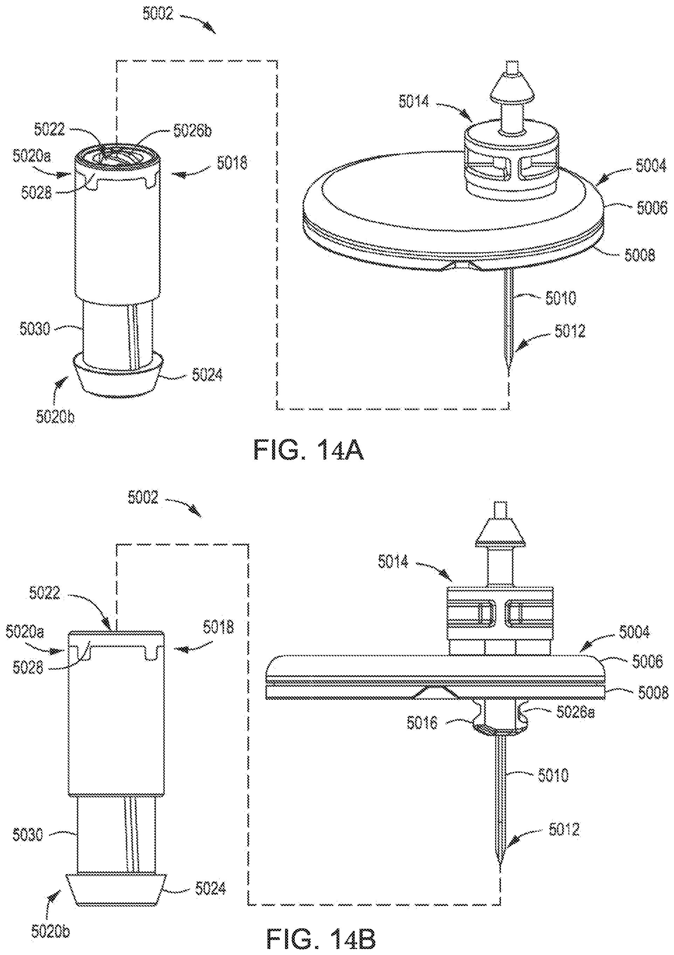

FIG. 13A is a perspective view depicting an example embodiment of sharp module 2500 prior to assembly within sensor module 504 (FIG. 6B). Sharp 2502 can include a distal tip 2506 which can penetrate the skin while carrying sensor in vivo portion in a hollow or recess of sharp shaft 2504 to put the active surface of the sensor in vivo portion into contact with bodily fluid. A hub push cylinder 2508 can provide a surface for a sharp carrier to push during insertion. A hub small cylinder 2512 can provide a space for the extension of sharp hub contact faces 1622 (FIG. 14B). A hub snap pawl locating cylinder 2514 can provide a distal-facing surface of hub snap pawl 2516 for sharp hub contact faces 1622 to abut. A hub snap pawl 2516 can include a conical surface that opens clip 1620 during installation of sharp module 2500.

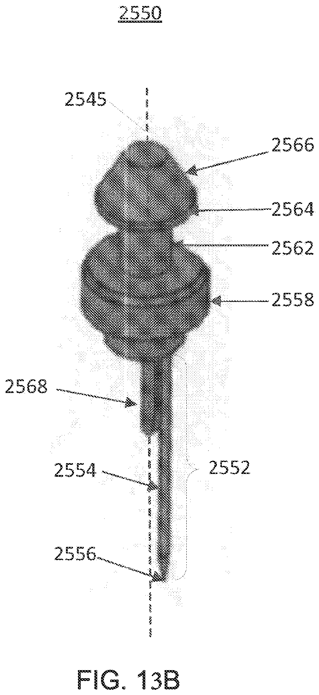

FIGS. 13B to 13H show example embodiments of sharp modules, in various stages of assembly, for use in the insertion of dermal analyte sensors. According to one aspect of the embodiments, angling the sensor and/or insertion sharp relative to a reference point can enable co-localization of the tip of the insertion needle and the tip of the sensor, and furthermore, can create a single contact point at the surface of the skin. As such, the sharp can create a leading edge at the surface of the skin to form an insertion path into the dermal layer for the sensor, as the sensor is inserted into a subject. In some embodiments, for example, the sharp and/or dermal sensor may be angled relative to a reference point (e.g., each other, surface of the skin, or the base of the applicator) for insertion, where the angle of the sharp differs from the angle of the sensor. For example, the reference point may be the skin surface to be breached for dermal insertion, or may be a reference or component of the sensor applicator set. In some embodiments, the sharp may be disposed at an angle relative to the sensor. For example, when designed so that that the sharp is angled relative to the sensor, the needle creates a leading edge for the sensor during operation of the applicator set. Furthermore, the needle design itself, and the positioning of the needle with respect to the sensor can be implemented in any desired configuration, including all of those configurations disclosed in U.S. Patent Publication No. 2014/0171771.

Furthermore, although many of the example embodiments described with respect to FIGS. 13B to 13J make reference to dermal analyte sensors and dermal insertion, it will be understood by those of skill in the art that any of the embodiments can be dimensioned and configured for use with analyte sensors that can be positioned beyond the dermal space, such as into (or even fully through) subcutaneous tissue (e.g., 3 mm to 10 mm beneath the surface of the skin depending on the location of the skin on the body).

FIG. 13B is a perspective view depicting an example embodiment of a sharp module 2550 that can be used for the insertion of a dermal sensor. Sharp module 2550 is shown here prior to assembly with sensor module 504 (FIG. 6B), and can include components similar to those of the embodiment described with respect to FIG. 13A, including sharp 2552, sharp shaft 2554, sharp distal tip 2556, hub push cylinder 2558, hub small cylinder 2562, hub snap pawl 2566 and hub snap pawl locating cylinder 2564. Sharp 2552 can be positioned within sharp module 2550 at an off-center location relative to a longitudinal axis 2545 that extends through center of hub snap pawl 2566, hub small cylinder 2562 and hub push cylinder 2558. In addition, sharp module 2550 can include a sharp spacer 2568 that is parallel to and adjacent with a portion of sharp 2552. Sharp spacer 2568 can be positioned in between sensor 104 (not shown) and sharp 2552 along a proximal portion of sharp 2552, and can ensure that sensor 104 and sharp 2552 remain spaced apart at a proximal portion of sharp 2552. Sharp 2552 can be positioned in an off-center location during a molding process with hub components 2558, 2562, 2566, each of which may consist of a rigid plastic material.

FIGS. 13C and 13D are two side views depicting sharp module 2550 prior to assembly with sensor module 504 (FIG. 6B), and include sharp 2552, spacer 2568, hub push cylinder 2558, hub small cylinder 2562 and hub snap pawl 2566. In some embodiments, the relative distances between the sharp 2552 and hub components can be positioned as follows. For example, distance, Si, between the sharp 2552 and the radial center of hub can range from 0.50 mm to 1 mm (e.g., 0.89 mm). Height, S₂, of sharp spacer 2568 can range from 3 to 5 mm (e.g., 3.26 mm). Height, S3, of hub can range from 5 to 10 mm (e.g., 6.77 mm). Length, S₄, of sharp 2552 can range from 1.5 mm to 25 mm (e.g., 8.55 mm), and may depend on the location of the insertion site on the subject.

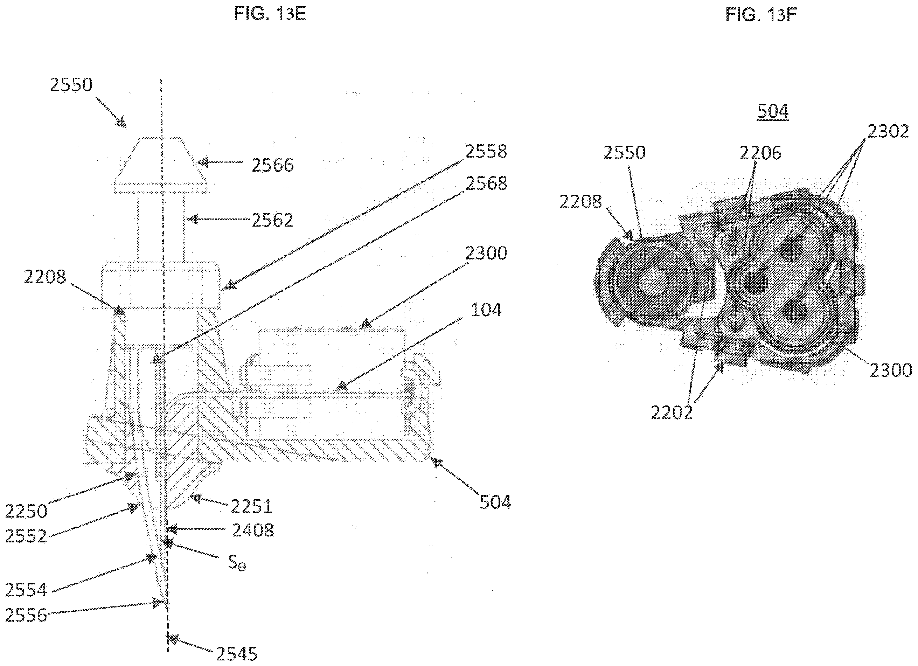

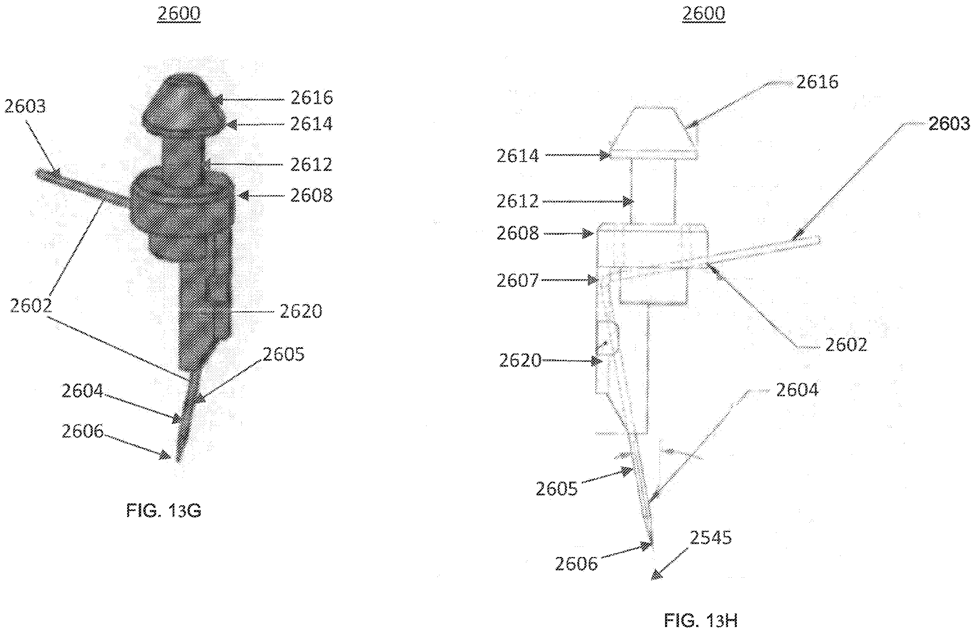

FIG. 13E depicts a side cross-sectional side view of sharp module 2550, including sharp 2552, sharp spacer 2568 and hub components (hub snap pawl 2566, hub small cylinder 2562, and hub push cylinder 2558), as assembled with sensor module 504. As can be seen in FIG. 13E, sharp 2552 is positioned within sharp slot 2208 of sensor module 504 that includes a curved interior surface 2250, located at a distal end. Curved interior surface 2250 of sensor module 504 can be in contact with a portion of sharp 2552 and cause a deflection such that sharp distal tip 2556 is oriented toward central longitudinal axis 2545. As best seen in FIG. 13H, sharp 2552 can be positioned such that the distal portion and central longitudinal axis 2545 form an acute angle, So, that can range between 5° and 20°. In some embodiments, for example, So, can range from 5° to 17°, or 7° to 15°, or 9° to 13°, e.g., 9°, 10°, 11°, 12°, or 13°

Referring still to FIG. 13E, near a distal end of sensor module 504 is protrusion 2251, which can enhance the perfusion of bodily fluid, such as dermal fluid. Although shown as a curved surface in FIG. 13E, protrusion 2251 can be shaped in any desired fashion. In addition, in some embodiments, multiple protrusions can be present. U.S. Patent Publication No. 2014/0275907 describes sensor devices having different protrusion configurations, each of which can be implemented with the embodiments described herein. Many of the embodiments described herein show the needle exiting from the protrusion, and in other embodiments, the needle can exit from the base of the sensor device adjacent the protrusion, and from that position extend over the tip of sensor 104.

Referring still to FIGS. 13E and 13F, sensor 104 can be a dermal sensor and can include sensor in vivo portion 2408, located at a distal end of sensor 104, and which can be positioned in a substantially parallel orientation to central longitudinal axis 2545. Distal end of sensor in vivo portion 2408 can be proximal to distal sharp tip 2556, either in a spaced relation with, at rest in, or at rest against a portion of sharp shaft 2554. As further depicted in FIG. 13E, sharp spacer 2568 provides a spaced relation between a proximal portion of sharp 2552 and sensor 104, such that the proximal portion of sharp 2552 and sensor 104 are not in contact. Sensor module 504 can further include sensor connector 2300 for housing a proximal portion of sensor 104 that is relatively perpendicular to a distal end of sensor 104.

FIG. 13F is a top-down cross-sectional view of sensor module 504. Sensor module 504 can include one or more sensor module snaps 2202 for coupling with a housing (not shown) of sensor control device 102. Sensor module 504 can also include sensor connector 2300, which can have sensor contacts 2302 for coupling with a proximal portion of sensor 104. Sensor connector 2300 can be made of silicone rubber that encapsulates compliant carbon impregnated polymer modules that serve as electrical conductive contacts 2302 between sensor 104 and electrical circuitry contacts for the electronics within sensor control device 102. The connector can also serve as a moisture barrier for sensor 104 when assembled in a compressed state after transfer from a container to an applicator and after application to a user's skin. Although three contacts 2302 are depicted, it should be understood that connector 2300 can have fewer contacts (e.g., two) or more contacts (e.g., four, five, six, etc.), depending on the particular type or configuration of sensor 104. Sensor connector 2300 can be further coupled with sensor module 504 by two connector posts 2206 positioned through a like number of apertures in connector 2300. Although two connector posts 2206 are depicted, it should be understood that any number of connector posts 2206 can be used to couple connector 2300 to sensor module 504.

FIGS. 13G and 13H are, respectively, a perspective view and a side view of another example embodiment of sharp module 2600 that can be used for the insertion of a dermal sensor. Sharp module 2600 is shown here prior to assembly with sensor module 504 (FIG. 6B), and can include components similar to those of the embodiments described with respect to FIGS. 13A and 13B, including sharp 2602, sharp shaft 2604, sharp distal tip 2606, hub push cylinder 2608, hub small cylinder 2612, hub snap pawl 2616 and hub snap pawl locating cylinder 2614. In some embodiments, sharp 2602 can be a "pre-bent" needle that includes a proximal portion 2603 that originates from a point external to sharp module 2600 and intersects, at an angle, a central point of the hub (e.g., through hub push cylinder 2608). Sharp 2602 can also include a distal portion 2605 that extends in a distal direction, at an angle, from a point near a distal portion of hub toward the insertion point of the user's skin. As shown in FIG. 13H, sharp 2602 can include an angled portion 2607 located external to hub push cylinder 2608, which can have a substantially 90° angle between proximal portion 2603 and distal portion 2605 of sharp 2602. Sharp module 2600 can also include a bend fin guide 2620 for maintaining "pre-bent" sharp 2602 in position during assembly and/or use, and can prevent lateral or rotational movement of sharp 2602 relative to hub components. Proximal portion 2603 of sharp 2602 can be "trimmed" from the hub after molding process is completed, and prior to assembly of sharp module 2600 with sensor module 504.

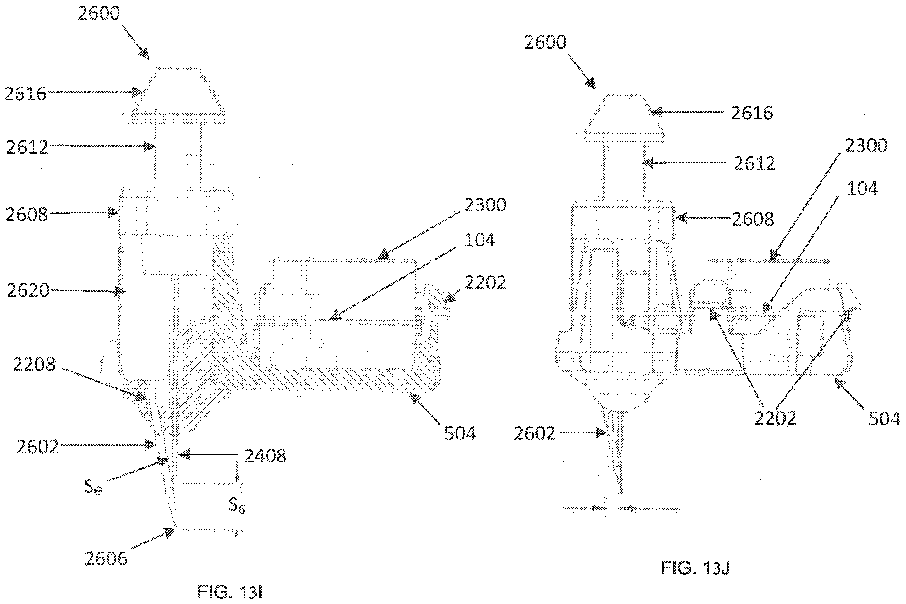

FIGS. 13I and 13J show, respectively, a side cross-sectional view and a side view of sharp module 2600 (including hub snap pawl 2616, hub small cylinder 2612, and hub push cylinder 2608), as assembled with sensor module 504. As can be seen in FIG. 13I, sensor module 504 includes sharp slot 2208, through which sharp 2602 can extend in an angled and distal direction. As described earlier, a proximal portion of sharp 2602 passes through bend fin guide 2620, which is coupled with a distal portion of sensor module 504. Sensor module 504 can also include sensor 104, which can be a dermal sensor. As seen in FIG. 13I, sharp 2602 and sensor in vivo portion 2408 can form an acute angle, So, at a point where their respective longitudinal axes converge. Angle So can range between 5° and 20°. In some embodiments, for example, So, can range from 5° to 17°, or 7° to 15°, or 9° to 13°, e.g., 9°, 10°, 11°, 12°, or 13° In some embodiments, distal sharp tip 2606 is located at a distance, S₆, that is proximal to an end of sensor in vivo portion 2408. Distance, S₆, can range between 0.02 mm to 0.10 mm, e.g., 0.05 mm, 0.06 mm or 0.07 mm.

Referring still to FIGS. 13I and 13J, sensor module 504 can also include sensor connector 2300 for housing a proximal portion of sensor 104 that is relatively perpendicular to a distal end of sensor 104. Sensor module 504 can further include one or more sensor module snaps 2202 for coupling with a housing (not shown) of sensor control device 102. Sensor connector 2300 can include the same structures described with respect to FIG. 13F.

In the above embodiments, the sharp can be made of stainless steel or a like flexible material (e.g., material used to manufacture acupuncture needles), and dimensioned such that the applicator provides for insertion of at least a portion of the dermal sensor into the dermal layer, but not through the dermal layer of the skin. According to certain embodiments, the sharp has a cross sectional diameter (width) of from 0.1 mm to 0.5 mm. For example, the sharp may have a diameter of from 0.1 mm to 0.3 mm, such as from 0.15 mm to 0.25 mm, e.g., 0.16 mm to 0.22 mm in diameter. A given sharp may have a constant, i.e., uniform, width along its entire length, or may have a varying, i.e., changing, width along at least a portion of its length, such as the tip portion used to pierce the surface of the skin. For example, with respect to the embodiment shown in FIG. 13I, width of sharp 2602 can narrow along a distal portion between bend fin guide 1620 and distal sharp tip 2606.

A sharp can also have a length to insert a dermal sensor just into the dermal layer, and no more. Insertion depth may be controlled by the length of the sharp, the configuration of the base and/or other applicator components that limit insertion depth. A sharp may have a length between 1.5 mm and 25 mm. For example, the sharp may have a length of from 1 mm to 3 mm, from 3 mm to 5 mm, from 5 mm to 7 mm, from 7 mm to 9 mm, from 9 mm to 11 mm, from 11 mm to 13 mm, from 13 mm to 15 mm, from 15 mm to 17 mm, from 17 mm to 19 mm, from 19 mm to 21 mm, from 21 mm to 23 mm, from 23 mm to 25 mm, or a length greater than 25 mm. It will be appreciated that while a sharp may have a length up to 25 mm, in certain embodiments the full length of the sharp is not inserted into the subject because it would extend beyond the dermal space. Non-inserted sharp length may provide for handling and manipulation of the sharp in an applicator set. Therefore, while a sharp may have a length up to 25 mm, the insertion depth of the sharp in the skin on a subject in those certain embodiments will be limited to the dermal layer, e.g., about 1.5 mm to 4 mm, depending on the skin location, as described in greater detail below. However, in all of the embodiments disclosed herein, the sharp can be configured to extend beyond the dermal space, such as into (or even fully through) subcutaneous tissue (e.g., 3 mm to 10 mm beneath the surface of the skin depending on the location of the skin on the body). Additionally, in some example embodiments, the sharps described herein can include hollow or partially hollow insertion needles, having an internal space or lumen. In other embodiments, however, the sharps described herein can include solid insertion needles, which do not have an internal space and/or lumen. Furthermore, a sharp of the subject applicator sets can also be bladed or non-bladed.

Likewise, in the above embodiments, a dermal sensor is sized so that at least a portion of the sensor is positioned in the dermal layer and no more, and a portion extends outside the skin in the transcutaneously positioned embodiments. That is, a dermal sensor is dimensioned such that when the dermal sensor is entirely or substantially entirely inserted into the dermal layer, the distal-most portion of the sensor (the insertion portion or insertion length) is positioned within the dermis of the subject and no portion of the sensor is inserted beyond a dermal layer of the subject when the sensor is operably dermally positioned.

The dimensions (e.g., the length) of the sensor may be selected according to the body site of the subject in which the sensor is to be inserted, as the depth and thickness of the epidermis and dermis exhibit a degree of variability depending on skin location. For example, the epidermis is only about 0.05 mm thick on the eyelids, but about 1.5 mm thick on the palms and the soles of the feet. The dermis is the thickest of the three layers of skin and ranges from about 1.5 mm to 4 mm thick, depending on the skin location. For implantation of the distal end of the sensor into, but not through, the dermal layer of the subject, the length of the inserted portion of the dermal sensor should be greater than the thickness of the epidermis, but should not exceed the combined thickness of the epidermis and dermis. Methods may include determining an insertion site on a body of a user and determining the depth of the dermal layer at the site, and selecting the appropriately-sized applicator set for the site.

In certain aspects, the sensor is an elongate sensor having a longest dimension (or "length") of from 0.25 mm to 4 mm. The length of the sensor that is inserted, in the embodiments in which only a portion of a sensor is dermally inserted, ranges from 0.5 mm to 3 mm, such as from 1 mm to 2 mm, e.g., 1.5 mm. The dimensions of the sensor may also be expressed in terms of its aspect ratio. In certain embodiments, a dermal sensor has an aspect ratio of length to width (diameter) of about 30:1 to about 6:1. For example, the aspect ratio may be from about 25:1 to about 10:1, including 20:1 and 15:1. The inserted portion of a dermal sensor has sensing chemistry.

However, all of the embodiments disclosed herein can be configured such that at least a portion of the sensor is positioned beyond the dermal layer, such as into (or through) the subcutaneous tissue (or fat). For example, the sensor can be dimensioned such that when the sensor is entirely or substantially entirely inserted into the body, the distal-most portion of the sensor (the insertion portion or insertion length) is positioned within the subcutaneous tissue (beyond the dermis of the subject) and no portion of the sensor is inserted beyond the subcutaneous tissue of the subject when the sensor is operably positioned. As mentioned, the subcutaneous tissue is typically present in the region that is 3 mm to 10 mm beneath the outer skin surface, depending on the location of the skin on the body.

### Example Embodiments of Applicators and Sensor Control Devices for One Piece Architectures

Referring briefly again to FIGS. 1 and 3A-3G, for the two-piece architecture system, the sensor tray 202 and the sensor applicator 102 are provided to the user as separate packages, thus requiring the user to open each package and finally assemble the system. In some applications, the discrete, sealed packages allow the sensor tray 202 and the sensor applicator 102 to be sterilized in separate sterilization processes unique to the contents of each package and otherwise incompatible with the contents of the other. More specifically, the sensor tray 202, which includes the plug assembly 207, including the sensor 110 and the sharp 220, may be sterilized using radiation sterilization, such as electron beam (or "e-beam") irradiation. Radiation sterilization, however, can damage the electrical components arranged within the electronics housing of the sensor control device 102. Consequently, if the sensor applicator 102, which contains the electronics housing of the sensor control device 102, needs to be sterilized, it may be sterilized via another method, such as gaseous chemical sterilization using, for example, ethylene oxide. Gaseous chemical sterilization, however, can damage the enzymes or other chemistry and biologies included on the sensor 110. Because of this sterilization incompatibility, the sensor tray 202 and the sensor applicator 102 are commonly sterilized in separate sterilization processes and subsequently packaged separately, which requires the user to finally assemble the components for use.

According to embodiments of the present disclosure, the sensor control device 102 may be modified to provide a one-piece architecture that may be subjected to sterilization techniques specifically designed for a one-piece architecture sensor control device. A one-piece architecture allows the sensor applicator 150 and the sensor control device 102 to be shipped to the user in a single, sealed package that does not require any final user assembly steps. Rather, the user need only open one package and subsequently deliver the sensor control device 102 to the target monitoring location. The one-piece system architecture described herein may prove advantageous in eliminating component parts, various fabrication process steps, and user assembly steps. As a result, packaging and waste are reduced, and the potential for user error or contamination to the system is mitigated.

FIGS. 14A and 14B are isometric and side views, respectively, of another example sensor control device 5002, according to one or more embodiments of the present disclosure. The sensor control device 5002 may be similar in some respects to the sensor control device 102 of FIG. 1 and therefore may be best understood with reference thereto. Moreover, the sensor control device 5002 may replace the sensor control device 102 of FIG. 1 and, therefore, may be used in conjunction with the sensor applicator 102 of FIG. 1, which may deliver the sensor control device 5002 to a target monitoring location on a user's skin.

Unlike the sensor control device 102 of FIG. 1, however, the sensor control device 5002 may comprise a one-piece system architecture not requiring a user to open multiple packages and finally assemble the sensor control device 5002 prior to application. Rather, upon receipt by the user, the sensor control device 5002 may already be fully assembled and properly positioned within the sensor applicator 150 (FIG. 1). To use the sensor control device 5002, the user need only open one barrier (e.g., the applicator cap 708 of FIG. 3B) before promptly delivering the sensor control device 5002 to the target monitoring location for use.

As illustrated, the sensor control device 5002 includes an electronics housing 5004 that is generally disc-shaped and may have a circular cross-section. In other embodiments, however, the electronics housing 2004 may exhibit other cross-sectional shapes, such as ovoid or polygonal, without departing from the scope of the disclosure. The electronics housing 5004 may be configured to house or otherwise contain various electrical components used to operate the sensor control device 5002. In at least one embodiment, an adhesive patch (not shown) may be arranged at the bottom of the electronics housing 5004. The adhesive patch may be similar to the adhesive patch 105 of FIG. 1, and may thus help adhere the sensor control device 5002 to the user's skin for use.

As illustrated, the sensor control device 5002 includes an electronics housing 5004 that includes a shell 5006 and a mount 5008 that is matable with the shell 5006. The shell 5006 may be secured to the mount 5008 via a variety of ways, such as a snap fit engagement, an interference fit, sonic welding, one or more mechanical fasteners (e.g., screws), a gasket, an adhesive, or any combination thereof. In some cases, the shell 5006 may be secured to the mount 5008 such that a sealed interface is generated therebetween.

The sensor control device 5002 may further include a sensor 5010 (partially visible) and a sharp 5012 (partially visible), used to help deliver the sensor 5010 transcutaneously under a user's skin during application of the sensor control device 5002. As illustrated, corresponding portions of the sensor 5010 and the sharp 5012 extend distally from the bottom of the electronics housing 5004 (e.g., the mount 5008). The sharp 5012 may include a sharp hub 5014 configured to secure and carry the sharp 5012. As best seen in FIG. 14B, the sharp hub 5014 may include or otherwise define a mating member 5016. To couple the sharp 5012 to the sensor control device 5002, the sharp 5012 may be advanced axially through the electronics housing 5004 until the sharp hub 5014 engages an upper surface of the shell 5006 and the mating member 5016 extends distally from the bottom of the mount 5008. As the sharp 5012 penetrates the electronics housing 5004, the exposed portion of the sensor 5010 may be received within a hollow or recessed (arcuate) portion of the sharp 5012. The remaining portion of the sensor 5010 is arranged within the interior of the electronics housing 5004.

The sensor control device 5002 may further include a sensor cap 5018, shown exploded or detached from the electronics housing 5004 in FIGS. 14A-14B. The sensor cap 5016 may be removably coupled to the sensor control device 5002 (e.g., the electronics housing 5004) at or near the bottom of the mount 5008. The sensor cap 5018 may help provide a sealed barrier that surrounds and protects the exposed portions of the sensor 5010 and the sharp 5012 from gaseous chemical sterilization. As illustrated, the sensor cap 5018 may comprise a generally cylindrical body having a first end 5020a and a second end 5020b opposite the first end 5020a. The first end 5020a may be open to provide access into an inner chamber 5022 defined within the body. In contrast, the second end 5020b may be closed and may provide or otherwise define an engagement feature 5024. As described herein, the engagement feature 5024 may help mate the sensor cap 5018 to the cap (e.g., the applicator cap 708 of FIG. 3B) of a sensor applicator (e.g., the sensor applicator 150 of FIGS. 1 and 3A-3G), and may help remove the sensor cap 5018 from the sensor control device 5002 upon removing the cap from the sensor applicator.

The sensor cap 5018 may be removably coupled to the electronics housing 5004 at or near the bottom of the mount 5008. More specifically, the sensor cap 5018 may be removably coupled to the mating member 5016, which extends distally from the bottom of the mount 5008. In at least one embodiment, for example, the mating member 5016 may define a set of external threads 5026a (FIG. 14B) matable with a set of internal threads 5026b (FIG. 14A) defined by the sensor cap 5018. In some embodiments, the external and internal threads 5026a, b may comprise a flat thread design (e.g., lack of helical curvature), which may prove advantageous in molding the parts. Alternatively, the external and internal threads 5026a,b may comprise a helical threaded engagement. Accordingly, the sensor cap 5018 may be threadably coupled to the sensor control device 5002 at the mating member 5016 of the sharp hub 5014. In other embodiments, the sensor cap 5018 may be removably coupled to the mating member 5016 via other types of engagements including, but not limited to, an interference or friction fit, or a frangible member or substance that may be broken with minimal separation force (e.g., axial or rotational force).

In some embodiments, the sensor cap 5018 may comprise a monolithic (singular) structure extending between the first and second ends 5020a, b. In other embodiments, however, the sensor cap 5018 may comprise two or more component parts. In the illustrated embodiment, for example, the sensor cap 5018 may include a seal ring 5028 positioned at the first end 5020a and a desiccant cap 5030 arranged at the second end 5020b. The seal ring 5028 may be configured to help seal the inner chamber 5022, as described in more detail below. In at least one embodiment, the seal ring 5028 may comprise an elastomeric O-ring. The desiccant cap 5030 may house or comprise a desiccant to help maintain preferred humidity levels within the inner chamber 5022. The desiccant cap 5030 may also define or otherwise provide the engagement feature 5024 of the sensor cap 5018.

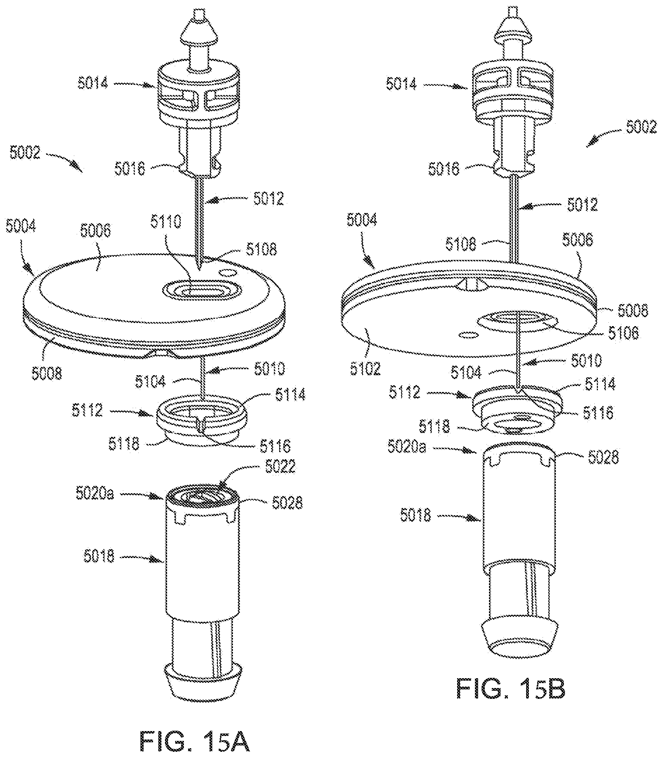

FIGS. 15A and 15B are exploded isometric top and bottom views, respectively, of the sensor control device 5002, according to one or more embodiments. The shell 5006 and the mount 5008 operate as opposing clamshell halves that enclose or otherwise substantially encapsulate various electronic components of the sensor control device 5002. More specifically, electronic components may include, but are not limited to, a printed circuit board (PCB), one or more resistors, transistors, capacitors, inductors, diodes, and switches. A data processing unit and one or more batteries may be mounted to or otherwise interact with the PCB. The data processing unit may comprise, for example, an application specific integrated circuit (ASIC) configured to implement one or more functions or routines associated with operation of the sensor control device 5002. More specifically, the data processing unit may be configured to perform data processing functions, where such functions may include, but are not limited to, filtering and encoding of data signals, each of which corresponds to a sampled analyte level of the user. The data processing unit may also include or otherwise communicate with one or more antennas for communicating with the reader device 120 (FIG. 1). The one or more batteries may provide power to the sensor control device 5002 and, more particularly, to the electronic components of the PCB. While not shown, the sensor control device 5002 may also include an adhesive patch that may be applied to the bottom 5102 (FIG. 15B) of the mount 5008, and may help adhere the sensor control device 5002 to the user's skin for use.

The sensor control device 5002 may provide or otherwise include a sealed subassembly that includes, among other component parts, the shell 5006, the sensor 5010, the sharp 5012, and the sensor cap 5018. The sealed subassembly of the sensor control device 5002 may help isolate the sensor 5010 and the sharp 5012 within the inner chamber 5022 (FIG. 15A) of the sensor cap 5018 during a gaseous chemical sterilization process, which might otherwise adversely affect the chemistry provided on the sensor 5010.

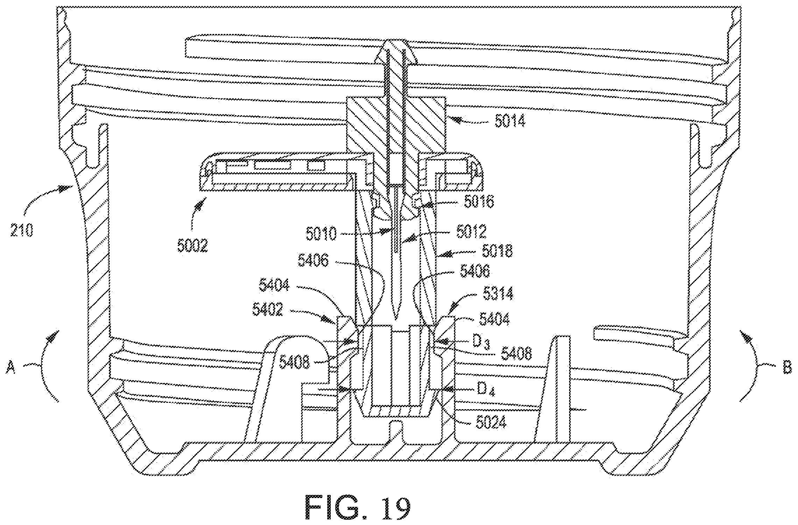

The sensor 5010 may include a in vivo portion 5104 that extends out an aperture 5106 (FIG. 15B) defined in the mount 5008 to be transcutaneously received beneath a user's skin. The in vivo portion 5104 may have an enzyme or other chemistry included thereon to help facilitate analyte monitoring. The sharp 5012 may include a sharp tip 5108 extendable through an aperture 5110 (FIG. 19 A) defined by the shell 5006, and the aperture 5110 may be coaxially aligned with the aperture 5106 of the mount 5008. As the sharp tip 5108 penetrates the electronics housing 5004, the in vivo portion 5104 of the sensor 5010 may be received within a hollow or recessed portion of the sharp tip 5108. The sharp tip 5108 may be configured to penetrate the skin while carrying the in vivo portion 5104 to put the active chemistry of the in vivo portion 5104 into contact with bodily fluids.

The sharp tip 5108 may be advanced through the electronics housing 5004 until the sharp hub 5014 engages an upper surface of the shell 5006 and the mating member 5016 extends out the aperture 5106 in the bottom 5102 of the mount 5008. In some embodiments, a seal member (not shown), such as an O-ring or seal ring, may interpose the sharp hub 5014 and the upper surface of the shell 5006 to help seal the interface between the two components. In some embodiments, the seal member may comprise a separate component part, but may alternatively form an integral part of the shell 5006, such as being a co-molded or overmolded component part.

The sealed subassembly may further include a collar 5112 that is positioned within the electronics housing 5004 and extends at least partially into the aperture 5106. The collar 5112 may be a generally annular structure that defines or otherwise provides an annular ridge 5114 on its top surface. In some embodiments, as illustrated, a groove 5116 may be defined in the annular ridge 5114 and may be configured to accommodate or otherwise receive a portion of the sensor 5010 extending laterally within the electronics housing 5004.

In assembling the sealed subassembly, a bottom 5118 of the collar 5112 may be exposed at the aperture 5106 and may sealingly engage the first end 5020a of the sensor cap 5018 and, more particularly, the seal ring 5028. In contrast, the annular ridge 5114 at the top of the collar 5112 may sealingly engage an inner surface (not shown) of the shell 5006. In at least one embodiment, a seal member (not shown) may interpose the annular ridge 5114 and the inner surface of the shell 5006 to form a sealed interface. In such embodiments, the seal member may also extend (flow) into the groove 5116 defined in the annular ridge 5114 and thereby seal about the sensor 5010 extending laterally within the electronics housing 5004. The seal member may comprise, for example, an adhesive, a gasket, or an ultrasonic weld, and may help isolate the enzymes and other chemistry included on the in vivo portion 5104.

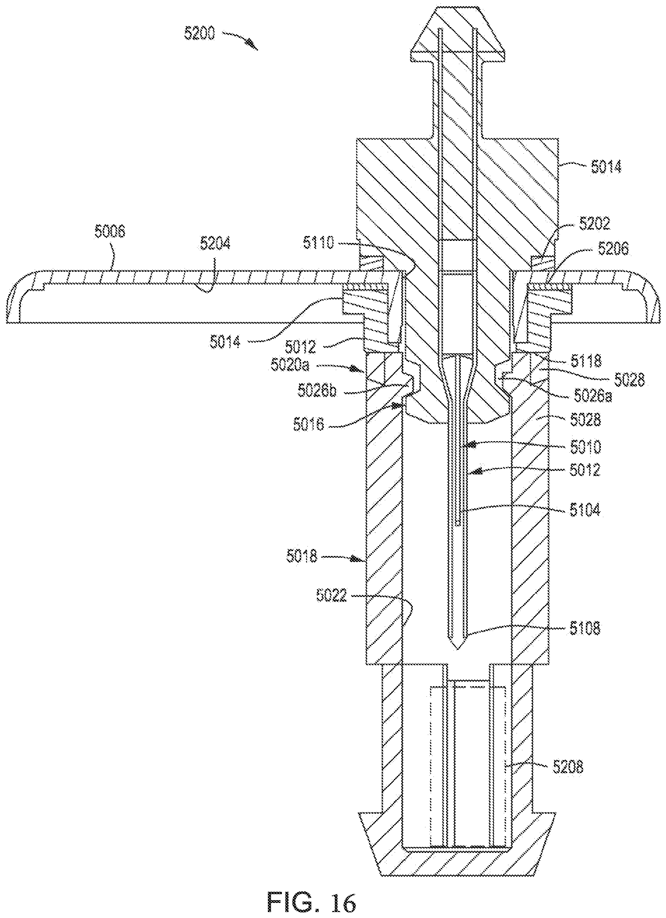

FIG. 16 is a cross-sectional side view of an assembled sealed subassembly 5200, according to one or more embodiments. The sealed subassembly 5200 may form part of the sensor control device 5002 of FIGS. 14A-14B and 15A-16B and may include portions of the shell 5006, the sensor 5010, the sharp 5012, the sensor cap 5018, and the collar 5112. The sealed subassembly 5200 may be assembled in a variety of ways. In one assembly process, the sharp 5012 may be coupled to the sensor control device 5002 by extending the sharp tip 5108 through the aperture 5110 defined in the top of the shell 5006 and advancing the sharp 5012 through the shell 5006 until the sharp hub 5014 engages the top of the shell 5006 and the mating member 196 extends distally from the shell 5006. In some embodiments, as mentioned above, a seal member 5202 (e.g., an O- ring or seal ring) may interpose the sharp hub 5014 and the upper surface of the shell 5006 to help seal the interface between the two components.

The collar 5112 may then be received over (about) the mating member 5016 and advanced toward an inner surface 5204 of the shell 5006 to enable the annular ridge 5114 to engage the inner surface 5204. A seal member 5206 may interpose the annular ridge 5114 and the inner surface 5204 and thereby form a sealed interface. The seal member 5206 may also extend (flow) into the groove 5116 (FIGS. 15A-16B) defined in the annular ridge 5114 and thereby seal about the sensor 5010 extending laterally within the electronics housing 5004 (FIGS. 15A-16B). In other embodiments, however, the collar 5112 may first be sealed to the inner surface 5204 of the shell 5006, following which the sharp 5012 and the sharp hub 5014 may be extended through the aperture 5110, as described above.

The sensor cap 5018 may be removably coupled to the sensor control device 5002 by threadably mating the internal threads 5026b of the sensor cap 5018 with the external threads 5026a of the mating member 5016. Tightening (rotating) the mated engagement between the sensor cap 5018 and the mating member 5016 may urge the first end 5020a of the sensor cap 5018 into sealed engagement with the bottom 5118 of the collar 5112. Moreover, tightening the mated engagement between the sensor cap 5018 and the mating member 5016 may also enhance the sealed interface between the sharp hub 5014 and the top of the shell 5006, and between the annular ridge 5114 and the inner surface 5204 of the shell 5006.

The inner chamber 5022 may be sized and otherwise configured to receive the in vivo portion 5104 and the sharp tip 5108. Moreover, the inner chamber 5022 may be sealed to isolate the in vivo portion 5104 and the sharp tip 5108 from substances that might adversely interact with the chemistry of the in vivo portion 5104. In some embodiments, a desiccant 5208 (shown in dashed lines) may be present within the inner chamber 5022 to maintain proper humidity levels.

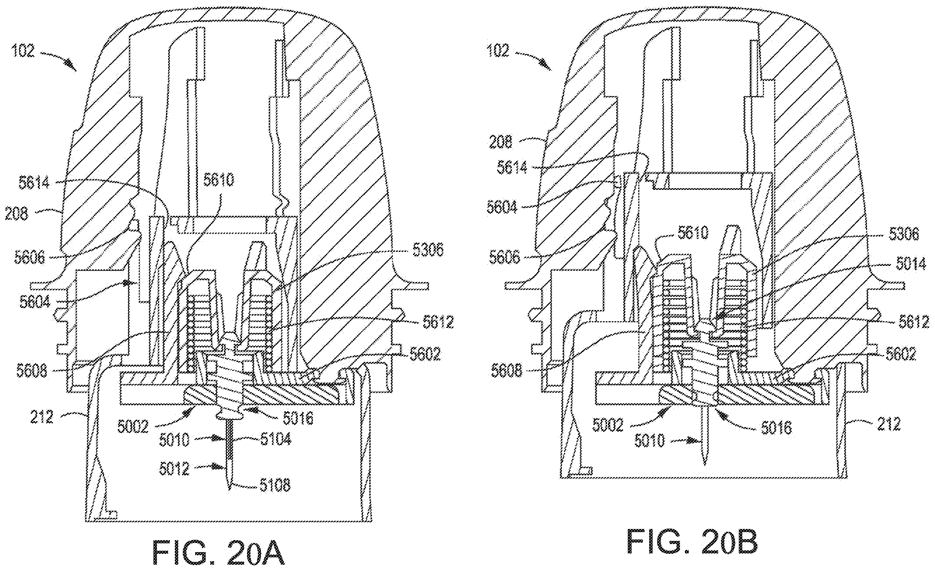

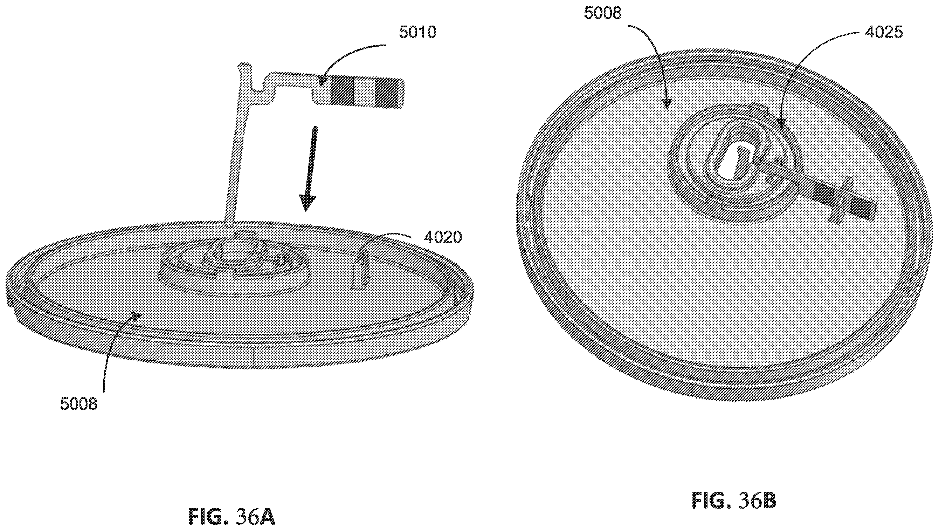

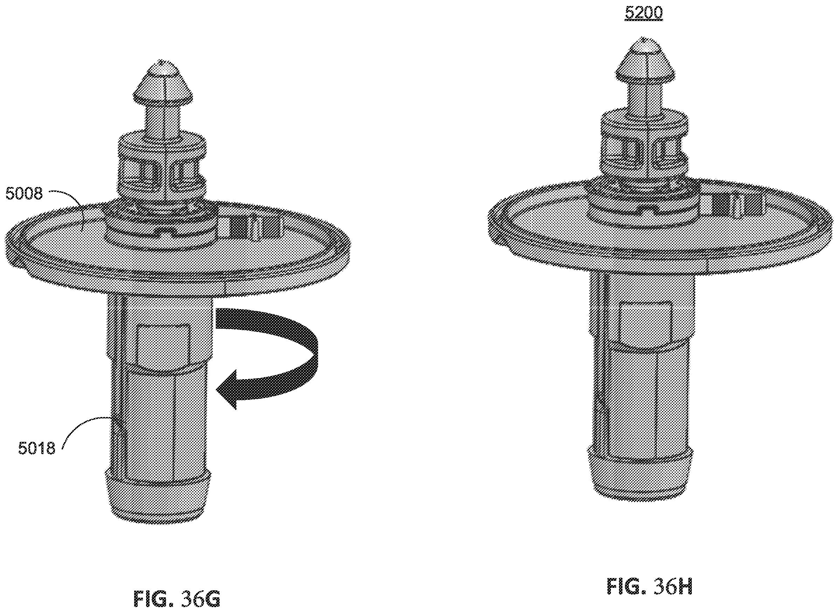

FIGS. 36A-36H illustrate steps of a manufacturing process for manufacturing a sensor subassembly, also referred to as a sealed subassembly such as the sealed subassembly 5200 (see FIGS. 36H, 20). In particular embodiments, assembled sensor subassembly 5200 can include a sensor 5010, sensor mount 5008, collar 5112, sharp 5012, and sensor cap 5018. As described herein, the sensor 5012 can include a body temperature sensor, blood pressure sensor, pulse or heart-rate sensor, glucose level sensor, analyte sensor, or physical activity sensor. Different sensors can be configured and made compatible with the sealed subassembly manufacturing techniques described herein based on the electrical or chemical treatments applied to or used with the sensor of choice.

In an exemplary step of the manufacturing process, as illustrated in FIG. 36A, the sensor 5010 is loaded into the sensor mount 5008. Based on the configuration of the sensor 5010, the sensor mount can include components to interface with and stabilize the sensor 5010 such as flanges 4020, 12112 (see FIG. 12E), 12104, etc. as described herein.

As illustrated in FIG. 36B, the manufacturing process can include dispensing adhesive into a mount channel 4025 of the sensor mount 5008. The adhesive can be dispensed manually or using suitable automation tools. For example, a specially-configured tool having a dispensing valve for dispensing the predetermined adhesive to the mount channel 4025 can be used.

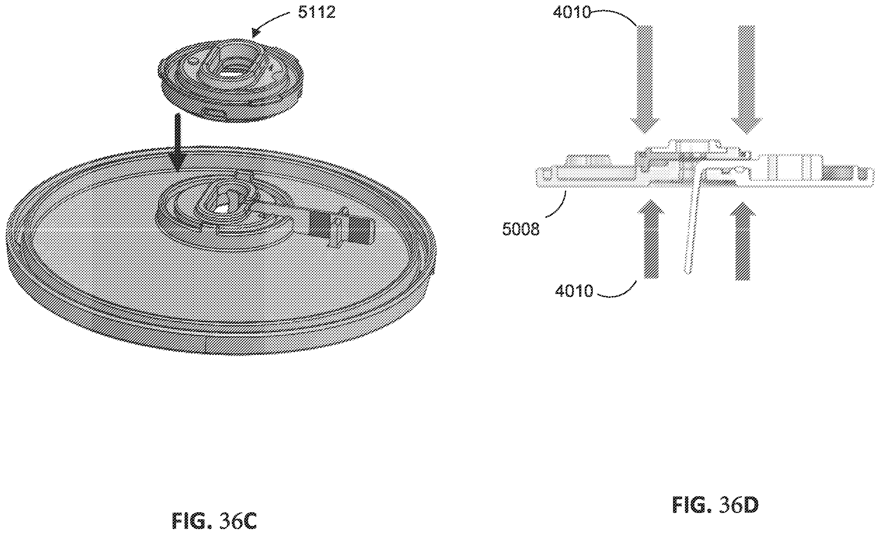

As illustrated in FIG. 36C, the manufacturing process can include loading a collar 5112 onto the sensor mount 5008. In particular, the collar 5112 is loaded to mate with the mount channel 4025 of the sensor 5008. The collar can be loaded manually, or using suitable manufacturing tools, including a manually-operated or robotic loading arm, vacuum or suction gripping arm, magnetic gripping arm, adaptive gripping arm or appendage, or other suitable tool. The collar 5112 can then be clamped to the sensor mount 4025 to ensure the collar 5112 is well-seated within the sensor mount 4025 and disburse the adhesive throughout the sensor mount 4025 and collar 5112. The collar 5112 can be clamped to the sensor mount 4025 using a suitable clamping tool, including a manual clamp, ratcheting clamp, linear slide, including an electric slide, pneumatic slide, ball-screw linear adapter, etc.

The adhesive is then cured to fix the collar 5112 to the sensor mount 5008, as illustrated in FIG. 36D. The adhesive can include a variety of curable adhesive suitable for use in high-throughput manufacturing environments. The adhesive used may be chosen based on cure method and cure time. For example, the adhesive may be chosen to reduce cure time while also limiting exposing the chemistry or electronics of the sensor 5010 to excessive heat, chemicals, etc. that may damage the effectiveness of the sensor, radiation, or excessive infrared or ultra-violet (UV) light. As an example, the adhesive can be a chemically-curable adhesive. Curing the adhesive would then include exposing the adhesive to one or more chemical bonding catalysts. As another example, the adhesive can be an aerobically-curable adhesive. Curing the adhesive would then include exposing the adhesive to air for a sufficient amount before the collar 5112 is mounted or before moving onto the next step. As another example, the adhesive can be a heat-curable adhesive. Curing the adhesive would then include exposing the adhesive to ambient heat or heating elements for a predetermined amount of time. As another example, the adhesive can be a UV-curable adhesive. Curing the adhesive would then include using one or more UV light sources. The UV light sources can include, for example, UV light emitting diodes (LED) arranged to cure the adhesive with a light pipe and multiple angled spot LEDs. FIG. 36D illustrates multiple sources of curing agents 4010 being used to cure the adhesive from above and below the sensor mount 5008.

While curing the adhesive, in certain embodiments, the collar 5112 and sensor mount 5008 can act to shield the sensor 5010 from exposure to curing agents that might otherwise damage the sensor 5010 or other components of the sealed subassembly 5200. Additionally, other temporary components can be used to further protect the sensor 5010. As an example, the collar 5112 can block exposure of chemical agents, heat, or UV light sources while curing the adhesive. Furthermore, depending on the adhesive and curing method, the materials making up the sensor mount 5008 or collar 5112 can be chosen to partially allow curing agents to selectively passthrough to the adhesive.

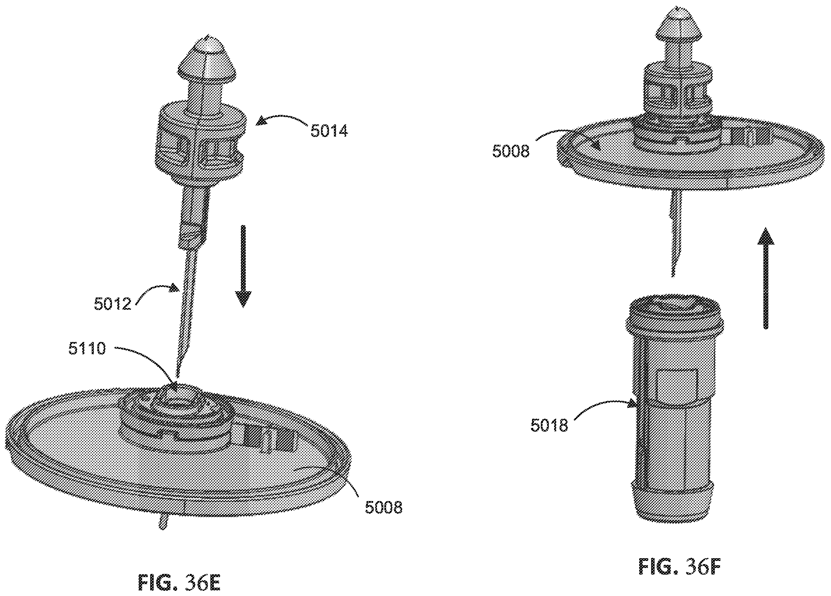

As illustrated in FIG. 36E, the manufacturing process can include mating the sharp hub 5014 to the sensor mount 5008, covering and mating with the sensor 5010. Mating the sharp hub 5014 to the sensor mount 5008 can include causing some or all of the sharp 5012 to pass through an aperture 5110 in the sensor mount 5008 and collar 5112. In some embodiments, the manufacturing process can further include inspecting the sharp 5012 for imperfections. The inspection can be performed prior to, or after, inserting the sharp hub 5014 into the sensor mount 5008. The inspection can be performed manually, e.g., by loading the sharp into a microscope or other magnifying apparatus and allowing a human operator to confirming condition of the sharp. Alternatively, the inspection can be performed automatically, e.g., by imaging the sharp using high-resolution cameras, x-ray imaging, or similar. Having imaged the sharp 5012, a computer vision system can compare the images to acceptable sharps or apply machine-learned models to the image to confirm the condition of the sharp. If the sharp is deemed to have imperfections, it can be discarded.

As illustrated in FIG. 36F, the manufacturing process can include attaching a sensor cap 5018 to the sensor mount 5008, covering the sensor 5010 and sharp 5012, to provide a sealed sensor subassembly 5200. In particular embodiments, the sensor cap 5018 can be composed of a singular structure. In other embodiments, the sensor cap 5018 can include multiple component parts. For example, as discussed herein, the sensor cap 5018 can include a desiccant cap 5030 or plug housing a desiccant to control moisture exposure to the sensor 5010 and sharp 5012. The manufacturing process can include assembling the sensor cap 5018 by inserting a desiccant into the desiccant cap 5030 and attaching the desiccant cap 5030 to the sensor cap 5018.

Attaching the sensor cap 5018 to the sensor mount 5008 can be performed by forcibly mating the sensor cap 5018 to the sensor mount 5008. For example, the sensor mount 5008 or sharp hub 5104 may define a set of external threads matable with a set of internal threads defined by the sensor cap 5018. The external and internal threads may comprise a flat thread design (e.g., lack of helical curvature), which may prove advantageous in molding the parts. The sensor cap 5018 may be removably coupled to the sensor mount 5018 via other types of engagements including, but not limited to, an interference or friction fit, or a frangible member or substance that may be broken with minimal separation force (e.g., axial or rotational force). The sensor cap 5018 can be locked into position manually or using machine tools, such as a pneumatic actuator, to force the sensor cap 5018 to mate with the sensor mount 5008.

As illustrated in FIG. 36G, attaching the sensor cap 5018 to the sensor mount 5008 can include twisting the sensor cap into position. The external and internal threads may comprise a helical threaded engagement. Accordingly, the sensor cap 5018 may be threadably coupled to the sensor mount 5008 or at a mating member of the sharp hub 5014. FIG. 36G illustrates a completed sensor subassembly 5200.

The manufacturing process can include dispensing adhesive to one or more surfaces of the sharp hub 5014. For example, the manufacturing process can include dispensing adhesive to a top surface of the sharp hub 5014, viewing the sensor subassembly 5200 with the sharp cap 5018 oriented downward. The manufacturing process can include dispensing adhesive to a region of the sharp hub 5014 where the sharp hub 5014 interfaces with the sensor mount 5008. The process can further include curing the adhesive. Curing the adhesive can fix the sharp hub 5014 to the sensor mount 5008. Curing the adhesive can seal the sharp hub to reduce leaks between the sharp hub 5014 and the sharp. The adhesive can be dispensed and cured in a manner similar to how the adhesive is dispensed to the mount channel 4025 and subsequently cured. The adhesive can be used to fix the sharp hub 5014 to the sensor mount 5008. The adhesive, when cured, can further promote the sealing of the sensor subassembly 5200.

The manufacturing process can further include testing the sealed sensor subassembly 5200 for leaks. The testing can be performed using a pressure-decay leak test, vacuum-decay leak test, tracer gas leak test, signature analysis test, or mass-flow leak test. In particular embodiments, the leak test can be automated using dedicated machine tooling to facilitate testing of an individual sealed sensor subassembly 5200 or multiple sealed sensor sub-assemblies simultaneously. If the sealed sensor subassembly fails the leak test, it can be discarded.

Once properly assembled, the sealed subassembly 5200 may be subjected to a sterilization process such as any of the radiation sterilization processes mentioned herein to properly sterilize the sensor 5010 and the sharp 5012. The sterilization process can further include heat treatment, electronic-beam sterilization, gamma sterilization, x-ray sterilization, ethylene oxide sterilization, autoclave steam sterilization, chlorine dioxide gas sterilization, hydrogen peroxide sterilization. In particular, the sterilization process can be configured using appropriate machine tools to facilitate sterilization of multiple seal subassemblies 5200 simultaneously. For example, a plurality of sealed subassemblies 5200 can be loaded into a tray for subsequent sterilization.

This sterilization step may be undertaken apart from the remaining portions of the sensor control device (FIGS. 14A-14B and 15A-16B) to prevent damage to sensitive electrical components. The sealed subassembly 5200 may be subjected to sterilization prior to or after coupling the sensor cap 5018 to the sharp hub 5014. When sterilized after coupling the sensor cap 5018 to the sharp hub 5014, the sensor cap 5018 may be made of a material that permits the propagation of sterilizing elements therethrough. In some embodiments, the sensor cap 5018 may be transparent or translucent, but can otherwise be opaque, without departing from the scope of the disclosure.

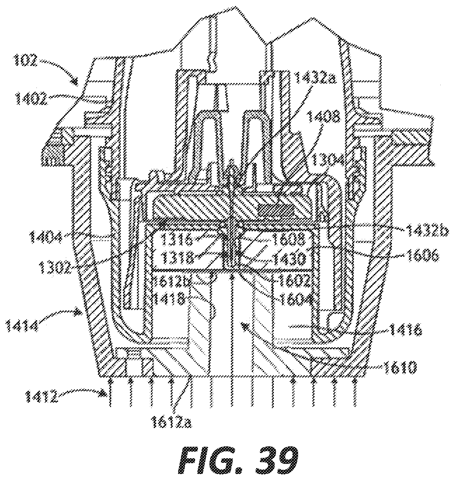

FIG. 39 is a cross-sectional side view of a sensor applicator 102 and an external sterilization assembly 1414, according to one or more additional embodiments. As illustrated, a sensor control device 1302 is received within the sensor applicator 102 and an applicator cap 1404 is coupled to a housing 1402 to secure the sensor control device 1302 therein.

In the illustrated embodiment, the applicator cap 1404 may again be inverted and may define or otherwise provide a cap post 1602 sized to receive the distal ends of a sensor 1316 and a sharp 1318 extending from the bottom of the electronics housing 1304. Moreover, a radiation shield 1416 may be positioned external to the sensor applicator 102 and may extend into the inverted portion of the applicator cap 1404. More specifically, the radiation shield 1416 may extend into the inverted portion of the applicator cap 1404 and to the bottom of the cap post 1602, which may be open ended. As embodied herein, a cap seal 1604 may be arranged at the interface between the cap post 1602 and the radiation shield 1416 to seal off the open end of the cap post 1602.

As embodied herein, a cap fill 1606 may be positioned within the applicator cap 1404. In one or more embodiments, the cap fill 1606 may comprise an integral part or extension of the applicator cap 1404, such as being molded with or overmolded onto the applicator cap 1404. In other embodiments, the cap fill 1606 may comprise a separate structure fitted within or otherwise attached to the applicator cap 1404, without departing from the scope of the disclosure. The cap fill 1606 may also provide or otherwise define an internal collimator 1608 that may help focus the radiation 1412 toward the components to be sterilized. In at least one embodiment, as illustrated, the cap post 1602 may be received within the internal collimator 1608.

The external and internal collimators 1418, 1608 may cooperatively define a sterilization zone 1610 that focuses radiation 1412 toward the sensor 1316 and the sharp 1318. The propagating radiation 1412 may traverse the sterilization zone 1610 to impinge upon and sterilize the sensor 1316 and the sharp 1318. However, the cap fill 1606 and the radiation shield 1416 may each be made of any of the materials mentioned herein that substantially prevent the radiation 1412 from penetrating the inner wall(s) of the sterilization zone 1610 and thereby damaging the radiation sensitive component 1408 within the housing 1304. In at least one embodiment, the cap fill 1606 may be made of machined or 3D printed polypropylene and the radiation shield 1416 may be made of stainless steel. Further, in some sensor embodiments such as those depicted in Figs. 9B and 11H, cap fill 1606 and radiation shield 1416 can prevent electronic components (e.g., electronic components 2418A, electronic components 11914A) which are mounted on the ex vivo portion (e.g., ex vivo portion 2404A, ex vivo portion 11904A) from being damaged by radiation 1412.

The external and internal collimators 1418, 1608 can exhibit any suitable cross-sectional shape necessary to properly focus the radiation 1412 toward the sensor 1316 and the sharp 1318 for sterilization. In the illustrated embodiment, for example, the external collimator 1418 exhibits a circular cross-section, and the internal collimator 1608 is substantially cylindrical with internal walls that are substantially parallel. In other embodiments, however, the external and internal collimators 1418, 1608 may exhibit other cross-sectional shapes, without departing from the scope of the disclosure.

In the illustrated embodiment, the external collimator 1418 defines a first aperture 1612 a that permits the radiation 1412 to enter the sterilization zone 1610 and a second aperture 1612 b positioned at or near the bottom opening to the cap post 1602 to focus the radiation 1412 at the sensor 1316 and the sharp 1318 positioned within the cap post 1602.

The cap seal 1604 may be arranged at the interface between the radiation shield 1416 and the cap post 1602 and/or the cap fill 1606. The cap seal 1604 may seal off a portion of the sterilization zone 1610 to help form part of the sealed region 1430 configured to isolate the sensor 1316 and the sharp 1318 from external contamination. The sealed region 1430 may include (encompass) select portions of the interior of the electronics housing 1304 and the sterilization zone 1610. In the illustrated embodiment, the sealed region 1430 may be defined and otherwise formed by the cap post 1602 and the top and bottom seals 1432 a,b, which create corresponding barriers at their respective sealing locations. The bottom seal 1432b may be arranged to seal an interface between the applicator cap 1404 and the bottom of electronics housing 1304.

Further details regarding embodiments of applicators, their components, methods of sterilizing such embodiments, and variants thereof, are described in U.S. Patent Publication No. 2021/0161437.

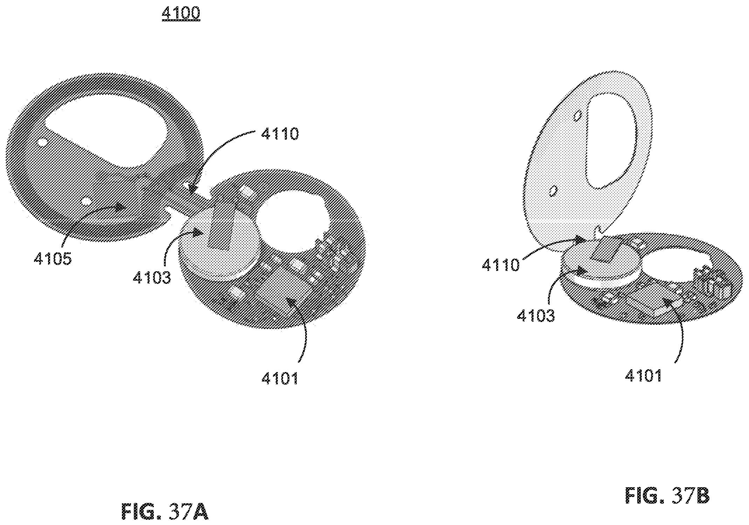

FIGS. 37A-37J illustrate steps of an exemplary process for manufacturing a sensor control device 5002. In particular, FIGS. 37A-37J illustrate steps for manufacturing an electronics housing 5004. As the sensor control device 5002 can be adhered to a user's skin for use with the assistance of an adhesive patch (e.g., adhesive patch 105), while also housing a sensor 5010, the sensor control device 5002 may optionally be referred to as an on-body sensor puck assembly. The electronics housing 5004 shown in FIGS. 37A-37J includes a printed circuit board (PCB) 4100, a shell cap 5006, and a sensor subassembly 5200, the sensor subassembly 5200 including a sensor 5010, a sensor mount 5008 that is matable with the shell cap 5006, a collar 5112, and a sensor cap 5018.

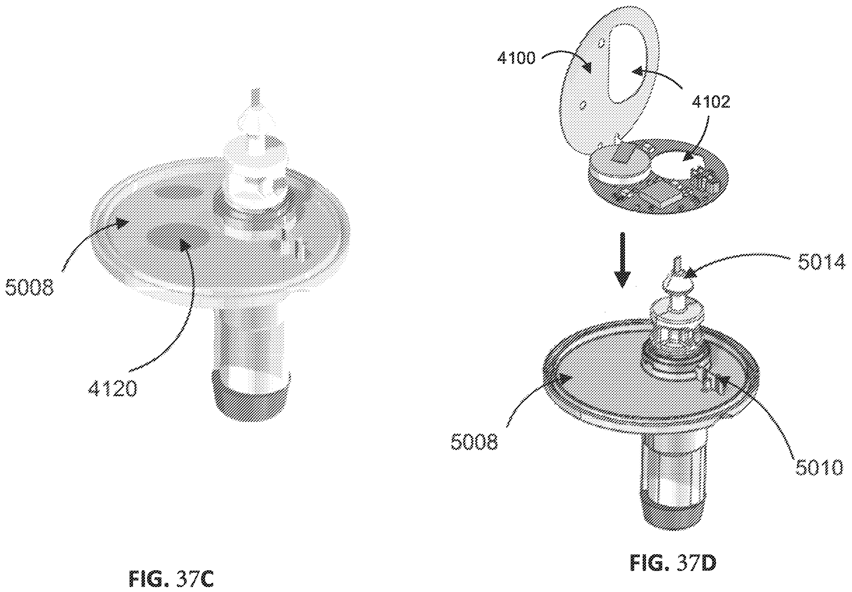

FIGS. 37A-37B illustrate an example PCB 4100 that can be used in the electronics housing 5004 of the on-body sensor puck assembly. The PCB 4100 can include components such as an ASIC 4101, one or more batteries 4103, and one or more antennas 4105. As illustrated, the PCB 4100 can be a foldable or flexible PCB, however non-foldable PCBs can also be used In foldable PCB embodiments, the manufacturing process can include folding the PCB 4100 at a fold point 4110 to fit the footprint of the mount 5008 and shell cap 5006 which defines the overall footprint of the electronics housing 5004. FIG. 37B illustrates the PCB 4100 during folding process. Folding the PCB 4100 can also connect components of the PCB 4100, for example connecting the one or more batteries 4103 to an appropriate battery terminal. As illustrated in FIG. 37C, the manufacturing process can include dispensing a first adhesive 4120 to a sensor mount 5008 of the sensor subassembly 5200. As an example, the adhesive can be dispensed at locations corresponding to components of the PCB 4100, such as the fold, the battery location, or PCB connectors. The adhesive can be dispensed manually or using suitable automation tools. For example, a specially-configured tool having a dispensing valve for dispensing the predetermined adhesive to the designated locations of the sensor mount 5008 can be used. As described herein, the dispensing valve can be used in combination with other components to manipulate the sensor mount 5008 as appropriate before, during, and after the dispensing. For example, the sensor mount 5008 can be rotated by a rotary motor to facilitate even distribution of the adhesive.

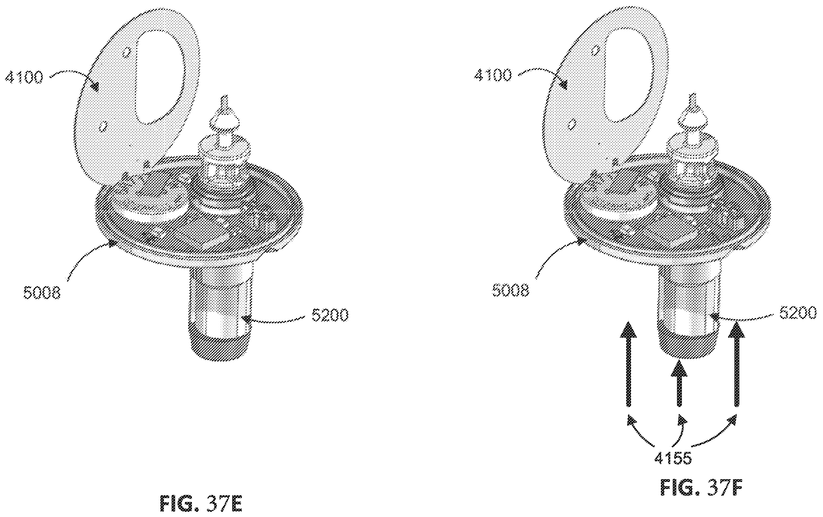

As illustrated in FIG. 37D, the manufacturing process can include loading the PCB 4100 onto the sensor mount 5008 of the sensor subassembly 5200 after aligning the PCB 4100 with the sensor 5010 and the sensor subassembly 5200. For example, the PCB 4100 may include one or more apertures 4102 sized to fit over the sharp hub 5014 of the sealed sensor sharp assembly 5200. FIG. 37E illustrates the PCB 4110 disposed on the sealed subassembly 5200.

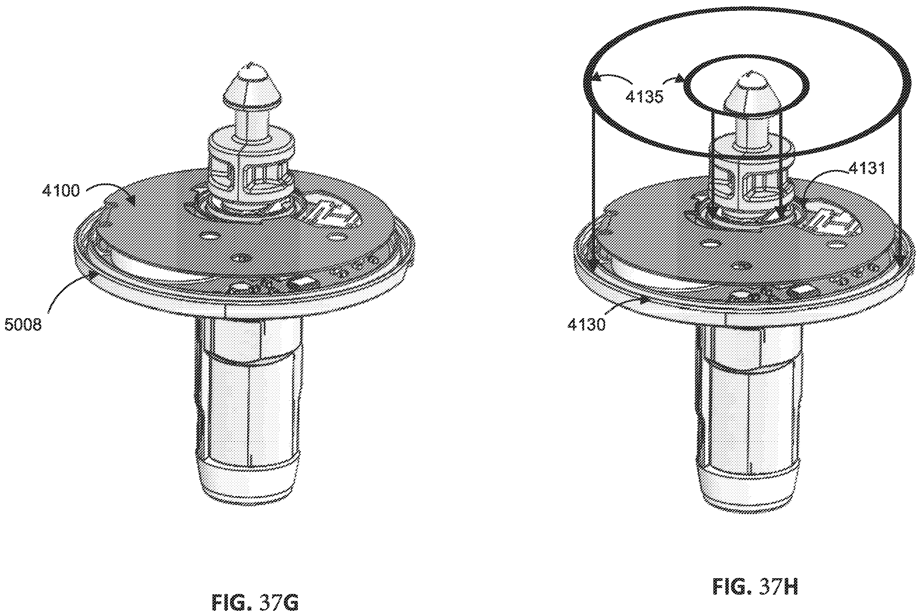

As illustrated in FIG. 37F, the manufacturing process can include curing the first adhesive to fix the PCB to the sensor mount. The adhesive and curing process can include any of the features described herein above. FIG. 37G illustrates the PCB 4100 in a folded state, fixed to the sensor mount 5008.

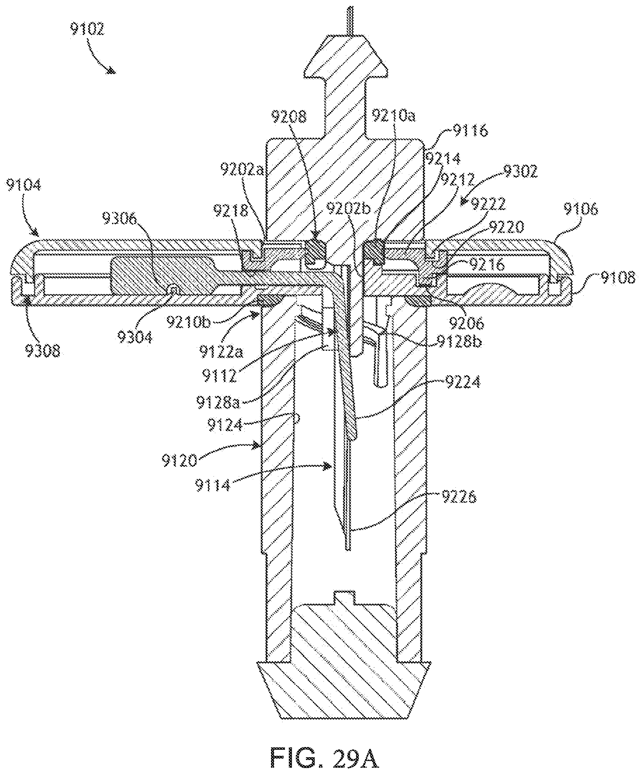

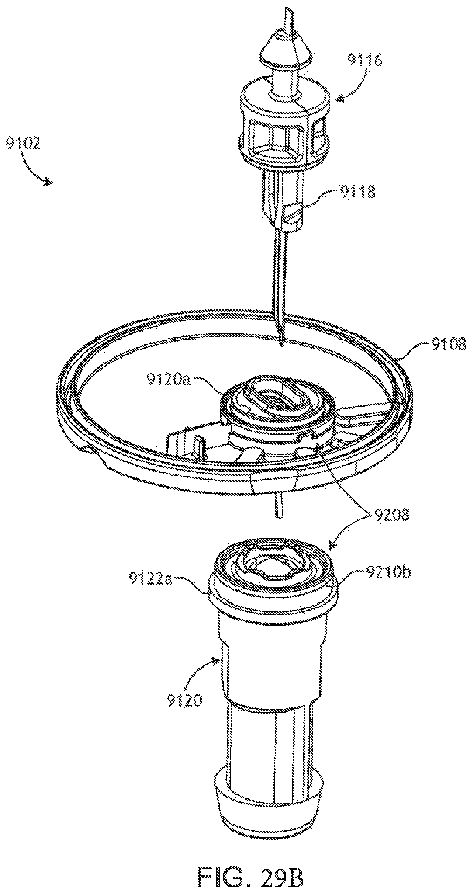

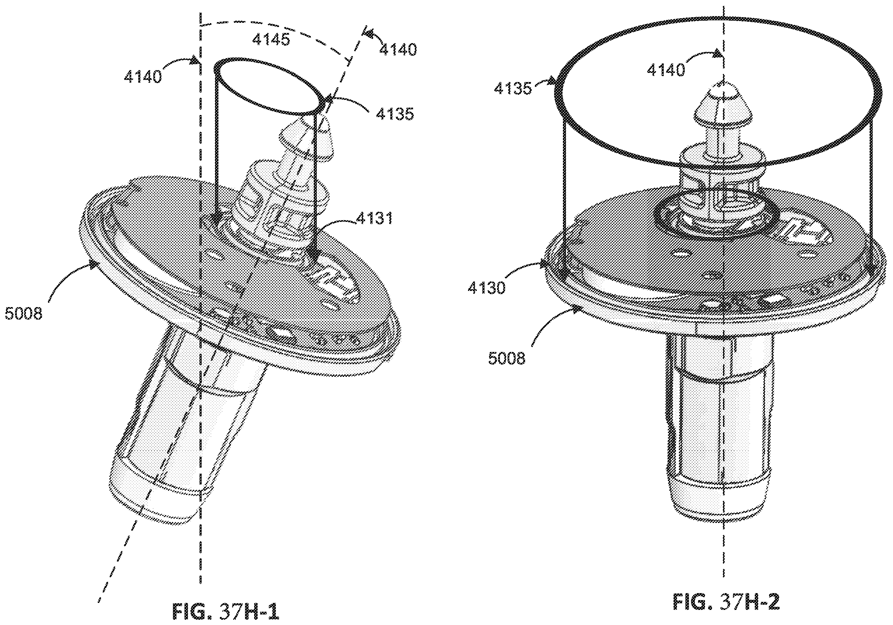

As illustrated in FIG. 37H, the manufacturing process can include dispensing a second adhesive 4135 onto an outer diameter 4130 of the sensor mount 5008 (e.g., channel 9206 shown in FIG. 29) and an inner diameter 4131 of the sensor mount 5008 or collar 5112 of the sensor subassembly 5200 (e.g., collar channel 9220 shown in FIG. 29). The adhesive can be dispensed manually or using suitable automation tools. For example, a specially-configured tool having a dispensing valve for dispensing the predetermined adhesive to the outer diameter 4130 and inner diameter 4131. As described herein, the dispensing valve can be used in combination with other components to manipulate the sensor mount 5008 as appropriate before, during, and after the dispensing.

As illustrated in FIG. 37H-1 for the purpose of illustration and not limitation, dispensing the second adhesive 4135 onto the outer diameter 4130 of the sensor mount 5008 and inner diameter 4131 of the sensor mount 5008 or collar 5112 of the sensor subassembly 5200 can include tilting the sensor mount 5200 along an axis 4140 to a predetermined angle 4145 before dispensing the second adhesive 4145 to the inner diameter 4131 of the sensor mount 5008 or collar 5112 of the sensor subassembly 5200. This tilting process can be used for any of the adhesive dispensing steps described herein. As illustrated in FIG. 37H-2, the sensor mount 5008 and sensor subassembly 5200 is returned to a substantially horizontal position by tilting the sensor mount 5008 along the axis 4140 before dispensing the second adhesive 4135 to the outer diameter 4130 of the sensor mount 5008.

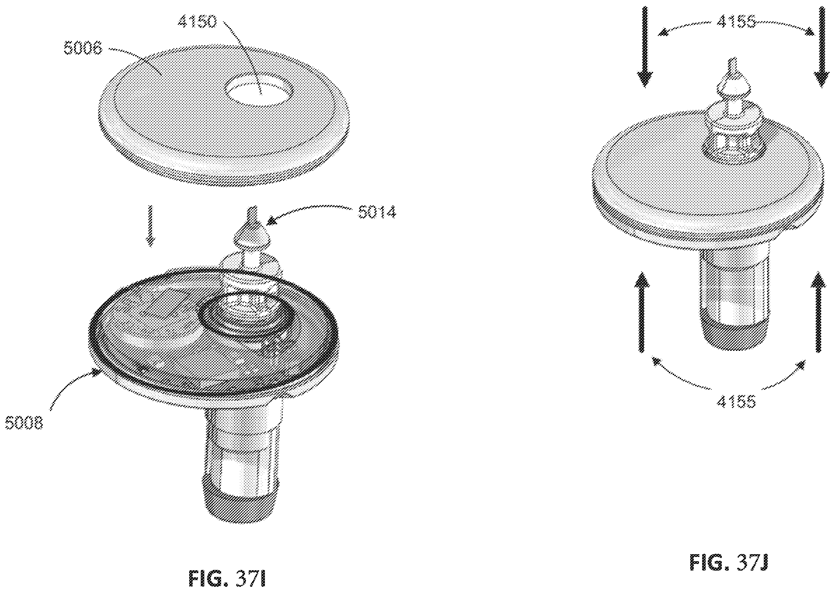

As illustrated in FIG. 37I, the manufacturing process includes attaching the shell cap 5006 to the sensor subassembly 5200 via the sensor mount 5008. An aperture 4150 in the shell cap 5006 is aligned with the sharp hub 5014 before the shell cap 5006 is lowered onto the mount 5008. The shell cap 5006 can be attached to the sensor subassembly 520 manually or using appropriate gripping or clamping tooling, including, but not limited to a manually-operated or robotic loading arm, vacuum or suction gripping arm, magnetic gripping arm, adaptive gripping arm or appendage, or other suitable tool.

As illustrated in FIG. 37J, the manufacturing process includes curing the second adhesive to form the on-body sensor puck assembly. The first adhesive 4130 or second adhesive 4135 can include a variety of curable adhesives suitable for use in high-throughput manufacturing environments. The adhesive(s) used may be chosen based on cure method and cure time. For example, the adhesive(s) may be chosen to reduce cure time while also limit exposing the chemistry or electronics of the sensor subassembly 5200 or PCB 4100 to excessive heat, chemicals, radiation, or excessive infrared or UV light. As an example, the adhesive(s) chosen for the first adhesive 4130 or second adhesive 4135 can be a chemically-curable adhesive. Curing the adhesive would then include exposing the first adhesive 4130 or second adhesive 4135 to one or more chemical bonding catalysts. As another example, the adhesive(s) can be an aerobically-curable adhesive. Curing the first adhesive 4130 or second adhesive 4135 would then include exposing the adhesive(s) to air for a sufficient amount of time before, for example, the shell cap 5006 is lowered to the mount 5008 or before moving onto the next step in the manufacturing process. As another example, the adhesive(s) chosen can be a heat-curable adhesive. Curing the first adhesive 4130 or second adhesive 4135 would then include exposing the adhesive(s) to ambient heat or heating elements for a predetermined amount of time sufficient to cause the adhesive to cure. As another example, the adhesive(s) chosen can be a UV-curable adhesive. Curing the first adhesive 4130 or second adhesive 4135 would then include exposing the adhesive(s) to UV light via one or more UV light sources. The UV light sources can include, for example, UV light emitting diodes (LED) arranged to cure the adhesive with a light pipe and multiple angled spot LEDs. FIGS. 37F and 37J illustrate sources of curing agents 4155 in one embodiment being used to cure the first adhesive 4130 and second adhesive 4135 from above and below the sensor mount 5008.

In certain embodiments, the sensor mount 5008 and shell cap 5006 comprise material that partially allow curing agents to selectively pass through to the first adhesive 4130 and the second adhesive 4135. The sensor mount 5008 and shall cap 5006 can also act to shield the sensor 5010, PCB 4100 and other components of the electronics housing 5004 from exposure to curing agents that might otherwise damage the components of the electronics housing 5004 and sealed subassembly 5200. Additionally, other temporary components can be used to further protect the components.

In some embodiments, the PCB 4100 includes a radio component and the manufacturing process further includes writing data to the radio component of the PCB 4100. For example, data to be written to the radio component of the PCB 4100 can be read from the sensor subassembly 5200, PCB 4100, a shell cap 5004, mount 5006 or other component associated with the electronics housing 5004. The data can then be written to the radio component of the PCB 4100.

In some embodiments, the manufacturing process can further include testing the electronics housing 5004 (e.g., the on-body sensor puck assembly) for leaks. The test can include using a pressure-decay leak test, vacuum-decay leak test, tracer gas leak test, signature analysis test, or mass-flow leak test. If the on-body sensor puck assembly fails the leak test, it can be discarded.

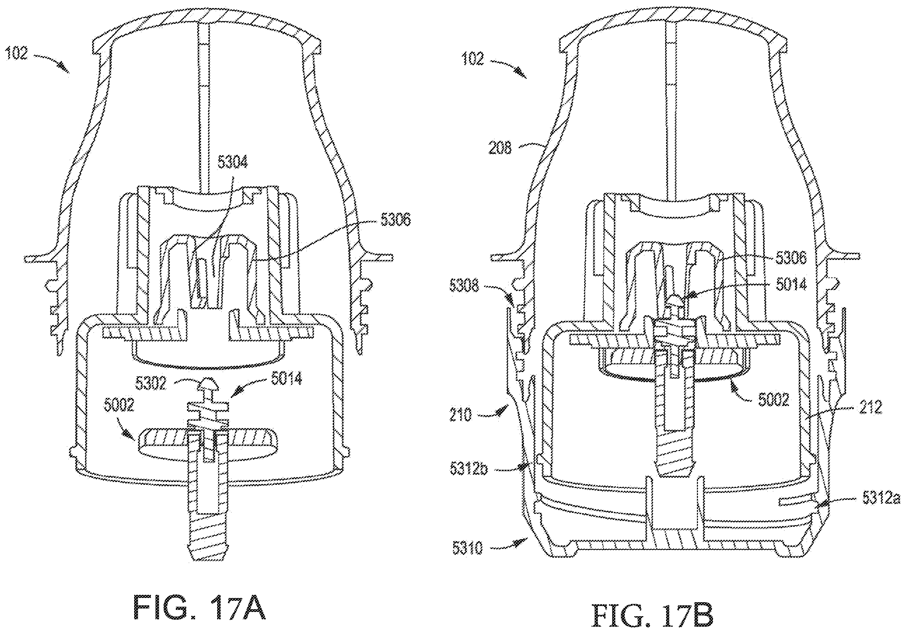

FIGS. 17A-17C are progressive cross-sectional side views showing assembly of the sensor applicator 102 with the sensor control device 5002, according to one or more embodiments. Once the sensor control device 5002 is fully assembled, it may then be loaded into the sensor applicator 102. With reference to FIG. 17A, the sharp hub 5014 may include or otherwise define a hub snap pawl 5302 configured to help couple the sensor control device 5002 to the sensor applicator 102. More specifically, the sensor control device 5002 may be advanced into the interior of the sensor applicator 102 and the hub snap pawl 5302 may be received by corresponding arms 5304 of a sharp carrier 5306 positioned within the sensor applicator 102.

In FIG. 17B, the sensor control device 5002 is shown received by the sharp carrier 5306 and, therefore, secured within the sensor applicator 102. Once the sensor control device 5002 is loaded into the sensor applicator 102, the applicator cap 210 may be coupled to the sensor applicator 102. In some embodiments, the applicator cap 210 and the housing 208 may have opposing, matable sets of threads 5308 that enable the applicator cap 210 to be screwed onto the housing 208 in a clockwise (or counter-clockwise) direction and thereby secure the applicator cap 210 to the sensor applicator 102.

As illustrated, the sheath 212 is also positioned within the sensor applicator 102, and the sensor applicator 102 may include a sheath locking mechanism 5310 configured to ensure that the sheath 212 does not prematurely collapse during a shock event. In the illustrated embodiment, the sheath locking mechanism 5310 may comprise a threaded engagement between the applicator cap 210 and the sheath 212. More specifically, one or more internal threads 53 l2a may be defined or otherwise provided on the inner surface of the applicator cap 210, and one or more external threads 53 l2b may be defined or otherwise provided on the sheath 212. The internal and external threads 53 l2a,b may be configured to threadably mate as the applicator cap 210 is threaded to the sensor applicator 102 at the threads 5308. The internal and external threads 53 l2a,b may have the same thread pitch as the threads 5308 that enable the applicator cap 210 to be screwed onto the housing 208.

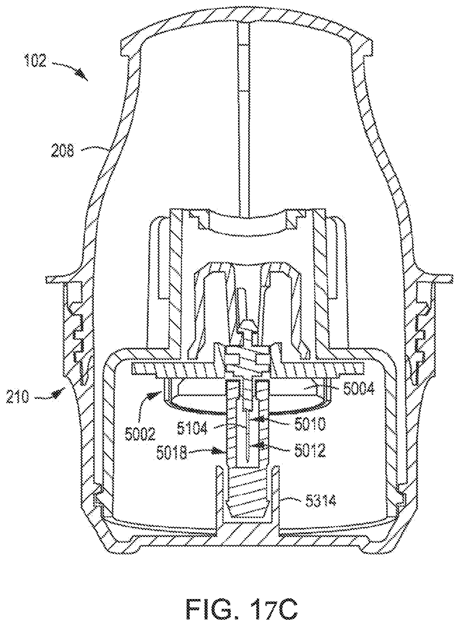

In FIG. 17C, the applicator cap 210 is shown fully threaded (coupled) to the housing 208. As illustrated, the applicator cap 210 may further provide and otherwise define a cap post 5314 centrally located within the interior of the applicator cap 210 and extending proximally from the bottom thereof. The cap post 5314 may be configured to receive at least a portion of the sensor cap 5018 as the applicator cap 210 is screwed onto the housing 208.

With the sensor control device 5002 loaded within the sensor applicator 102 and the applicator cap 210 properly secured, the sensor control device 5002 may then be subjected to a gaseous chemical sterilization configured to sterilize the electronics housing 5004 and any other exposed portions of the sensor control device 5002. Since the distal portions of the sensor 5010 and the sharp 5012 are sealed within the sensor cap 5018, the chemicals used during the gaseous chemical sterilization process are unable to interact with the enzymes, chemistry, and biologies provided on the in vivo portion 5104, and other sensor components, such as membrane coatings that regulate analyte influx.

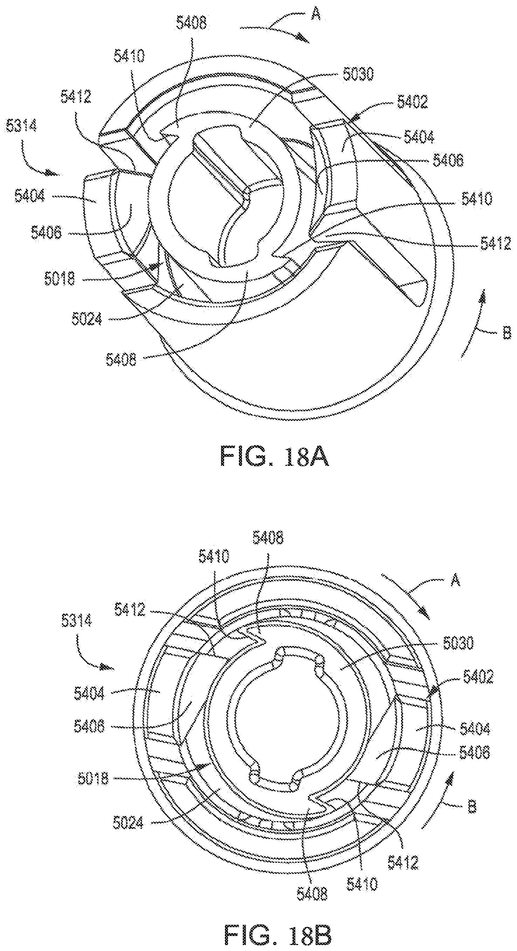

FIGS. 18A and 18B are perspective and top views, respectively, of the cap post 5314, according to one or more additional embodiments. In the illustrated depiction, a portion of the sensor cap 5018 is received within the cap post 5314 and, more specifically, the desiccant cap 5030 of the sensor cap 5018 is arranged within cap post 5314.

As illustrated, the cap post 5314 may define a receiver feature 5402 configured to receive the engagement feature 5024 of the sensor cap 5018 upon coupling (e.g., threading) the applicator cap 210 (FIG. 17C) to the sensor applicator 102 (FIGS. 17A-17C). Upon removing the applicator cap 210 from the sensor applicator 102, however, the receiver feature 5402 may prevent the engagement feature 914 from reversing direction and thus prevent the sensor cap 5018 from separating from the cap post 5314. Instead, removing the applicator cap 210 from the sensor applicator 102 will simultaneously detach the sensor cap 5018 from the sensor control device 5002 (FIGS. 14A-14B and 17A-17C), and thereby expose the distal portions of the sensor 5010 (FIGS. 17A-17C) and the sharp 5012 (FIGS. 17A-17C).

Many design variations of the receiver feature 5402 may be employed, without departing from the scope of the disclosure. In the illustrated embodiment, the receiver feature 5402 includes one or more compliant members 5404 (two shown) that are expandable or flexible to receive the engagement feature 5024 (FIGS. 14A-14B). The engagement feature 5024 may comprise, for example, an enlarged head and the compliant member(s) 5404 may comprise a collet- type device that includes a plurality of compliant fingers configured to flex radially outward to receive the enlarged head.

The compliant member(s) 5404 may further provide or otherwise define corresponding ramped surfaces 5406 configured to interact with one or more opposing camming surfaces 5408 provided on the outer wall of the engagement feature 5024. The configuration and alignment of the ramped surface(s) 5406 and the opposing camming surface(s) 5408 is such that the applicator cap 210 is able to rotate relative to the sensor cap 5018 in a first direction A (e.g., clockwise), but the cap post 5314 binds against the sensor cap 5018 when the applicator cap 210 is rotated in a second direction B (e.g., counter clockwise). More particularly, as the applicator cap 210 (and thus the cap post 5314) rotates in the first direction A, the camming surfaces 5408 engage the ramped surfaces 5406, which urge the compliant members 5404 to flex or otherwise deflect radially outward and results in a ratcheting effect. Rotating the applicator cap 210 (and thus the cap post 5314) in the second direction B, however, will drive angled surfaces 5410 of the camming surfaces 5408 into opposing angled surfaces 5412 of the ramped surfaces 5406, which results in the sensor cap 5018 binding against the compliant member(s) 5404.

FIG. 19 is a cross-sectional side view of the sensor control device 5002 positioned within the applicator cap 210, according to one or more embodiments. As illustrated, the opening to the receiver feature 5402 exhibits a first diameter D3, while the engagement feature 5024 of the sensor cap 5018 exhibits a second diameter D4 that is larger than the first diameter D3 and greater than the outer diameter of the remaining portions of the sensor cap 5018. As the sensor cap 5018 is extended into the cap post 5314, the compliant member(s) 5404 of the receiver feature 5402 may flex (expand) radially outward to receive the engagement feature 5024. In some embodiments, as illustrated, the engagement feature 5024 may provide or otherwise define an angled or frustoconical outer surface that helps bias the compliant member(s) 5404 radially outward. Once the engagement feature 5024 bypasses the receiver feature 5402, the compliant member(s) 5404 are able to flex back to (or towards) their natural state and thus lock the sensor cap 5018 within the cap post 5314.

As the applicator cap 210 is threaded to (screwed onto) the housing 208 (FIGS. 17A-17C) in the first direction A, the cap post 5314 correspondingly rotates in the same direction and the sensor cap 5018 is progressively introduced into the cap post 5314. As the cap post 5314 rotates, the ramped surfaces 5406 of the compliant members 5404 ratchet against the opposing camming surfaces 5408 of the sensor cap 5018. This continues until the applicator cap 210 is fully threaded onto (screwed onto) the housing 208. In some embodiments, the ratcheting action may occur over two full revolutions of the applicator cap 210 before the applicator cap 210 reaches its final position.

To remove the applicator cap 210, the applicator cap 210 is rotated in the second direction B, which correspondingly rotates the cap post 5314 in the same direction and causes the camming surfaces 5408 (i.e., the angled surfaces 5410 of FIGS. 18A-18B) to bind against the ramped surfaces 5406 (i.e., the angled surfaces 5412 of FIGS. 18A-18B). Consequently, continued rotation of the applicator cap 210 in the second direction B causes the sensor cap 5018 to correspondingly rotate in the same direction and thereby unthread from the mating member 5016 to allow the sensor cap 5018 to detach from the sensor control device 5002. Detaching the sensor cap 5018 from the sensor control device 5002 exposes the distal portions of the sensor 5010 and the sharp 5012, and thus places the sensor control device 5002 in position for firing (use).

FIGS. 20A and 20B are cross-sectional side views of the sensor applicator 102 ready to deploy the sensor control device 5002 to a target monitoring location, according to one or more embodiments. More specifically, FIG. 20A depicts the sensor applicator 102 ready to deploy (fire) the sensor control device 5002, and FIG. 20B depicts the sensor applicator 102 in the process of deploying (firing) the sensor control device 5002. As illustrated, the applicator cap 210 (FIGS. 17A-17C and 55) has been removed, which correspondingly detaches (removes) the sensor cap 5018 (FIGS. 17A-17C and 55 and thereby exposes the in vivo portion 5104 of the sensor 5010 and the sharp tip 5108 of the sharp 5012, as described above. In conjunction with the sheath 212 and the sharp carrier 5306, the sensor applicator 102 also includes a sensor carrier 5602 (alternately referred to as a "puck" carrier) that helps position and secure the sensor control device 5002 within the sensor applicator 102.

Referring first to FIG. 20A, as illustrated, the sheath 212 includes one or more sheath arms 5604 (one shown) configured to interact with a corresponding one or more detents 5606 (one shown) defined within the interior of the housing 208. The detent(s) 5606 are alternately referred to as "firing" detent(s). When the sensor control device 5002 is initially installed in the sensor applicator 102, the sheath arms 5604 may be received within the detents 5606, which places the sensor applicator 102 in firing position. In the firing position, the mating member 5016 extends distally beyond the bottom of the sensor control device 5002. As discussed below, the process of firing the sensor applicator 102 causes the mating member 5016 to retract so that it does not contact the user's skin.

The sensor carrier 5602 may also include one or more carrier arms 5608 (one shown) configured to interact with a corresponding one or more grooves 5610 (one shown) defined on the sharp carrier 5306. A spring 5612 may be arranged within a cavity defined by the sharp carrier 5306 and may passively bias the sharp carrier 5306 upward within the housing 208. When the carrier arm(s) 5608 are properly received within the groove(s) 5610, however, the sharp carrier 5306 is maintained in position and prevented from moving upward. The carrier arm(s) 5608 interpose the sheath 212 and the sharp carrier 5306, and a radial shoulder 5614 defined on the sheath 212 may be sized to maintain the carrier arm(s) 5608 engaged within the groove(s) 5610 and thereby maintain the sharp carrier 5306 in position.

In FIG. 20B, the sensor applicator 102 is in the process of firing. As discussed herein with reference to FIGS. 3F-3G, this may be accomplished by advancing the sensor applicator 102 toward a target monitoring location until the sheath 212 engages the skin of the user. Continued pressure on the sensor applicator 102 against the skin may cause the sheath arm(s) 5604 to disengage from the corresponding detent(s) 5606, which allows the sheath 212 to collapse into the housing 208. As the sheath 212 starts to collapse, the radial shoulder 5614 eventually moves out of radial engagement with the carrier arm(s) 5608, which allows the carrier arm(s) 5608 to disengage from the groove(s) 5610. The passive spring force of the spring 5612 is then free to push upward on the sharp carrier 5306 and thereby force the carrier arm(s) 5608 out of engagement with the groove(s) 5610, which allows the sharp carrier 5306 to move slightly upward within the housing 208. In some embodiments, fewer coils may be incorporated into the design of the spring 5612 to increase the spring force necessary to overcome the engagement between carrier arm(s) 5608 and the groove(s) 5610. In at least one embodiment, one or both of the carrier arm(s) 5608 and the groove(s) 5610 may be angled to help ease disengagement.

As the sharp carrier 5306 moves upward within the housing 208, the sharp hub 5014 may correspondingly move in the same direction, which may cause partial retraction of the mating member 5016 such that it becomes flush, substantially flush, or sub-flush with the bottom of the sensor control device 5002. As will be appreciated, this ensures that the mating member 5016 does not come into contact with the user's skin, which might otherwise adversely impact sensor insertion, cause excessive pain, or prevent the adhesive patch (not shown) positioned on the bottom of the sensor control device 5002 from properly adhering to the skin.

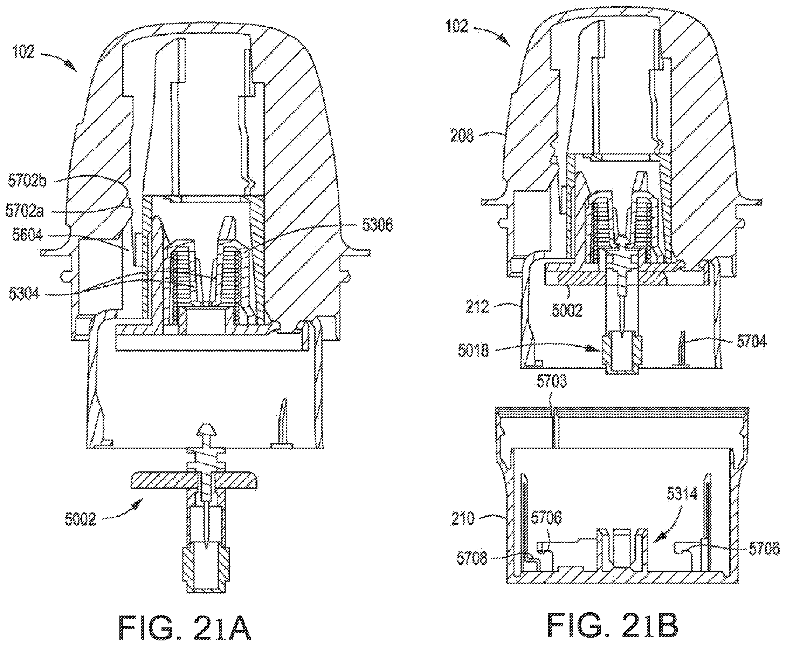

FIGS. 21A-21C are progressive cross-sectional side views showing assembly and disassembly of an alternative embodiment of the sensor applicator 102 with the sensor control device 5002, according to one or more additional embodiments. A fully assembled sensor control device 5002 may be loaded into the sensor applicator 102 by coupling the hub snap pawl 5302 into the arms 5304 of the sharp carrier 5306 positioned within the sensor applicator 102, as generally described above.

In the illustrated embodiment, the sheath arms 5604 of the sheath 212 may be configured to interact with a first detent 5702a and a second detent 5702b defined within the interior of the housing 208. The first detent 5702a may alternately be referred to a "locking" detent, and the second detent 5702b may alternately be referred to as a "firing" detent. When the sensor control device 5002 is initially installed in the sensor applicator 102, the sheath arms 5604 may be received within the first detent 5702a. As discussed below, the sheath 212 may be actuated to move the sheath arms 5604 to the second detent 5702b, which places the sensor applicator 102 in firing position.

In FIG. 21B, the applicator cap 210 is aligned with the housing 208 and advanced toward the housing 208 so that the sheath 212 is received within the applicator cap 210. Instead of rotating the applicator cap 210 relative to the housing 208, the threads of the applicator cap 210 may be snapped onto the corresponding threads of the housing 208 to couple the applicator cap 210 to the housing 208. Axial cuts or slots 5703 (one shown) defined in the applicator cap 210 may allow portions of the applicator cap 210 near its threading to flex outward to be snapped into engagement with the threading of the housing 208. As the applicator cap 210 is snapped to the housing 208, the sensor cap 5018 may correspondingly be snapped into the cap post 5314.

Similar to the embodiment of FIGS. 17A-17C, the sensor applicator 102 may include a sheath locking mechanism configured to ensure that the sheath 212 does not prematurely collapse during a shock event. In the illustrated embodiment, the sheath locking mechanism includes one or more ribs 5704 (one shown) defined near the base of the sheath 212 and configured to interact with one or more ribs 5706 (two shown) and a shoulder 5708 defined near the base of the applicator cap 210. The ribs 5704 may be configured to inter-lock between the ribs 5706 and the shoulder 5708 while attaching the applicator cap 210 to the housing 208. More specifically, once the applicator cap 210 is snapped onto the housing 208, the applicator cap 210 may be rotated (e.g., clockwise), which locates the ribs 5704 of the sheath 212 between the ribs 5706 and the shoulder 5708 of the applicator cap 210 and thereby "locks" the applicator cap 210 in place until the user reverse rotates the applicator cap 210 to remove the applicator cap 210 for use. Engagement of the ribs 5704 between the ribs 5706 and the shoulder 5708 of the applicator cap 210 may also prevent the sheath 212 from collapsing prematurely.

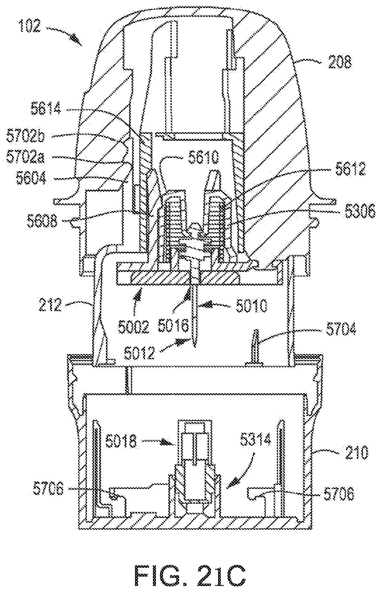

In FIG. 21C, the applicator cap 210 is removed from the housing 208. As with the embodiment of FIGS. 17A-17C, the applicator cap 210 can be removed by reverse rotating the applicator cap 210, which correspondingly rotates the cap post 5314 in the same direction and causes sensor cap 5018 to unthread from the mating member 5016, as generally described above. Moreover, detaching the sensor cap 5018 from the sensor control device 5002 exposes the distal portions of the sensor 5010 and the sharp 5012.

As the applicator cap 210 is unscrewed from the housing 208, the ribs 5704 defined on the sheath 212 may slidingly engage the tops of the ribs 5706 defined on the applicator cap 210. The tops of the ribs 5706 may provide corresponding ramped surfaces that result in an upward displacement of the sheath 212 as the applicator cap 210 is rotated, and moving the sheath 212 upward causes the sheath arms 5604 to flex out of engagement with the first detent 5702a to be received within the second detent 5702b. As the sheath 212 moves to the second detent 5702b, the radial shoulder 5614 moves out of radial engagement with the carrier arm(s) 5608, which allows the passive spring force of the spring 5612 to push upward on the sharp carrier 5306 and force the carrier arm(s) 5608 out of engagement with the groove(s) 5610. As the sharp carrier 5306 moves upward within the housing 208, the mating member 5016 may correspondingly retract until it becomes flush, substantially flush, or sub-flush with the bottom of the sensor control device 5002. At this point, the sensor applicator 102 in firing position. Accordingly, in this embodiment, removing the applicator cap 210 correspondingly causes the mating member 5016 to retract.

FIG. 2A is an isometric bottom view of the housing 208, according to one or more embodiments. As illustrated, one or more longitudinal ribs 5802 (four shown) may be defined within the interior of the housing 208. The ribs 5802 may be equidistantly or non-equidistantly spaced from each other and extend substantially parallel to centerline of the housing 208. The first and second detents 5702a, b may be defined on one or more of the longitudinal ribs 5802.

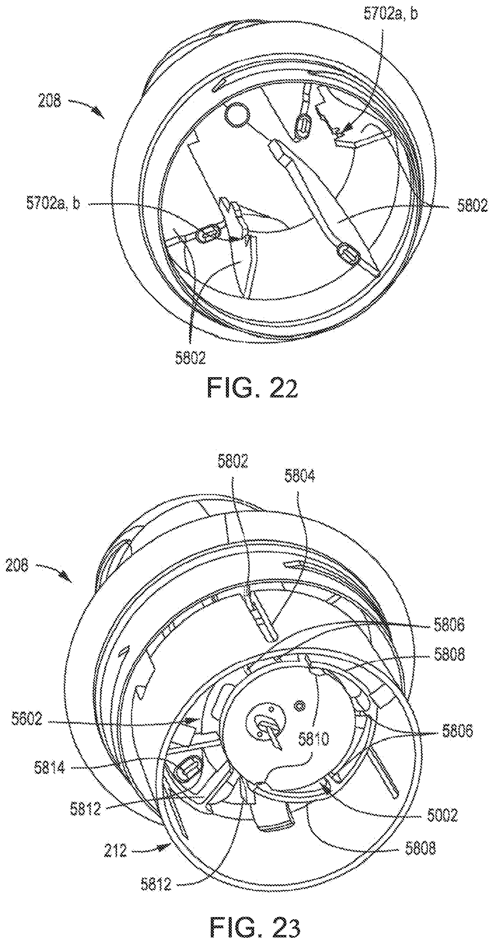

FIG. 23A is an isometric bottom view of the housing 208 with the sheath 212 and other components at least partially positioned within the housing 208. As illustrated, the sheath 212 may provide or otherwise define one or more longitudinal slots 5804 configured to mate with the longitudinal ribs 5802 of the housing 208. As the sheath 212 collapses into the housing 208, as generally described above, the ribs 5802 may be received within the slots 5804 to help maintain the sheath 212 aligned with the housing during its movement. As will be appreciated, this may result in tighter circumferential and radial alignment within the same dimensional and tolerance restrictions of the housing 208.

In the illustrated embodiment, the sensor carrier 5602 may be configured to hold the sensor control device 5002 in place both axially (e.g., once the sensor cap 5018 is removed) and circumferentially. To accomplish this, the sensor carrier 5602 may include or otherwise define one or more support ribs 5806 and one or more flexible arms 5808. The support ribs 5806 extend radially inward to provide radial support to the sensor control device 5002. The flexible arms 5808 extend partially about the circumference of the sensor control device 5002 and the ends of the flexible arms 5808 may be received within corresponding grooves 5810 defined in the side of the sensor control device 5002. Accordingly, the flexible arms 5808 may be able to provide both axial and radial support to the sensor control device 5002. In at least one embodiment, the ends of the flexible arms 5808 may be biased into the grooves 5810 of the sensor control device 5002 and otherwise locked in place with corresponding sheath locking ribs 5812 provided by the sheath 212.

In some embodiments, the sensor carrier 5602 may be ultrasonically welded to the housing 208 at one or more points 5814. In other embodiments, however, the sensor carrier 5602 may alternatively be coupled to the housing 208 via a snap-fit engagement, without departing from the scope of the disclosure. This may help hold the sensor control device 5002 in place during transport and firing.

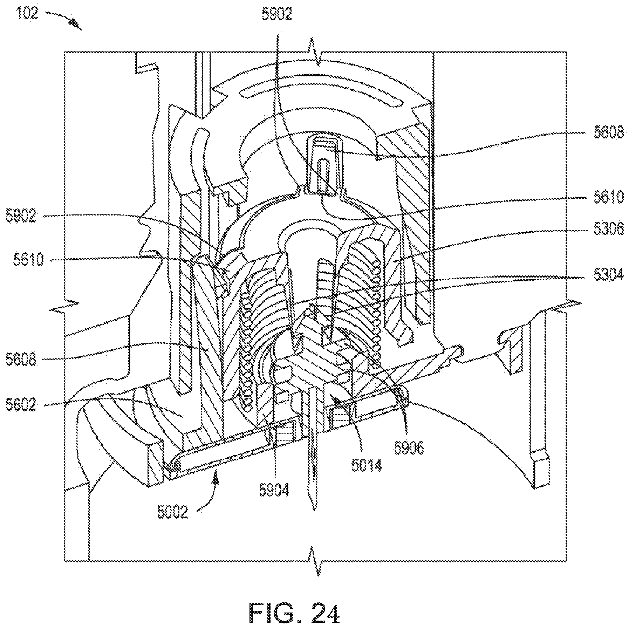

FIG. 24 is an enlarged cross-sectional side view of the sensor applicator 102 with the sensor control device 5002 installed therein, according to one or more embodiments. As discussed above, the sensor carrier 5602 may include one or more carrier arms 5608 (two shown) engageable with the sharp carrier 5306 at corresponding grooves 5610. In at least one embodiment, the grooves 5610 may be defined by pairs of protrusions 5902 defined on the sharp carrier 5306. Receiving the carrier arms 5608 within the grooves 5610 may help stabilize the sharp carrier 5306 from unwanted tilting during all stages of retraction (firing).

In the illustrated embodiment, the arms 5304 of the sharp carrier 5306 may be stiff enough to control, with greater refinement, radial and bi-axial motion of the sharp hub 5014. In some embodiments, for example, clearances between the sharp hub 5014 and the arms 5304 may be more restrictive in both axial directions as the relative control of the height of the sharp hub 5014 may be more critical to the design.

In the illustrated embodiment, the sensor carrier 5602 defines or otherwise provides a central boss 5904 sized to receive the sharp hub 5014. In some embodiments, as illustrated, the sharp hub 5014 may provide one or more radial ribs 5906 (two shown). In at least one embodiment, the inner diameter of the central boss 5904 helps provide radial and tilt support to the sharp hub 5014 during the life of sensor applicator 102 and through all phases of operation and assembly. Moreover, having multiple radial ribs 5906 increases the length-to-width ratio of the sharp hub 5014, which also improves support against tilting.

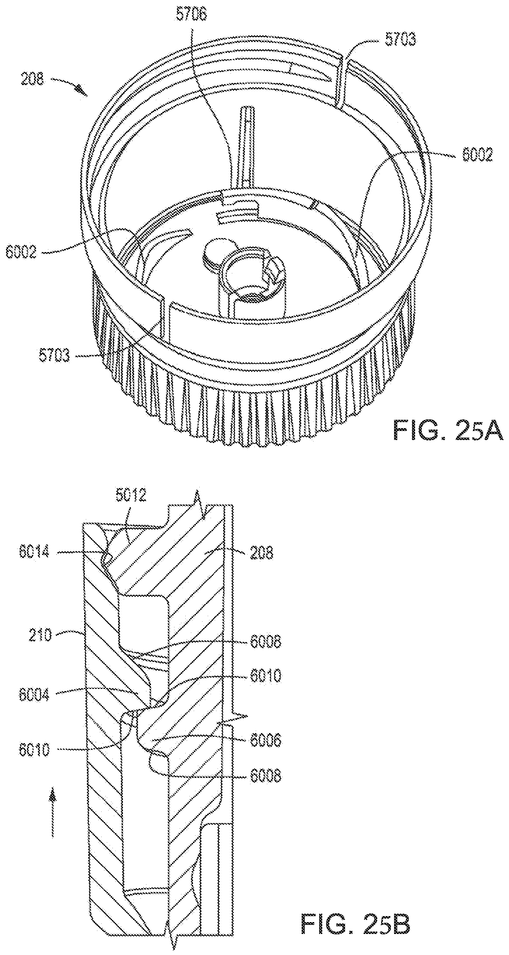

FIG. 25A is an isometric top view of the applicator cap 210, according to one or more embodiments. In the illustrated embodiment, two axial slots 5703 are depicted that separate upper portions of the applicator cap 210 near its threading. As mentioned above, the slots 5703 may help the applicator cap 210 flex outward to be snapped into engagement with the housing 208 (FIG. 21B). In contrast, the applicator cap 210 may be twisted (unthreaded) off the housing 208 by an end user.

FIG. 25A also depicts the ribs 5706 (one visible) defined by the applicator cap 210. By interlocking with the ribs 5704 (FIG. 21C) defined on the sheath 212 (FIG. 21C), the ribs 5706 may help lock the sheath 212 in all directions to prevent premature collapse during a shock or drop event. The sheath 212 may be unlocked when the user unscrews the applicator cap 210 from the housing, as generally described above. As mentioned herein, the top of each rib 5706 may provide a corresponding ramped surface 6002, and as the applicator cap 210 is rotated to unthread from the housing 208, the ribs 5704 defined on the sheath 212 may slidingly engage the ramped surfaces 6002, which results in the upward displacement of the sheath 212 into the housing 208.

In some embodiments, additional features may be provided within the interior of the applicator cap 210 to hold a desiccant component that maintains proper moisture levels through shelf life. Such additional features may be snaps, posts for press-fitting, heat-staking, ultrasonic welding, etc.

FIG. 25B is an enlarged cross-sectional view of the engagement between the applicator cap 210 and the housing 208, according to one or more embodiments. As illustrated, the applicator cap 210 may define a set of inner threads 6004 and the housing 208 may define a set of outer threads 6006 engageable with the inner threads 6004. As mentioned herein, the applicator cap 210 may be snapped onto the housing 208, which may be accomplished by advancing the inner threads 6004 axially past the outer threads 6006 in the direction indicated by the arrow, which causes the applicator cap 210 to flex outward. To help ease this transition, as illustrated, corresponding surfaces 6008 of the inner and outer threads 6004, 6006 may be curved, angled, or chamfered. Corresponding flat surfaces 6010 may be provided on each thread 6004, 6006 and configured to matingly engage once the applicator cap 210 is properly snapped into place on the housing 208. The flat surfaces 6010 may slidingly engage one another as the user unthreads the applicator cap 210 from the housing 208.

The threaded engagement between the applicator cap 210 and the housing 208 results in a sealed engagement that protects the inner components against moisture, dust, etc. In some embodiments, the housing 208 may define or otherwise provide a stabilizing feature 6012 configured to be received within a corresponding groove 1914 defined on the applicator cap 210. The stabilizing feature 6012 may help stabilize and stiffen the applicator cap 210 once the applicator cap 210 is snapped onto the housing 208. This may prove advantageous in providing additional drop robustness to the sensor applicator 102. This may also help increase the removal torque of the applicator cap 210.

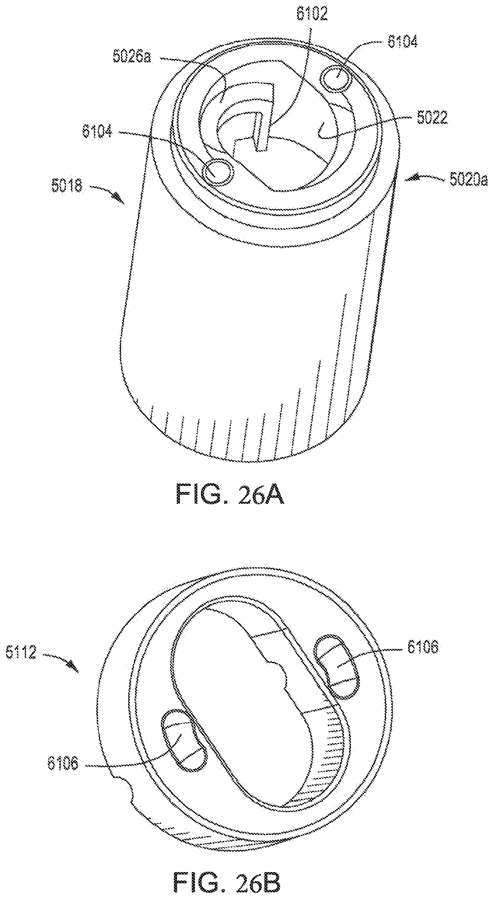

FIGS. 26A and 26B are isometric views of the sensor cap 5018 and the collar 5112, respectively, according to one or more embodiments. Referring to FIG. 26A, in some embodiments, the sensor cap 5018 may comprise an injection molded part. This may prove advantageous in molding the internal threads 5026a defined within the inner chamber 5022, as opposed to installing a threaded core or threading the inner chamber 5022. In some embodiments, one or more stop ribs 6102 (on visible) may be defined within the inner chamber 5022 to prevent over travel relative to mating member 5016 of the sharp hub 5014 (FIGS. 14A-14B).

Referring to both FIGS. 26A and 26B, in some embodiments, one or more protrusions 6104 (two shown) may be defined on the first end 5020a of the sensor cap 5018 and configured to mate with one or more corresponding indentations 6106 (two shown) defined on the collar 5112. In other embodiments, however, the protrusions 6104 may instead be defined on the collar 5112 and the indentations 6106 may be defined on the sensor cap 5018, without departing from the scope of the disclosure.

The matable protrusions 6104 and indentations 6106 may prove advantageous in rotationally locking the sensor cap 5018 to prevent unintended unscrewing of the sensor cap 5018 from the collar 5112 (and thus the sensor control device 5002) during the life of the sensor applicator 102 and through all phases of operation/assembly. **In** some embodiments, as illustrated, the indentations 6106 may be formed or otherwise defined in the general shape of a kidney bean. This may prove advantageous in allowing for some over-rotation of the sensor cap 5018 relative to the collar 5112. Alternatively, the same benefit may be achieved via a flat end threaded engagement between the two parts.

Embodiments disclosed herein include:
A. A sensor control device that includes an electronics housing, a sensor arranged within the electronics housing and having a in vivo portion extending from a bottom of the electronics housing, a sharp extending through the electronics housing and having a sharp tip extending from the bottom of the electronics housing, and a sensor cap removably coupled at the bottom of the electronics housing and defining a sealed inner chamber that receives the in vivo portion and the sharp.
B. An analyte monitoring system that includes a sensor applicator, a sensor control device positioned within the sensor applicator and including an electronics housing, a sensor arranged within the electronics housing and having a in vivo portion extending from a bottom of the electronics housing, a sharp extending through the electronics housing and having a sharp tip extending from the bottom of the electronics housing, and a sensor cap removably coupled at the bottom of the electronics housing and defining an engagement feature and a sealed inner chamber that receives the in vivo portion and the sharp. The analyte monitoring system may further include a cap coupled to the sensor applicator and providing a cap post defining a receiver feature that receives the engagement feature upon coupling the cap to the sensor applicator, wherein removing the cap from the sensor applicator detaches the sensor cap from the electronics housing and thereby exposes the in vivo portion and the sharp tip.
C. A method of preparing an analyte monitoring system that includes loading a sensor control device into a sensor applicator, the sensor control device including an electronics housing, a sensor arranged within the electronics housing and having a in vivo portion extending from a bottom of the electronics housing, a sharp extending through the electronics housing and having a sharp tip extending from the bottom of the electronics housing, and a sensor cap removably coupled at the bottom of the electronics housing and defining a sealed inner chamber that receives the in vivo portion and the sharp. The method further including securing a cap to the sensor applicator, sterilizing the sensor control device with gaseous chemical sterilization while the sensor control device is positioned within the sensor applicator, and isolating the in vivo portion and the sharp tip within the inner chamber from the gaseous chemical sterilization.

Each of embodiments A, B, and C may have one or more of the following additional elements in any combination: Element 1 : wherein the sensor cap comprises a cylindrical body having a first end that is open to access the inner chamber, and a second end opposite the first end and providing an engagement feature engageable with a cap of a sensor applicator, wherein removing the cap from the sensor applicator correspondingly removes the sensor cap from the electronics housing and thereby exposes the in vivo portion and the sharp tip. Element 2: wherein the electronics housing includes a shell matable with a mount, the sensor control device further comprising a sharp and sensor locator defined on an inner surface of the shell, and a collar received about the sharp and sensor locator, wherein the sensor cap is removably coupled to the collar. Element 3 : wherein the sensor cap is removably coupled to the collar by one or more of an interference fit, a threaded engagement, a frangible member, and a frangible substance. Element 4: wherein an annular ridge circumscribes the sharp and sensor locator and the collar provides a column and an annular shoulder extending radially outward from the column, and wherein a seal member interposes the annular shoulder and the annular ridge to form a sealed interface. Element 5: wherein the annular ridge defines a groove and a portion of the sensor is seated within the groove, and wherein the seal member extends into the groove to seal about the portion of the sensor. Element 6: wherein the seal member is a first seal member, the sensor control device further comprising a second seal member interposing the annular shoulder and a portion of the mount to form a sealed interface. Element 7: wherein the electronics housing includes a shell matable with a mount, the sensor control device further comprising a sharp hub that carries the sharp and is engageable with a top surface of the shell, and a mating member defined by the sharp hub and extending from the bottom of the electronics housing, wherein the sensor cap is removably coupled to the mating member. Element 8: further comprising a collar at least partially receivable within an aperture defined in the mount and sealingly engaging the sensor cap and an inner surface of the shell. Element 9: wherein a seal member interposes the collar and the inner surface of the shell to form a sealed interface. Element 10: wherein the collar defines a groove and a portion of the sensor is seated within the groove, and wherein the seal member extends into the groove to seal about the portion of the sensor.

Element 11 : wherein the receiver feature comprises one or more compliant members that flex to receive the engagement feature, and wherein the one or more compliant members prevent the engagement feature from exiting the cap post upon removing the cap from the sensor applicator. Element 12: further comprising a ramped surface defined on at least one of the one or more compliant members, and one or more camming surfaces provided by the engagement feature and engageable with the ramped surface, wherein the ramped surface and the one or more camming surfaces allow the cap and the cap post to rotate relative to the sensor cap in a first direction, but prevent the cap and the cap post from rotating relative to the sensor cap in a second direction opposite the first direction. Element 13 : wherein the electronics housing includes a shell matable with a mount, the sensor control device further comprising a sharp hub that carries the sharp and is engageable with a top surface of the shell, and a mating member defined by the sharp hub and extending from the bottom of the electronics housing, wherein the sensor cap is removably coupled to the mating member and rotating the cap in the second direction detaches the sensor cap from the mating member. Element 14: wherein the electronics housing includes a shell matable with a mount and the sensor control device further includes a sharp and sensor locator defined on an inner surface of the shell, and a collar received about the sharp and sensor locator, wherein the sensor cap is removably coupled to the collar.

Element 15: wherein the cap provides a cap post defining a receiver feature and the sensor cap defines an engagement feature, the method further comprising receiving the engagement feature with the receiver feature as the cap is secured to the sensor applicator. Element 16: further comprising removing the cap from the sensor applicator, and engaging the engagement feature on the receiver feature as the cap is being removed and thereby detaching the sensor cap from the electronics housing and exposing the in vivo portion and the sharp tip. Element 17: wherein loading the sensor control device into a sensor applicator is preceded by sterilizing the in vivo portion and the sharp tip with radiation sterilization, and sealing the in vivo portion and the sharp tip within the inner chamber.

By way of non-limiting example, exemplary combinations applicable to A, B, and C include: Element 2 with Element 3; Element 2 with Element 4; Element 4 with Element 5; Element 4 with Element 6; Element 7 with Element 8; Element 8 with Element 9; Element 9 with Element 10; Element 11 with Element 12; and Element 15 with Element 16.

### Example Embodiments of Seal Arrangement for Analyte Monitoring Systems

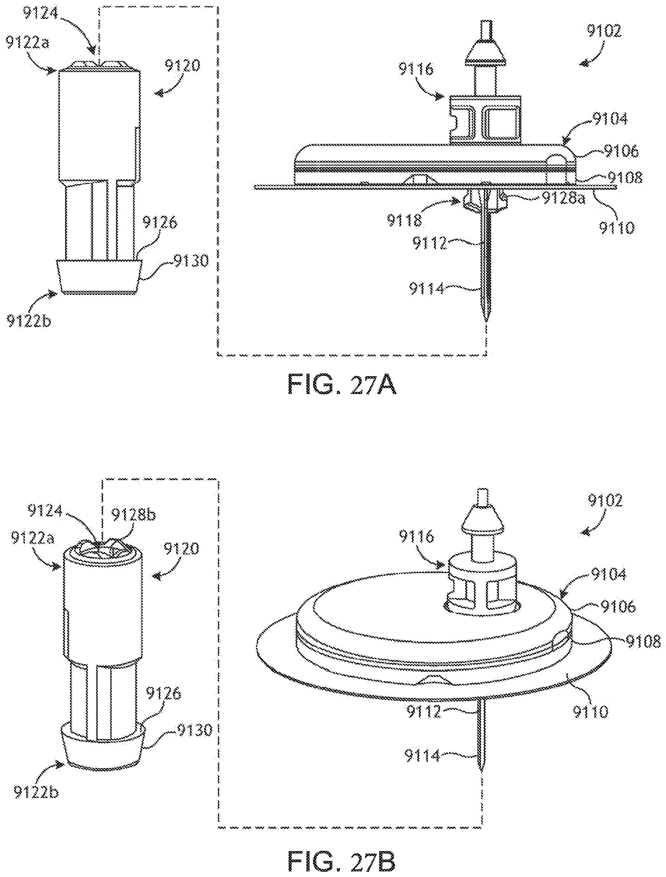

FIGS. 27A and 27B are side and isometric views, respectively, of an example sensor control device 9102, according to one or more embodiments of the present disclosure. The sensor control device 9102 may be similar in some respects to the sensor control device 102 of FIG. 1 and therefore may be best understood with reference thereto. Moreover, the sensor control device 9102 may replace the sensor control device 102 of FIG. 1 and, therefore, may be used in conjunction with the sensor applicator 102 of FIG. 1, which may deliver the sensor control device 9102 to a target monitoring location on a user's skin.

As illustrated, the sensor control device 9102 includes an electronics housing 9104, which may be generally disc-shaped and have a circular cross-section. In other embodiments, however, the electronics housing 9104 may exhibit other cross-sectional shapes, such as ovoid, oval, or polygonal, without departing from the scope of the disclosure. The electronics housing 9104 includes a shell 9106 and a mount 9108 that is matable with the shell 9106. The shell 9106 may be secured to the mount 9108 via a variety of ways, such as a snap fit engagement, an interference fit, sonic welding, laser welding, one or more mechanical fasteners (e.g., screws), a gasket, an adhesive, or any combination thereof. In some cases, the shell 9106 may be secured to the mount 9108 such that a sealed interface is generated therebetween. An adhesive patch 9110 may be positioned on and otherwise attached to the underside of the mount 9108. Similar to the adhesive patch 105 of FIG. 1, the adhesive patch 9110 may be configured to secure and maintain the sensor control device 9102 in position on the user's skin during operation.

The sensor control device 9102 may further include a sensor 9112 and a sharp 9114 used to help deliver the sensor 9112 transcutaneously under a user's skin during application of the sensor control device 9102. Corresponding portions of the sensor 9112 and the sharp 9114 extend distally from the bottom of the electronics housing 9104 (e.g., the mount 9108). A sharp hub 9116 may be overmolded onto the sharp 9114 and configured to secure and carry the sharp 9114. As best seen in FIG. 27A, the sharp hub 9116 may include or otherwise define a mating member 9118. In assembling the sharp 9114 to the sensor control device 9102, the sharp 9114 may be advanced axially through the electronics housing 9104 until the sharp hub 9116 engages an upper surface of the electronics housing 9104 or an internal component thereof and the mating member 9118 extends distally from the bottom of the mount 9108. As described herein below, in at least one embodiment, the sharp hub 9116 may sealingly engage an upper portion of a seal overmolded onto the mount 9108. As the sharp 9114 penetrates the electronics housing 9104, the exposed portion of the sensor 9112 may be received within a hollow or recessed (arcuate) portion of the sharp 9114. The remaining portion of the sensor 9112 is arranged within the interior of the electronics housing 9104.

The sensor control device 9102 may further include a sensor cap 9120, shown detached from the electronics housing 9104 in FIGS. 27A-27B. The sensor cap 9120 may help provide a sealed barrier that surrounds and protects exposed portions of the sensor 9112 and the sharp 9114. As illustrated, the sensor cap 9120 may comprise a generally cylindrical body having a first end 9122a and a second end 9122b opposite the first end 9122a. The first end 9122a may be open to provide access into an inner chamber 9124 defined within the body. In contrast, the second end 9l22b may be closed and may provide or otherwise define an engagement feature 9126. As described in more detail below, the engagement feature 9126 may help mate the sensor cap 9120 to an applicator cap of a sensor applicator (e.g., the sensor applicator 102 of FIG. 1), and may help remove the sensor cap 9120 from the sensor control device 9102 upon removing the sensor cap from the sensor applicator.

The sensor cap 9120 may be removably coupled to the electronics housing 9104 at or near the bottom of the mount 9108. More specifically, the sensor cap 9120 may be removably coupled to the mating member 9118, which extends distally from the bottom of the mount 9108. In at least one embodiment, for example, the mating member 9118 may define a set of external threads 9l28a (FIG. 27A) matable with a set of internal threads 9l28b (FIG. 27B) defined within the inner chamber 9124 of the sensor cap 9120. In some embodiments, the external and internal threads 9l28a,b may comprise a flat thread design (e.g., lack of helical curvature), but may alternatively comprise a helical threaded engagement. Accordingly, in at least one embodiment, the sensor cap 9120 may be threadably coupled to the sensor control device 9102 at the mating member 9118 of the sharp hub 9116. In other embodiments, the sensor cap 9120 may be removably coupled to the mating member 9118 via other types of engagements including, but not limited to, an interference or friction fit, or a frangible member or substance (e.g., wax, an adhesive, etc.) that may be broken with minimal separation force (e.g., axial or rotational force).

In some embodiments, the sensor cap 9120 may comprise a monolithic (singular) structure extending between the first and second ends 9l22a,b. In other embodiments, however, the sensor cap 9120 may comprise two or more component parts. In the illustrated embodiment, for example, the body of the sensor cap 9120 may include a desiccant cap 9130 arranged at the second end 9l22b. The desiccant cap 9130 may house or comprise a desiccant to help maintain preferred humidity levels within the inner chamber 9124. Moreover, the desiccant cap 9130 may also define or otherwise provide the engagement feature 9126 of the sensor cap 9120. In at least one embodiment, the desiccant cap 9130 may comprise an elastomeric plug inserted into the bottom end of the sensor cap 9120.

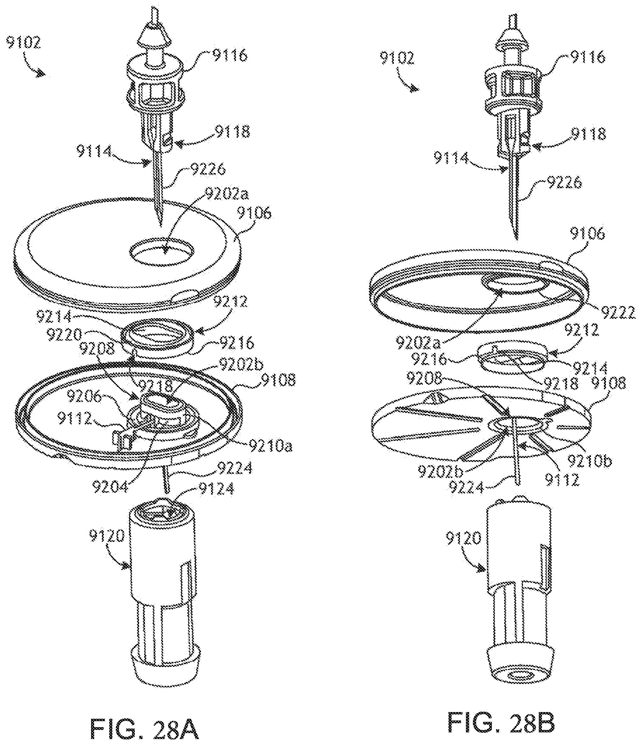

FIGS. 28A and 28B are exploded, isometric top and bottom views, respectively, of the sensor control device 9102, according to one or more embodiments. The shell 9106 and the mount 9108 operate as opposing clamshell halves that enclose or otherwise substantially encapsulate various electronic components (not shown) of the sensor control device 9102. Example electronic components that may be arranged between the shell 9106 and the mount 9108 include, but are not limited to, one or more batteries, resistors, transistors, capacitors, inductors, diodes, and switches.

The shell 9106 may define a first aperture 9202a and the mount 9108 may define a second aperture 9202b, and the apertures 9202a, b may align when the shell 9106 is properly mounted to the mount 9108. As best seen in FIG. 28A, the mount 9108 may provide or otherwise define a pedestal 9204 that protrudes from the inner surface of the mount 9108 at the second aperture 9202b. The pedestal 9204 may define at least a portion of the second aperture 9202b. Moreover, a channel 9206 may be defined on the inner surface of the mount 9108 and may circumscribe the pedestal 9202. In the illustrated embodiment, the channel 9206 is circular in shape, but could alternatively be another shape, such as oval, ovoid, or polygonal.

The mount 9108 may comprise a molded part made of a rigid material, such as plastic or metal. In some embodiments, a seal 9208 may be overmolded onto the mount 9108 and may be made of an elastomer, rubber, a -polymer, or another pliable material suitable for facilitating a sealed interface. In embodiments where the mount 9108 is made of a plastic, the mount 9108 may be molded in a first "shot" of injection molding, and the seal 9208 may be overmolded onto the mount 9108 in a second "shot" of injection molding. Accordingly, the mount 9108 may be referred to or otherwise characterized as a "two-shot mount."

In the illustrated embodiment, the seal 9208 may be overmolded onto the mount 9108 at the pedestal 9204 and also on the bottom of the mount 9108. More specifically, the seal 9208 may define or otherwise provide a first seal element 92l0a overmolded onto the pedestal 9204, and a second seal element 9210b (FIG. 28B) interconnected to (with) the first seal element 92l0a and overmolded onto the mount 9108 at the bottom of the mount 9108. In some embodiments, one or both of the seal elements 92l0a,b may help form corresponding portions (sections) of the second aperture 9202b. While the seal 9208 is described herein as being overmolded onto the mount 9108, it is also contemplated herein that one or both of the seal elements 92l0a,b may comprise an elastomeric component part independent of the mount 9208, such as an O-ring or a gasket.

The sensor control device 9102 may further include a collar 9212, which may be a generally annular structure that defines a central aperture 9214. The central aperture 9214 may be sized to receive the first seal element 92l0a and may align with both the first and second apertures 9202a, b when the sensor control device 9102 is properly assembled. The shape of the central aperture 9214 may generally match the shape of the second aperture 9202b and the first seal element 92l0a.

In some embodiments, the collar 9212 may define or otherwise provide an annular lip 9216 on its bottom surface. The annular lip 9216 may be sized and otherwise configured to mate with or be received into the channel 9206 defined on the inner surface of the mount 9108. In some embodiments, a groove 9218 may be defined on the annular lip 9216 and may be configured to accommodate or otherwise receive a portion of the sensor 9112 extending laterally within the mount 9108. In some embodiments, the collar 9212 may further define or otherwise provide a collar channel 9220 (FIG. 28A) on its upper surface sized to receive and otherwise mate with an annular ridge 9222 (FIG. 28B) defined on the inner surface of the shell 9106 when the sensor control device 9102 is properly assembled.

The sensor 9112 may include a in vivo portion 9224 that extends through the second aperture 9202b defined in the mount 9108 to be transcutaneously received beneath a user's skin. The in vivo portion 9224 may have an enzyme or other chemistry included thereon to help facilitate analyte monitoring. The sharp 9114 may include a sharp tip 9226 extendable through the first aperture 9202a defined by the shell 9106. As the sharp tip 9226 penetrates the electronics housing 9104, the in vivo portion 9224 of the sensor 9112 may be received within a hollow or recessed portion of the sharp tip 9226. The sharp tip 9226 may be configured to penetrate the skin while carrying the in vivo portion 9224 to put the active chemistry of the in vivo portion 9224 into contact with bodily fluids.

The sensor control device 9102 may provide a sealed subassembly that includes, among other component parts, portions of the shell 9106, the sensor 9112, the sharp 9114, the seal 9208, the collar 9212, and the sensor cap 9120. The sealed subassembly may help isolate the sensor 9112 and the sharp 9114 within the inner chamber 9124 (FIG. 28A) of the sensor cap 9120. In assembling the sealed subassembly, the sharp tip 9226 is advanced through the electronics housing 9104 until the sharp hub 9116 engages the seal 9208 and, more particularly, the first seal element 92l0a. The mating member 9118 provided at the bottom of the sharp hub 9116 may extend out the second aperture 9202b in the bottom of the mount 9108, and the sensor cap 9120 may be coupled to the sharp hub 9116 at the mating member 9118. Coupling the sensor cap 9120 to the sharp hub 9116 at the mating member 9118 may urge the first end 9122a of the sensor cap 9120 into sealed engagement with the seal 9208 and, more particularly, into sealed engagement with the second seal element 9210b on the bottom of the mount 9108. In some embodiments, as the sensor cap 9120 is coupled to the sharp hub 9116, a portion of the first end 9l22a of the sensor cap 9120 may bottom out (engage) against the bottom of the mount 9108, and the sealed engagement between the sensor hub 9116 and the first seal element 92l0a may be able to assume any tolerance variation between features.

FIG. 29 is a cross-sectional side view of the sensor control device 9102, according to one or more embodiments. As indicated above, the sensor control device 9102 may include or otherwise incorporate a sealed subassembly 9302, which may be useful in isolating the sensor 9112 and the sharp 9114 within the inner chamber 9124 of the sensor cap 9120. To assemble the sealed subassembly 9302, the sensor 9112 may be located within the mount 9108 such that the in vivo portion 9224 extends through the second aperture 9202b at the bottom of the mount 9108. In at least one embodiment, a locating feature 9304 may be defined on the inner surface of the mount 9108, and the sensor 9112 may define a groove 9306 that is matable with the locating feature 9304 to properly locate the sensor 9112 within the mount 9108.

Once the sensor 9112 is properly located, the collar 9212 may be installed on the mount 9108. More specifically, the collar 9212 may be positioned such that the first seal element 92l0a of the seal 9208 is received within the central aperture 9214 defined by the collar 9212 and the first seal element 92l0a generates a radial seal against the collar 9212 at the central aperture 9214. Moreover, the annular lip 9216 defined on the collar 9212 may be received within the channel 9206 defined on the mount 9108, and the groove 9218 defined through the annular lip 9216 may be aligned to receive the portion of the sensor 9112 that traverses the channel 9206 laterally within the mount 9108. In some embodiments, an adhesive may be injected into the channel 9206 to secure the collar 9212 to the mount 9108. The adhesive may also facilitate a sealed interface between the two components and generate a seal around the sensor 9112 at the groove 9218, which may isolate the in vivo portion 9224 from the interior of the electronics housing 9104.

The shell 9106 may then be mated with or otherwise coupled to the mount 9108. In some embodiments, as illustrated, the shell 9106 may mate with the mount 9108 via a tongue- and-groove engagement 9308 at the outer periphery of the electronics housing 9104. An adhesive may be injected (applied) into the groove portion of the engagement 9308 to secure the shell 9106 to the mount 9108, and also to create a sealed engagement interface. Mating the shell 9106 to the mount 9108 may also cause the annular ridge 9222 defined on the inner surface of the shell 9106 to be received within the collar channel 9220 defined on the upper surface of the collar 9212. In some embodiments, an adhesive may be injected into the collar channel 9220 to secure the shell 9106 to the collar 9212, and also to facilitate a sealed interface between the two components at that location. When the shell 9106 mates with the mount 9108, the first seal element 92l0a may extend at least partially through (into) the first aperture 9202a defined in the shell 9106.

The sharp 9114 may then be coupled to the sensor control device 9102 by extending the sharp tip 9226 through the aligned first and second apertures 9202a, b defined in the shell 9106 and the mount 9108, respectively. The sharp 9114 may be advanced until the sharp hub 9116 engages the seal 9208 and, more particularly, engages the first seal element 92l0a. The mating member 9118 may extend (protrude) out the second aperture 9202b at the bottom of the mount 9108 when the sharp hub 9116 engages the first seal element 92l0a.

The sensor cap 9120 may then be removably coupled to the sensor control device 9102 by threadably mating the internal threads 9128b of the sensor cap 9120 with the external threads 9l28a of the mating member 9118. The inner chamber 9124 may be sized and otherwise configured to receive the in vivo portion 9224 and the sharp tip 9226 extending from the bottom of the mount 9108. Moreover, the inner chamber 9124 may be sealed to isolate the in vivo portion 9224 and the sharp tip 9226 from substances that might adversely interact with the chemistry of the in vivo portion 9224. In some embodiments, a desiccant (not shown) may be present within the inner chamber 9124 to maintain proper humidity levels.

Tightening (rotating) the mated engagement between the sensor cap 9120 and the mating member 9118 may urge the first end 9122a of the sensor cap 9120 into sealed engagement with the second seal element 9210b in an axial direction (e.g., along the centerline of the apertures 9202a, b), and may further enhance the sealed interface between the sharp hub 9116 and the first seal element 92l0a in the axial direction. Moreover, tightening the mated engagement between the sensor cap 9120 and the mating member 9118 may compress the first seal element 92l0a, which may result in an enhanced radial sealed engagement between the first seal element 92l0a and the collar 9212 at the central aperture 9214. Accordingly, in at least one embodiment, the first seal element 92l0a may help facilitate axial and radial sealed engagements.

As mentioned above, the first and second seal elements 92l0a,b may be overmolded onto the mount 9108 and may be physically linked or otherwise interconnected. Consequently, a single injection molding shot may flow through the second aperture 9202b of the mount 9108 to create both ends of the seal 9208. This may prove advantageous in being able to generate multiple sealed interfaces with only a single injection molded shot. An additional advantage of a two-shot molded design, as opposed to using separate elastomeric components (e.g., O-rings, gaskets, etc.), is that the interface between the first and second shots is a reliable bond rather than a mechanical seal. Hence, the effective number of mechanical sealing barriers is effectively cut in half. Moreover, a two-shot component with a single elastomeric shot also has implications to minimizing the number of two-shot components needed to achieve all the necessary sterile barriers. Once properly assembled, the sealed subassembly 9302 may be subjected to a radiation sterilization process to sterilize the sensor 9112 and the sharp 9114. The sealed subassembly 9302 may be subjected to the radiation sterilization prior to or after coupling the sensor cap 9120 to the sharp hub 9116. When sterilized after coupling the sensor cap 9120 to the sharp hub 9116, the sensor cap 9120 may be made of a material that permits the propagation of radiation therethrough. In some embodiments, the sensor cap 9120 may be transparent or translucent, but can otherwise be opaque, without departing from the scope of the disclosure.

FIG. 29A is an exploded isometric view of a portion of another embodiment of the sensor control device 9102 of FIGS. 27A-27B and 28A-28B. Embodiments included above describe the mount 9108 and the seal 9208 being manufactured via a two-shot injection molding process. In other embodiments, however, as briefly mentioned above, one or both of the seal elements 92l0a,b of the seal 9208 may comprise an elastomeric component part independent of the mount 9208. In the illustrated embodiment, for example, the first seal element 92l0a may be overmolded onto the collar 9212 and the second seal element 92l0b may be overmolded onto the sensor cap 9120. Alternatively, the first and second seal elements 92l0a,b may comprise a separate component part, such as a gasket or O-ring positioned on the collar 9212 and the sensor cap 9120, respectively. Tightening (rotating) the mated engagement between the sensor cap 9120 and the mating member 9118 may urge the second seal element 9210b into sealed engagement with the bottom of the mount 9108 in an axial direction, and may enhance a sealed interface between the sharp hub 9116 and the first seal element 92l0a in the axial direction.

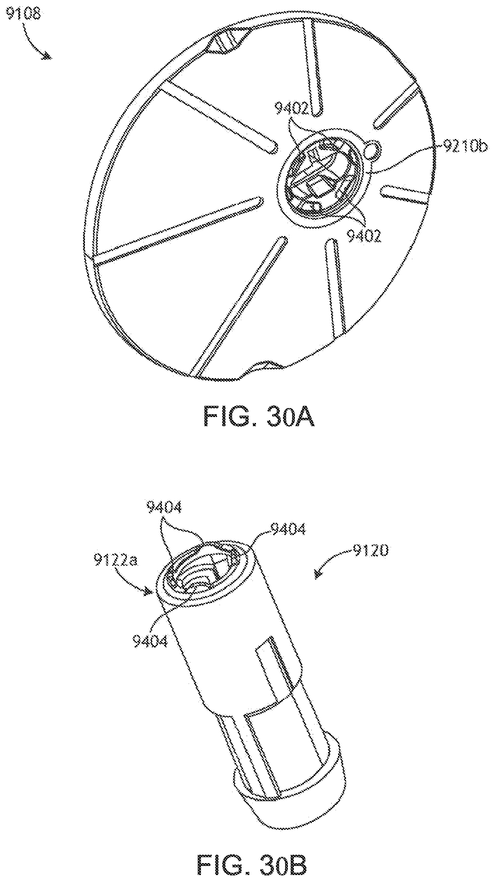

FIG. 30A is an isometric bottom view of the mount 9108, and FIG. 30B is an isometric top view of the sensor cap 9120, according to one or more embodiments. As shown in FIG. 30A, the mount 9108 may provide or otherwise define one or more indentations or pockets 9402 at or near the opening to the second aperture 9202b. As shown in FIG. 30B, the sensor cap 9120 may provide or otherwise define one or more projections 9404 at or near the first end 9l22a of the sensor cap 9120. The projections 9404 may be received within the pockets 9402 when the sensor cap 9120 is coupled to the sharp hub 9116 (FIGS. 28A-28B and 93). More specifically, as described above, as the sensor cap 9120 is coupled to the mating member 9118 (FIGS. 28A-28B and 93) of the sensor hub 9116, the first end 9l22a of the sensor cap 9120 is brought into sealed engagement with the second seal element 92l0b. In this process, the projections 9404 may also be received within the pockets 9402, which may help prevent premature unthreading of the sensor cap 9120 from the sharp hub 9116.

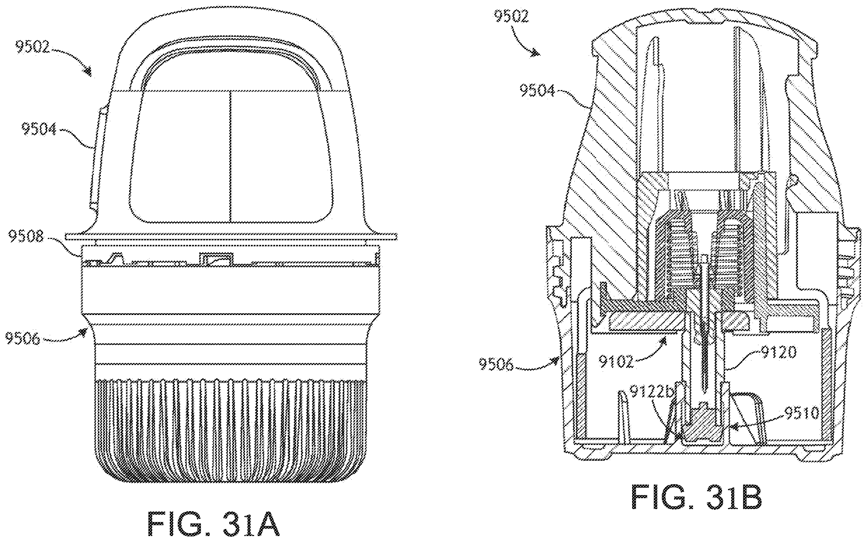

FIGS. 31A and 31B are side and cross-sectional side views, respectively, of an example sensor applicator 9502, according to one or more embodiments. The sensor applicator 9502 may be similar in some respects to the sensor applicator 102 of FIG. 1 and, therefore, may be designed to deliver (fire) a sensor control device, such as the sensor control device 9102. FIG. 31A depicts how the sensor applicator 9502 might be shipped to and received by a user, and FIG. 31B depicts the sensor control device 9102 arranged within the interior of the sensor applicator 9502.

As shown in FIG. 31A, the sensor applicator 9502 includes a housing 9504 and an applicator cap 9506 removably coupled to the housing 9504. In some embodiments, the applicator cap 9506 may be threaded to the housing 9504 and include a tamper ring 9508. Upon rotating (e.g., unscrewing) the applicator cap 9506 relative to the housing 9504, the tamper ring 9508 may shear and thereby free the applicator cap 9506 from the sensor applicator 9502.

In FIG. 31B, the sensor control device 9102 is positioned within the sensor applicator 9502. Once the sensor control device 9102 is fully assembled, it may then be loaded into the sensor applicator 9502 and the applicator cap 9506 may be coupled to the sensor applicator 9502. In some embodiments, the applicator cap 9506 and the housing 9504 may have opposing, matable sets of threads that enable the applicator cap 9506 to be screwed onto the housing 9504 in a clockwise (or counter-clockwise) direction and thereby secure the applicator cap 9506 to the sensor applicator 9502.

Securing the applicator cap 9506 to the housing 9504 may also cause the second end 9l22b of the sensor cap 9120 to be received within a cap post 9510 located within the interior of the applicator cap 9506 and extending proximally from the bottom thereof. The cap post 9510 may be configured to receive at least a portion of the sensor cap 9120 as the applicator cap 9506 is coupled to the housing 9504.

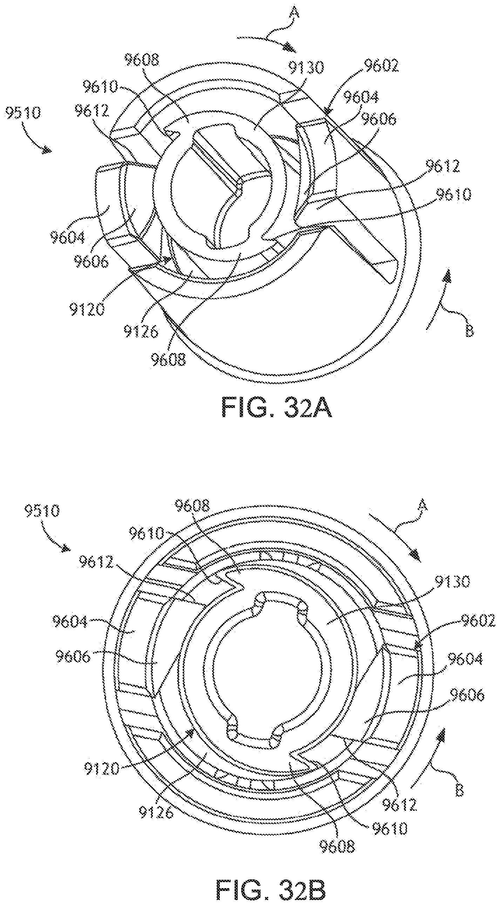

FIGS. 32A and 32B are perspective and top views, respectively, of the cap post 9510, according to one or more additional embodiments. In the illustrated depiction, a portion of the sensor cap 9120 is received within the cap post 9510 and, more specifically, the desiccant cap 9130 of the sensor cap 9120 is arranged within cap post 9510. The cap post 9510 may define a receiver feature 9602 configured to receive the engagement feature 9126 of the sensor cap 9120 upon coupling (e.g., threading) the applicator cap 9506 (FIG. 31B) to the sensor applicator 9502 (FIGS. 31A-31B). Upon removing the applicator cap 9506 from the sensor applicator 9502, however, the receiver feature 9602 may prevent the engagement feature 9126 from reversing direction and thus prevent the sensor cap 9120 from separating from the cap post 9510. Instead, removing the applicator cap 9506 from the sensor applicator 9502 will simultaneously detach the sensor cap 9120 from the sensor control device 9102 (FIGS. 27A-27B and 28A-28B), and thereby expose the distal portions of the sensor 9112 (28A-28B) and the sharp 9114 (FIGS. 28A-28B).

Many design variations of the receiver feature 9602 may be employed, without departing from the scope of the disclosure. In the illustrated embodiment, the receiver feature 9602 includes one or more compliant members 9604 (two shown) that are expandable or flexible to receive the engagement feature 9126. The engagement feature 9126 may comprise, for example, an enlarged head and the compliant member(s) 9604 may comprise a collet-type device that includes a plurality of compliant fingers configured to flex radially outward to receive the enlarged head.

The compliant member(s) 9604 may further provide or otherwise define corresponding ramped surfaces 9606 configured to interact with one or more opposing camming surfaces 9608 provided on the outer wall of the engagement feature 9126. The configuration and alignment of the ramped surface(s) 9606 and the opposing camming surface(s) 9608 is such that the applicator cap 9506 is able to rotate relative to the sensor cap 9120 in a first direction A (e.g., clockwise), but the cap post 9510 binds against the sensor cap 9120 when the applicator cap 9506 is rotated in a second direction B (e.g., counter clockwise). More particularly, as the applicator cap 9506 (and thus the cap post 9510) rotates in the first direction A, the camming surfaces 9608 engage the ramped surfaces 9606, which urge the compliant members 9604 to flex or otherwise deflect radially outward and results in a ratcheting effect. Rotating the applicator cap 9506 (and thus the cap post 9510) in the second direction B, however, will drive angled surfaces 9610 of the camming surfaces 9608 into opposing angled surfaces 9612 of the ramped surfaces 9606, which results in the sensor cap 9120 binding against the compliant member(s) 9604.

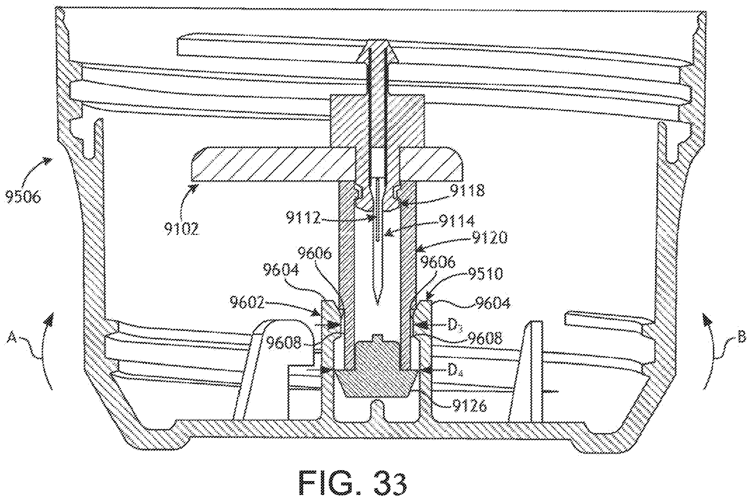

FIG. 33 is a cross-sectional side view of the sensor control device 9102 positioned within the applicator cap 9506, according to one or more embodiments. As illustrated, the opening to the receiver feature 9602 exhibits a first diameter D3, while the engagement feature 9126 of the sensor cap 9120 exhibits a second diameter D4 that is larger than the first diameter D3 and greater than the outer diameter of the remaining portions of the sensor cap 9120. As the sensor cap 9120 is extended into the cap post 9510, the compliant member(s) 9604 of the receiver feature 9602 may flex (expand) radially outward to receive the engagement feature 9126. In some embodiments, as illustrated, the engagement feature 9126 may provide or otherwise define an angled outer surface that helps bias the compliant member(s) 9604 radially outward. Once the engagement feature 9126 bypasses the receiver feature 9602, the compliant member(s) 9604 are able to flex back to (or towards) their natural state and thus lock the sensor cap 9120 within the cap post 9510.

As the applicator cap 9506 is threaded to (screwed onto) the housing 9504 (FIGS. 31A-31B) in the first direction A, the cap post 9510 correspondingly rotates in the same direction and the sensor cap 9120 is progressively introduced into the cap post 9510. As the cap post 9510 rotates, the ramped surfaces 9606 of the compliant members 9604 ratchet against the opposing camming surfaces 9608 of the sensor cap 9120. This continues until the applicator cap 9506 is fully threaded onto (screwed onto) the housing 9504. In some embodiments, the ratcheting action may occur over two full revolutions of the applicator cap 9506 before the applicator cap 9506 reaches its final position.

To remove the applicator cap 9506, the applicator cap 9506 is rotated in the second direction B, which correspondingly rotates the cap post 9510 in the same direction and causes the camming surfaces 9608 (i.e., the angled surfaces 9610 of FIGS. 32A-2B) to bind against the ramped surfaces 9606 (i.e., the angled surfaces 9612 of FIGS. 32A-32B). Consequently, continued rotation of the applicator cap 9506 in the second direction B causes the sensor cap 9120 to correspondingly rotate in the same direction and thereby unthread from the mating member 9118 to allow the sensor cap 9120 to detach from the sensor control device 9102. Detaching the sensor cap 9120 from the sensor control device 9102 exposes the distal portions of the sensor 9112 and the sharp 9114, and thus places the sensor control device 9102 in position for firing (use).

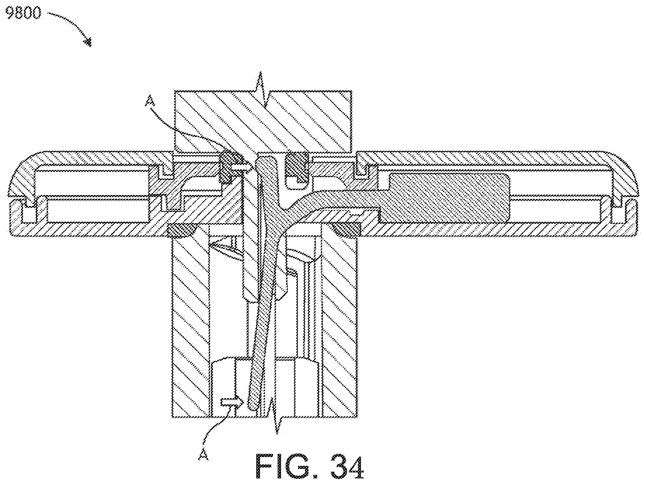

FIG. 34 is a cross-sectional view of a sensor control device 9800 showing example interaction between the sensor and the sharp. After assembly of the sharp, the sensor should sit in a channel defined by the sharp. The sensor control device in FIG. 9 does not show the sensor deflected inwards and otherwise aligned fully with the sharp, but such may be the case upon full assembly as slight bias forces may be assumed by the sensor at the locations indicated by the two arrows A. Biasing the sensor against the sharp may be advantageous so that any relative motion between the sensor and the sharp during subcutaneous insertion does not expose the sensor tip (i.e., the in vivo portion) outside the sharp channel, which could potentially cause an insertion failure.

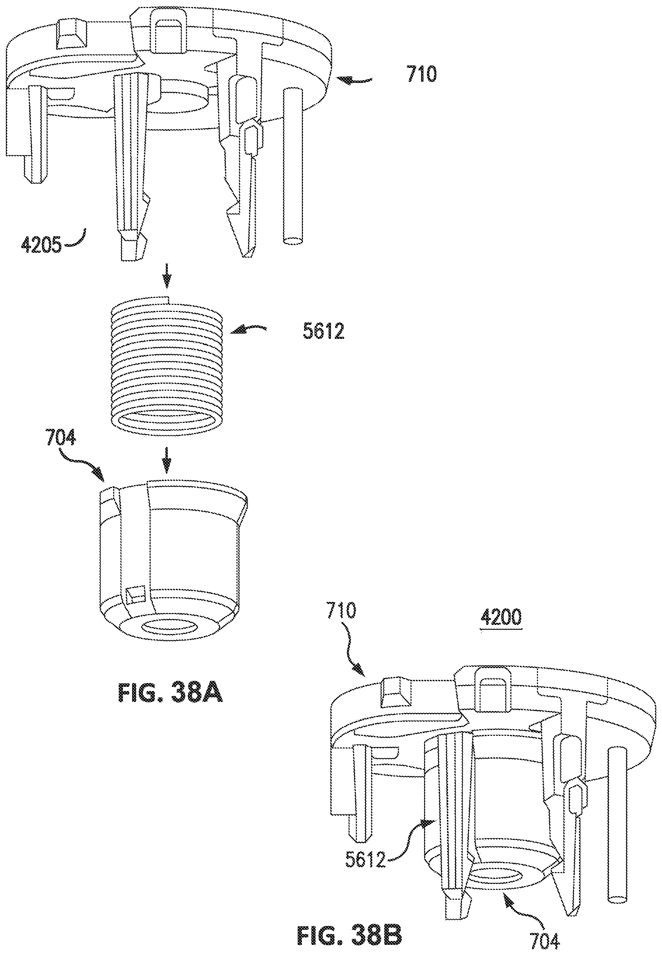

FIGS. 38A-38K illustrate steps of an example process for manufacturing an applicator assembly (e.g., an applicator device 150). The applicator assembly includes an inserter 4200, on-body sensor puck assembly (e.g., a sensor control device 5002) coupled to a puck carrier 710 (e.g., sensor electronics carrier 710 of FIG. 4A or sensor carrier 5602 of FIGS. 17A-17C), a sheath 704, an applicator housing 702, and a cap 708.

As illustrated in FIGS. 38A-38B, the manufacturing process includes assembling the inserter 4200 by loading a spring 5612 to a sharp carrier 704, lowering a puck carrier 710 to the sharp carrier 704 and compressing the spring 5612 until seated within the sharp carrier 704. The spring 5612 can be compressed manually or using a suitable compression tool, including, but not limited to a manually-operated or robotic loading arm, vacuum or suction gripping arm, magnetic gripping arm, adaptive gripping arm or appendage, pneumatic guided actuator or servo actuator, or other suitable tool. After the spring 5612 is compressed, the process involves locking one or more retention features 4205 of the puck carrier 710 with the sharp carrier 704 to retrain spring compression. The locking may be performed while clamping the puck carrier 710 to the sharp carrier 704 using any suitable clamping mechanism.

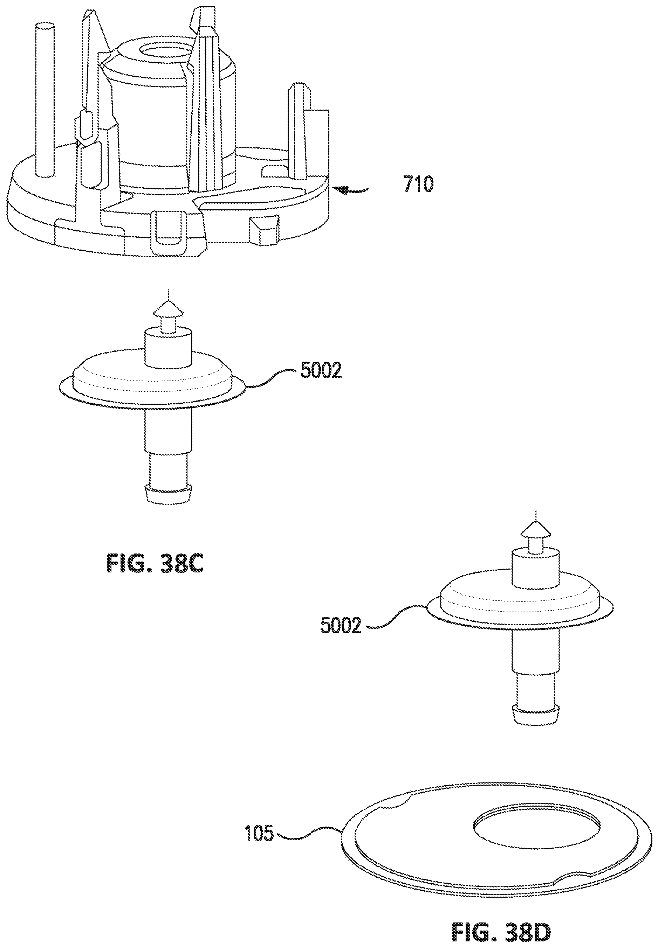

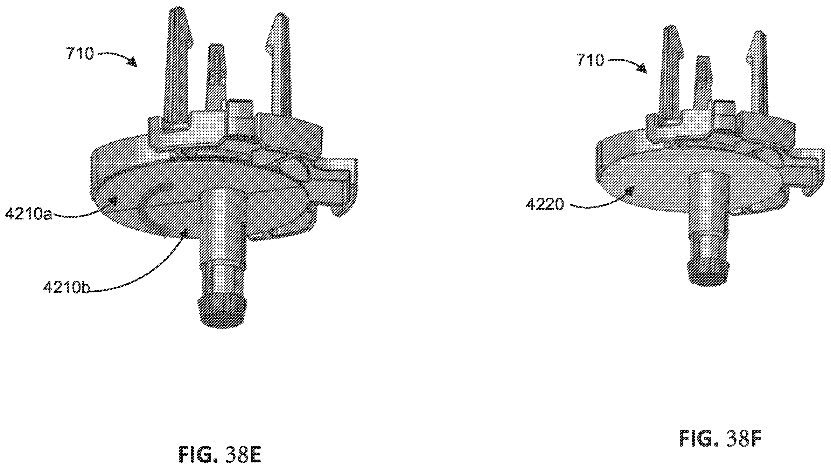

As illustrated in FIG. 38C, the manufacturing process can include coupling the on-body sensor puck assembly 5002 to the puck carrier 710. For example, mount retention features of the can be aligned with arms of the puck carrier 710 and the puck assembly 5002 can be advanced until it snaps into place. As illustrated in FIG. 38D, the manufacturing process can include applying an adhesive patch 105 (or adhesive patch 9110) to the on-body sensor puck assembly or to the puck carrier. The adhesive patch can be applied manually, or using a gripping or applicator machine tooling, vacuum or suction gripping arm, magnetic gripping arm, adaptive gripping arm or appendage, pneumatic guided actuator or servo actuator, or other suitable tool. Prior to applying the adhesive patch, the on-body sensor puck assembly (including puck carrier) and adhesive patch can be loaded into suitable holding tool. The adhesive patch can be configured to fit the contours and components of the on-body sensor puck assembly, for example, the adhesive patch can include a hold to accommodate the sharp cap. The adhesive patch can be aligned with the on-body sensor puck assembly (for example, manually, using optically-guided alignment arms, a spring-loaded alignment tool, etc.) and lowered onto the on-body sensor puck assembly manually or using suitable machine tooling, as described herein. Once the adhesive patch 105 is applied to the on-body sensor puck assembly 5002 or puck carrier 710, as illustrated in FIGS. 38E and 38F, the manufacturing process can include removing tabs 4210a and 4210b of the adhesive patch 105 to expose a side 4220 of the adhesive patch 150 that will attach, for example, to the body of a wearer, for example by securing an exposed corner of the liner and peeling from the patch manually or using automated equipment.

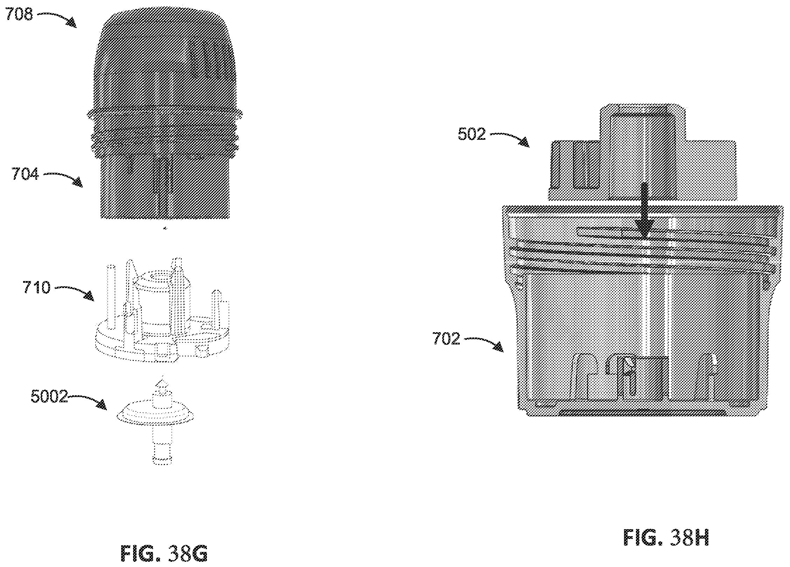

As illustrated in FIG. 38G, the manufacturing process can include attaching a sheath 704 to the puck carrier 710. Attaching the sheath the puck carrier can include loading the sheath into a fixture nest (not illustrated) and lowering the puck carrier 710 with compressed spring into the sheath 704. The manufacturing process can further include attaching the sheath 704 to the applicator housing 708. Attaching the sheath 704 to the applicator housing 708 can include loading the applicator housing 708 into a fixture nest (not illustrated) and engaging an alignment rib of the applicator housing 708 with a notch in the fixture nest. Then, the sheath 704 is lowered onto the applicator housing 708 until it engages the alignment rib of the applicator housing 708. The sheath 704 and puck carrier 710 can be manipulated manually or using suitable machine tooling, e.g., pneumatic guided actuator, to forcibly attach the components, as described herein.

As illustrated in FIG. 38H, the manufacturing process can include loading a desiccant 502 into the cap 702. The desiccant 502 can be used to control moisture exposure of the on-body sensor puck assembly 5002 and adhesive patch 105. The desiccant can be loaded manually or using suitable tooling such as a manually-operated or robotic loading arm, vacuum or suction gripping arm, magnetic gripping arm, adaptive gripping arm or appendage, pneumatic guided actuator, or other suitable tool.

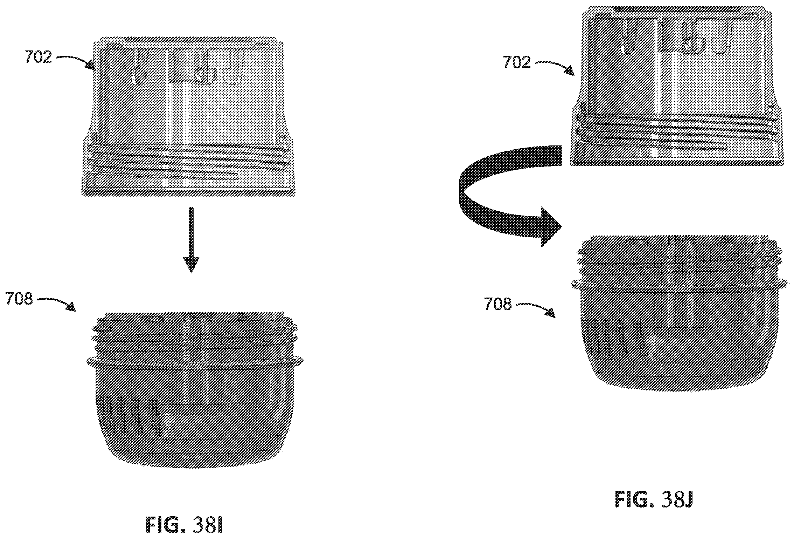

As illustrated in FIG. 38I, the manufacturing process can include coupling the cap 702 to the applicator housing 708. Coupling the cap 702 to the applicator housing 708 can include lowering the cap 702 onto the applicator housing 708. As illustrated in FIG. 38J, coupling the cap 702 to the applicator housing 708 can include lowering the cap 702 onto the applicator housing 708 and screwing the cap 702 to the applicator housing 708 to a pre-determined torque. The cap 702 can be screwed to the applicator housing 708 manually or using suitable automation tooling, for example, a servo rotary actuator can be used to rotate the cap 702 to a suitable motor torque.

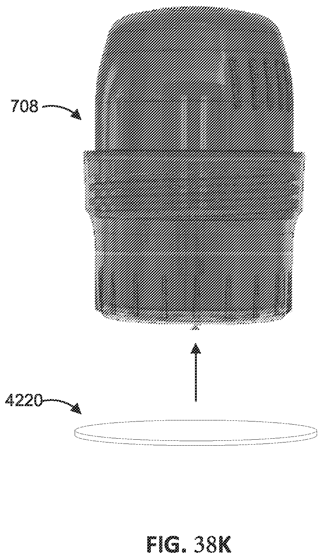

**In** particular embodiments, a tamper-evident sticker or other method of detecting that the applicator housing 702 has been opened can be applied to the interior or exterior of the applicator housing 708. As illustrated in FIG. 38K, the manufacturing process can include applying a label 4220 to the exterior of the assembled applicator housing 708.

Embodiments disclosed herein include:
D. A sensor control device that includes an electronics housing including a shell that defines a first aperture and a mount that defines a second aperture alignable with the first aperture when the shell is coupled to the mount, a seal overmolded onto the mount at the second aperture and comprising a first seal element overmolded onto a pedestal protruding from an inner surface of the mount, and a second seal element interconnected with the first seal element and overmolded onto a bottom of the mount, a sensor arranged within the electronics housing and having a in vivo portion extending through the second aperture and past the bottom of the mount, and a sharp that extends through the first and second apertures and past the bottom of the electronics housing.
E. An assembly that includes a sensor applicator, a sensor control device positioned within the sensor applicator and including an electronics housing including a shell that defines a first aperture and a mount that defines a second aperture alignable with the first aperture when the shell is mated to the mount, a seal overmolded onto the mount at the second aperture and comprising a first seal element overmolded onto a pedestal protruding from an inner surface of the mount, and a second seal element interconnected with the first seal element and overmolded onto a bottom of the mount, a sensor arranged within the electronics housing and having a in vivo portion extending through the second aperture and past the bottom of the mount, and a sharp that extends through the first and second apertures and past the bottom of the electronics housing. The assembly further including a sensor cap removably coupled to the sensor control device at the bottom of the mount and defining a sealed inner chamber that receives the in vivo portion and the sharp, and an applicator cap coupled to the sensor applicator.

Each of embodiments D and E may have one or more of the following additional elements in any combination: Element 1 : wherein the mount comprises a first injection molded part molded in a first shot, and the seal comprises a second injection molded part overmolded onto the first injection molded part in a second shot. Element 2: further comprising a sharp hub that carries the sharp and sealingly engages the first seal element, and a sensor cap removably coupled to the sharp hub at the bottom of the mount and sealingly engaging the second seal element, wherein the sensor cap defines an inner chamber that receives the in vivo portion and the sharp. Element 3 : wherein the sharp hub provides a mating member that extends past the bottom of the mount and the sensor cap is removably coupled to the mating member. Element 4: further comprising one or more pockets defined on the bottom of the mount at the second aperture, and one or more projections defined on an end of the sensor cap and receivable within the one or more pockets when the sensor cap is coupled to the sharp hub. Element 5: further comprising a collar positioned within the electronics housing and defining a central aperture that receives and sealingly engages the first seal element in a radial direction. Element 6: further comprising a channel defined on the inner surface of the mount and circumscribing the pedestal, an annular lip defined on an underside of the collar and matable with the channel, and an adhesive provided in the channel to secure and seal the collar to the mount at the channel. Element 7 : further comprising a groove defined through the annular lip to accommodate a portion of the sensor extending laterally within the mount, wherein the adhesive seals about the sensor at the groove. Element 8: further comprising a collar channel defined on an upper surface of the collar, an annular ridge defined on an inner surface of the shell and matable with the collar channel, and an adhesive provided in the collar channel to secure and seal the shell to the collar. Element 9: wherein one or both of the first and second seal elements define at least a portion of the second aperture. Element 10: wherein the first seal element extends at least partially through the first aperture when the shell is coupled to the mount.

Element 11 : wherein the sensor control device further includes a sharp hub that carries the sharp and sealingly engages the first seal element, and wherein the sensor cap is removably coupled to the sharp hub at the bottom of the mount and sealingly engages the second seal element. Element 12: wherein the sensor control device further includes one or more pockets defined on the bottom of the mount at the second aperture, and one or more projections defined on an end of the sensor cap and receivable within the one or more pockets when the sensor cap is coupled to the sharp hub. Element 13 : wherein the sensor control device further includes a collar positioned within the electronics housing and defining a central aperture that receives and sealingly engages the first seal element in a radial direction. Element 14: wherein the sensor control device further includes a channel defined on the inner surface of the mount and circumscribing the pedestal, an annular lip defined on an underside of the collar and matable with the channel, and an adhesive provided in the channel to secure and seal the collar to the mount at the channel. Element 15: wherein the sensor control device further includes a groove defined through the annular lip to accommodate a portion of the sensor extending laterally within the mount, and wherein the adhesive seals about the sensor at the groove. Element 16: wherein the sensor control device further includes a collar channel defined on an upper surface of the collar, an annular ridge defined on an inner surface of the shell and matable with the collar channel, and an adhesive provided in the collar channel to secure and seal the shell to the collar. Element 17: wherein one or both of the first and second seal elements define at least a portion of the second aperture. Element 18: wherein the first seal element extends at least partially through the first aperture.

By way of non-limiting example, exemplary combinations applicable to D and E include: Element 2 with Element 3; Element 2 with Element 4; Element 5 with Element 6; Element 6 with Element 7; Element 5 with Element 8; Element 11 with Element 12; Element 13 with Element 14; Element 14 with Element 15; and Element 13 with Element 16.

Additional details of suitable devices, systems, methods, components and the operation thereof along with related features are set forth in International Publication No. WO2018/136898 to Rao et. al., International Publication No. WO2019/236850 to Thomas et. al., International Publication No. WO2019/236859 to Thomas et. al., International Publication No. WO2019/236876 to Thomas et. al., and U.S. Patent Application No. 16/433,931.

### Example Embodiments of Sensor Structures and Related Manufacturing Processes

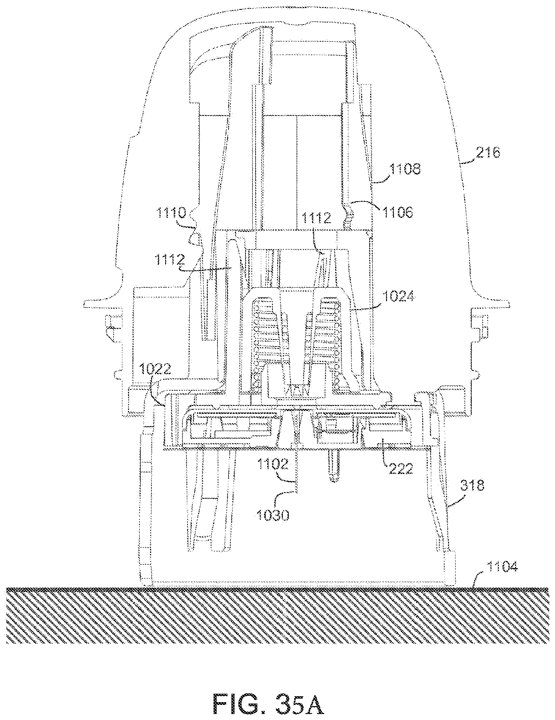

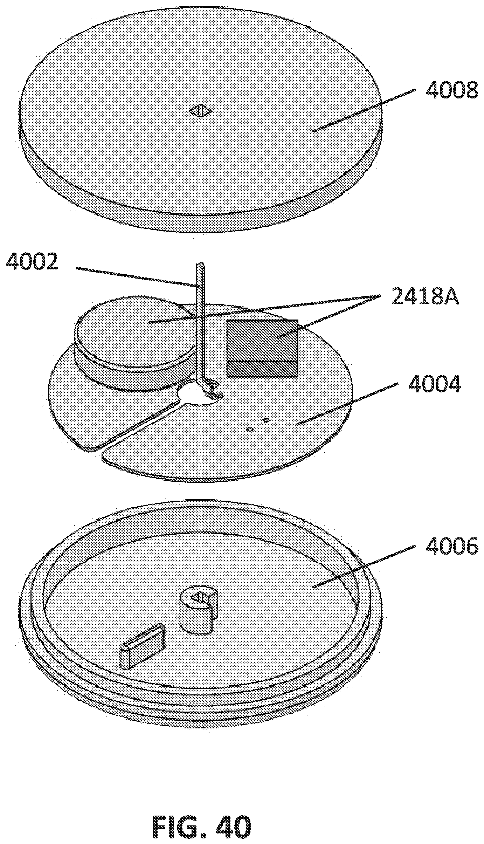

Example embodiments of sensor structures and related manufacturing processes will now be described, as depicted in Figs. 9B and 11H. In accordance with disclosed subject matter, a system for measurement of an analyte level is provided comprising an analyte sensor (e.g., sensor 104A, sensor 11900A) having an in vivo portion 4002 and an ex vivo portion 4004. The in vivo portion 4002 can have a first surface and a second surface, and can be configured to be positioned in contact with an interstitial fluid of a user and to generate signals associated with a measured analyte level. The ex vivo portion 4004 comprises a plurality of electronic components (2418A, 11914A) mounted thereon. In this way, and as shown in FIG. 40, an integral, monolithic sensor having an in vivo portion comprising a substrate with one or more electrodes printed thereon and an ex vivo portion having the substrate with electronic components mounted thereon can be formed. An integral, monolithic sensor as described herein can be advantageous in reducing the number of required components, thereby reducing the overall size of the sensor control device, reducing manufacturing complexity and cost, and potentially increasing user access to these devices. By reducing the size, comfort and convenience to the user can be improved. For example, previous embodiments disclose an analyte sensor having ex vivo portion 2404, 11904 configured to electrically couple with a circuit board using an electrical connector, as shown in FIGS. 9A and 35A. According to embodiments disclosed here, mounting the electronic components on the substrate of the analyte sensor on the ex vivo portion 4004 to comprise a single component eliminates the need for a connector, thereby enabling reduction of the size of the sensor control device 102 and increasing the reliability of the connection between the electronic components and the circuit board. By eliminating the need for a connector, and by shrinking the size of the overall sensor control device 102, manufacturing and purchase costs can be reduced. Indeed, in order to measure multiple analytes, one working electrode for each analyte is required. As a result, a sensor configured to measure multiple analytes includes a corresponding number of multiple working electrodes. The greater the number of electrodes in a sensor, the larger the connector needs to be, further exacerbating the problem for multiple analyte sensors. A reduced size and lower associated cost of manufacture is advantageous because the sensor control device 102 is single use with a limited lifespan, thereby requiring frequent replacement.

Because the on-body unit can be mounted to the body of the patient, increasing the flexibility of the substrate, and in turn the ex vivo portion, can increase the on-body unit's resistance to forces resulting from the patient's movements. To achieve the desired flexibility, the substrate can be made from polyamide or polyethylene terephthalate (PET). In some embodiments, a PET substrate can have the material properties shown below.

**Table 1: Material Properties of PET**

| | |
|---|---|
| Young's modulus, E | 2800-3100 MPa |
| Tensile strength, σt | 55-75 MPa |
| Elastic limit | 50-150% |
| Notch test | 3.6 kJ/m2 |
| Glass transition temperature, Tg | 67-81 °C |
| Vicat B | 82 °C |
| Linear expansion coefficient, α | 7×10⁻⁵ K⁻¹ |
| Water absorption (ASTM) | 0.16 |
| Chemical formula | (C10H8O4)n |
| Molar mass | 10-50 kg/mol, varies |
| Density | 1.38 g/cm3, 20 °C |
| | 1.370 g/cm³, amorphous |
| | 1.455 g/cm³, single crystal |
| Melting point | > 250 °C (482 °F; 523 K) 260 °C |
| Boiling point | > 350 °C (662 °F; 623 K) (decomposes) |
| Solubility in water | Practically insoluble |
| log P | 0.9454 |
| Thermal conductivity | 0.15 to 0.24 W/(m·K) |
| Refractive index (nD) | 1.57-1.58, 1.5750 |
| Heat capacity (C) | 1.0 kJ/(kg·K) |
| Related Monomers | Terephthalic acid |
| | Ethylene glycol |

Additionally, the substrate can be made of one or more layers. The one or more layers can comprise a variety of materials, including Polyamide or PET, copper, fiberglass, and/or a gradient mix of materials, including any of the above-referenced materials. For example, the gradient mix of materials can include approximately 10% fiberglass. As another example, the gradient mix of materials can include fiberglass and/or another material, including materials having a melting temperature which is 5° F, 10 ° F, 15 ° F, 20 ° F, or any other melting temperature, higher than the melting temperature of fiberglass and/or PET. The in vivo portion 4002 can also include one or more layer, each of which also optionally having a gradient mix of materials. In one non-limiting example, a layer of the in vivo portion can include PET and a layer of the ex vivo portion can be approximately 10% fiberglass. Because of the substrate's flexibility, and because the electronic components are mounted directly onto the substrate without the need for a connector, the substrate can be folded in half such that the size of the on-body unit can be further decreased. To additionally reduce height, the one or more batteries can be disposed such that the solder contacts are soldered to the substrate, but the battery itself is offset from the substrate. As can be seen in FIGS. 9B and 40, the ex vivo portion 4004 can be any suitable shape including but not limited to circular (e.g., as shown in FIG. 40), clover-shaped (e.g., as shown in FIG. 9B), semi-circular, square, triangular, or diamond-shaped. Additionally, the in vivo portion 2408A, 4002A can extend from an edge of the ex vivo portion 2408A (e.g., as shown in FIG. 9B), can be centrally located with respect to the ex vivo portion 4004 (e.g., as shown in FIG. 40), or positioned in any other suitable location. In the latter case, the in vivo portion 4002 can extend through an aperture in the ex vivo portion 4004. As will also be appreciated in the art, the in vivo portion 4002 can also be located offset from the center of the ex vivo portion 4004. The electronic components 2418 can be positioned at any suitable location on the ex vivo portion 4004, and on either or both surface of the ex vivo portion 4004. For example, in one embodiment, a battery can be positioned on a first surface of in vivo portion 4004 and an antenna can be positioned on a second surface of ex vivo portion 4004. As shown in FIG. 40, the on-body unit includes shell 4006 and mount 4008, the mount includes an aperture for the in vivo portion 4002.

According to embodiments, as described above, the sensor of the disclosed subject matter can include an in vivo portion having a substrate, at least one working electrode, and a reference electrode configured such that the electrodes are printed on the substrate. Exemplary embodiment and methods of printed analyte sensors having one or more electrodes are disclosed in U.S. Patent Application No. 17/661,531. According to embodiments disclosed herein, the at least one working electrode can include one or more working electrodes. For example, the at least one or more working electrodes can include two, three, four or more working electrodes. Each working electrode can be configured to measure an analyte of interest (such as, without limitation, glucose, ketone, lactate, etc.) can be printed on the first surface of the in vivo portion 4002 and the reference electrode can be printed on the second surface of the in vivo portion 4002. More specifically, in some embodiments, the working electrodes can all be printed on a first side of in vivo portion 4002, and the reference electrode can be printed on a second side of the in vivo portion 4002; alternatively, in some embodiments, one working electrode can be printed on the first side of the in vivo portion 4002 and a second working electrode can be printed on the second side of the in vivo portion 4002. In yet another embodiment, a third working electrode can be printed on the first or the second side of the in vivo portion 4002, along with the first or second electrode, respectively. According to embodiments, in vivo portion 4002 can include four, five, or more electrodes. According to embodiments, analyte sensor (including in vivo 4002 portion and ex vivo portion 4004) can include a wire sensor (for example, not limitation, a platinum sensor, an analyte sensor with a platinum core as a working electrode, etc.).

As can be seen in FIG. 40, the ex vivo portion 4004 of sensor 104a includes a plurality of electronic components (2418A, 11914A). The plurality of electronic components (2418A, 11914A) can be communicatively coupled to the at least one working electrode and reference electrode, and can be configured to receive the signals associated with the measured analyte level generated by the electrodes. In some embodiments, all electronic components (e.g., electronic components 2418A, electronic components 11914A) can be mounted on the ex vivo portion 4004 of the sensor. As a result, a separate printed circuit board is not needed to mount electrical components 2418A, 11914A. The ex vivo portion 4004 can include electronic components 2418A mounted thereon for receiving the analyte measurement signals generated by the sensor in the in vivo portion 4002. These electronic components 2418A can include one or more processors, one or more batteries, one or more antennas, resistors, transistors, capacitors, inductors, diodes, and/or switches. The electronic components are mounted on the substrate of the ex vivo portion. For example, as can be seen in FIG. 40, the electronic components 2418A can include at least a battery and an antenna. The substrate of the ex vivo portion can similarly include any or all of the disclosed electronic components. Additionally or alternatively, one or more antennas are not required to be directly mounted to the substrate and can be mounted on an additional, second substrate. Additionally or alternatively, an antenna can be a raised antenna, as described in US20220079475A1. The one or more antennas can be powered by the one or more batteries. Batteries can be mounted to the first substrate or by an alternative or additional battery which can be mounted to the second substrate alongside the one or more antennas. Alternatively, or additionally, one or more batteries can be mounted or located in the sensor control device 102 and provide power to one or more antennas and/or other electronic components mounted on the ex vivo portion 4004. The battery or batteries can include, for example without limitation, a coin battery, a fed battery, a printed battery, or any other type of battery. According to embodiments, the processor or processors can be one or more ASICs. As embodied herein the electronic components can be configured to transmit the sensor data using the one or more antennas over Wi-Fi, NFC, Bluetooth, BTLE, or GPS, which can be received by a remote device having a display screen such as a hand-held analyte monitoring device, a mobile phone, a wrist-mounted device, or any other computing device. The remote device can be further configured to display the received sensor data on the display screen. Furthermore, as described herein, the analyte sensor can be sterilized to prevent contamination; as embodied herein, this sterilization can occur either before or after the electronic components are mounted to the ex vivo portion 4004.

In accordance with disclosed subject matter, the electronic components can be mounted onto the substrate using photonic soldering. Photonic soldering can allow the electronic components to be mounted without damaging the substrate during the mounting process, which can occur due to heat exposure in the reflow oven, as described further herein. More specifically, because of the different material properties of the solder and substrate, the light can cause the solder to heat and reflow without causing the substrate to become heated. By contrast, a traditional reflow oven subjects the substrate higher temperature levels, which can cause the substrate to melt or experience other damage. The use of photonic soldering can also enable high volume automated assembly. Furthermore, photonic soldering can be used to mount a wire sensor on the substrate. For example, not limitation, a wire sensor can include a platinum sensor, a sensor have a platinum working electrode as a core, etc.

Photonic soldering uses flashes of light to reflow the solder paste in a molten state, thereby creating a permanent connection between the substrate and the electronic components. In particular, this can occur when the substrate is a lighter color than the electronic components and/or solder paste, thus causing the darker components to absorb more light and therefore reach the requisite heat for soldering without affecting the lighter colored components. For example, without limitation, the substrate can be clear or white to allow light to pass through or reflect, thereby improving the performance of the photonic soldering process. The amount of light and energy delivered to the substrate and electrical components can be well controlled by controlling the power of the lamp used, the wavelengths of the light generated, the duration and frequency of the pulses generated, and the area being exposed to light. The process of rapid heating and cooling via flashes of light allows reflow of the solder without damage to the polymer substrate. Although photonic soldering can be done with a laser focused on the solder tabs of individual components, a flash lamp provides a larger exposure area allowing multiple components to be soldered at the same time and facilitates high volume manufacturing. By contrast, during traditional reflow soldering, solder paste is used to temporarily attach electronic components to a substrate, and is subsequently molten in a reflow oven, thereby creating permanent connections between the electronic components and the substrate. Certain flexible substrate materials, such as polyamide or PET, can be damaged during reflow soldering by the heat of the reflow oven.

As embodied herein, the photonic soldering process can use pulses of light (for example, without limitation, xenon light), to reflow the solder paste. The light can be delivered to the targeted components using multiple, repeated light pulses to controllably increase the solder paste temperature. As embodied herein, a user can vary the photonic soldering process by controlling the input power, the pulse duration, the number of pulses, and/or the flash rate of the pulses. As embodied herein, the electronic components can be hand-placed on the substrate or can be machine-placed on the substrate. Further, the components can be soldered either individually or simultaneously. The components can also be mounted on the first surface of the sensor; as embodied herein, this can occur after printing the electrodes.

Further information regarding photonic soldering, and methods of uses thereof, are described in *Photonic Flash Soldering on Flex Foils for Flexible Electronic Systems,* by Arutinov et. al., (G. Arutinov, R. Hendriks and J. Van Den Brand, "Photonic Flash Soldering on Flex Foils for Flexible Electronic Systems," 2016 IEEE 66th Electronic Components and Technology Conference (ECTC), 2016, pp. 95-100, doi: 10.1109/ECTC.2016.179.).

In some embodiments, multiple substrates can be subject to the photonic soldering at the same time. For example, a sheet of substrate material can have an array of 2X2, 3X3, 4X4, 5X5, or any other arrangement of substrate blanks. Next, to print carbon traces prior to photonic soldering, the substrate can be etched to outline the contacts and the contacts can then be masked to prevent light absorption as described herein In some embodiments, as described above, the electrodes can be printed on the substrate using a carbon ink and include contacts (e.g., contacts 2418 as shown in FIG. 9A) on the ex vivo portion to electrically couple the active portion of the sensor to the electronic components disposed on the ex vivo portion of the sensor. The traces can alternatively be copper. According to embodiments disclosed herein, to further protect the substrate during the photonic soldering process, the substrate can be masked to prevent light pulses from contacting the substrate, except at the components or leads to be soldered. The mask can include a titanium dioxide material, which can reflect and minimize light absorption. As an example, and not limitation, a suitable mask can have the following material properties:

**Table 2: Material Properties of an Exemplary Solder Mask**

| **TEST** | **METHOD** | **RESULT** | | **CLASSIFICATION** |
|---|---|---|---|---|
| **Hardness (pencil)** | SM-840C | 6H | | Pass, class H |
| | | Copper: 0% removal | | |
| **Adhesion** | SM-840C | Base laminate: 0% removal | | Pass, class H |
| | | SnPb: <10% removal | | |
| **Chemical resistance** | SM-840C | | | |
| Isopropanol (min. 120s) | Room temp. 120s | No surface roughness | | |
| | 46 (± 2)°C 15 min | No blisters | | |
| Isopropanol/H₂0 (75/25) D-Limonene | Room temp. 120s | No delamination | | |
| | 57 (± 2)°C 120s | No swelling | | Pass, class H |
| 10% Alkaline detergent | 57 (± 2)°C 120s | No colour change | | |
| | Room temp. 60s | No cracking | | |
| Monoethanolamine | 60 (± 2)°C 5 min | | | |
| Methylene chloride | | | | |
| Deionised water | | | | |
| **Hydrolytic stability** | SM-840C | No evidence of reversion | | Pass, class H |
| **Insulation resistance** | SM-840C | Before solder | 10¹¹-10¹² Ω | Pass, class H |
| | | After solder | 10¹¹-10¹² Ω | |
| **Moisture & insulation** | SM-840C | No blistering, separation, degradation. | | Pass, class H |
| | | Initial | 10¹¹ - 10¹² Ω | |
| | | During | 10⁹ - 10¹⁰ Ω | |
| | | After | 10¹¹ - 10¹² Ω | |
| **Wave-solder resistance** | SM-840C | No loss of adhesion or solder pick- up. | | Pass, class H |
| **10 (± 1)s at 260 (±** 5)°C | | | | |
| **Hot-air-solder-level** | N/A | Minimum 5 cycles | | Pass |
| **Thermal shock** | SM840 C | No cracks, delamination, crazing or blistering | | Pass, class H |
| **Dielectric strength** | SM840 C | | | Pass, class H |
| **Dielectric constant** | | 4 (1 MHz) | | |

Source: Electra Technical Datasheet for Carapace EMP110W-LED Photoimageable Soldermask for LED, EMP110 W-LED (cool-white/extra-cool-white/warm-white)_rev5, which can be accessed https://electrapolymers.com/wp-content/uploads/sds_files/EMP110%20W-LED.pdf. In some embodiments, the mask can be applied using a silk screen. Additionally or alternatively, the substrate can be coated with a reflective coating prior to the photonic soldering process and/or the light source can used with a UV light filter to prevent light from penetrating the substrate material. The printed traces may also be at risk of heat damage from the light exposure caused by photonic soldering. Molten reflow from heated solder may also pose a risk of shorting the carbon traces. Therefore, a metal-based removable mask can be applied over the circuit board traces to prevent light absorption in the traces and block solder reflow from shorting the traces. After masking the traces, solder material can be dispensed on the substrate, and the electronic components can be disposed on the solder either manually or in an automated fashion. In some embodiments, the one or more batteries can be manually soldered to the substrate because the battery solder tabs can be non-coplanar, thus causing them to heat at different rates. Then, the substrate and electronic components can undergo the photonic soldering process described above. After the soldering process has been completed, the substrates can be laser cut out of the sheet to constitute a final product, and the in vivo portion 4002 of the sensor can be dipped in a membrane material to form the membrane.

According to embodiments disclosed herein, in order to prevent movement of the substrate during the photonic soldering process due to potential warping, the substrate can be secured to the work bench or other work surface using a vice, clips or any clamping means. The substrate can also be secured to the work surface using a vacuum. The photonic soldering process can be conducted with any type of known, commercially available solder. In some embodiments, however, solders with relatively low melting points can be used in the photonic soldering process. For example, PET panels have a melting point of approximately 260° C, and standard solder having a tin-copper-silver alloy has a melting point of approximately 220° C. Although the PET panels have a higher melting point than standard solder, heating the standard solder to its melting point risks causing damage to the PET panels. By contrast, low temperature solders - for example, a solder having a bismuth-tin-silver alloy which has a melting point of approximately 140° C - require lower temperatures to be melted, thus decreasing the likelihood of damaging the PET panels. For example and not limitation, additional solder types having the below listed alloys and properties may also be used with substrates having suitably high melting ranges.

**Table 3: Exemplary Solder Alloys and Properties**

| **Alloy** | **Melting Range °C** | **Melting Range °F** |
|---|---|---|
| **HIGH-TEMP** | | |
| Pb100 | 327 | 621 |
| Sn1Pb97.5Ag1.5 | 309 | 588 |
| Sn5Pb95 | 301-314 | 574-597 |
| Sn5Pb93.5Ag1.5 | 296-301 | 565-574 |
| Sn5Pb92.5Ag2.5 | 280 | 536 |
| Sn10Pb88Ag2 | 268-299 | 514-570 |

| **MID-RANGE** | | |
|---|---|---|
| Sn35Pb65 | 183-247 | 361-477 |
| Sn40Pb60 | 183-238 | 361-460 |
| Sn50Pb50 | 183-216 | 361-420 |
| Sn60Pb40 | 183-190 | 361-374 |
| Sn63Pb37 | 183 | 361 |
| Sn62Pb36Ag2 | 179 | 354 |

| **LOW-TEMP** | | |
|---|---|---|
| Sn43Pb43Bi14 | 144-163 | 291-324 |
| Sn42Bi57Ag1 | 138 | 281 |

| **LEAD-FREE** | | |
|---|---|---|
| Sn97Ag3 | 221-224 | 430-435 |
| Sn95Sb5 | 232-240 | 450-464 |
| Sn100 | 232 | 450 |
| K100LD | 227 | 441 |
| Sn99.3Cu0.7 | 227 | 441 |
| Sn95Ag5 | 221-245 | 430-473 |
| Sn96.3Ag3.7 | 221-229 | 430-444 |
| Sn96.5Ag3.5 | 221 | 430 |
| Sn97Ag0.2Sb0.8Cu2 | 220-234 | 428-454 |
| Sn99Ag0.3Cu0.7 | 217-228 | 423-442 |
| Sn96.5Ag3Cu0.5 | 217-220 | 422-428 |
| Sn95.5Ag4Cu0.5 | 217 | 423 |
| Sn95.5Ag3.8Cu0.7 | 217 | 423 |

Further details on solder alloys can be found in *Kestser Alloy Temperature Chart.*

### Example Embodiments of Firing Mechanism of One-Piece and Two-Piece Applicators

FIGS. 35A-35F illustrate example details of embodiments of the internal device mechanics of "firing" the applicator 216 to apply sensor control device 222 to a user and including retracting sharp 1030 safely back into used applicator 216. All together, these drawings represent an example sequence of driving sharp 1030 (supporting a sensor coupled to sensor control device 222) into the skin of a user, withdrawing the sharp while leaving the sensor behind in operative contact with interstitial fluid of the user, and adhering the sensor control device to the skin of the user with an adhesive. Modification of such activity for use with the alternative applicator assembly embodiments and components can be appreciated in reference to the same by those with skill in the art. Moreover, applicator 216 may be a sensor applicator having one-piece architecture or a two-piece architecture as disclosed herein.

Turning now to FIG. 35A, a sensor 1102 is supported within sharp 1030, just above the skin 1104 of the user. Rails 1106 (optionally three of them) of an upper guide section 1108 may be provided to control applicator 216 motion relative to sheath 318. The sheath 318 is held by detent features 1110 within the applicator 216 such that appropriate downward force along the longitudinal axis of the applicator 216 will cause the resistance provided by the detent features 1110 to be overcome so that sharp 1030 and sensor control device 222 can translate along the longitudinal axis into (and onto) skin 1104 of the user. In addition, catch arms 1112 of sensor carrier 1022 engage the sharp retraction assembly 1024 to maintain the sharp 1030 in a position relative to the sensor control device 222.

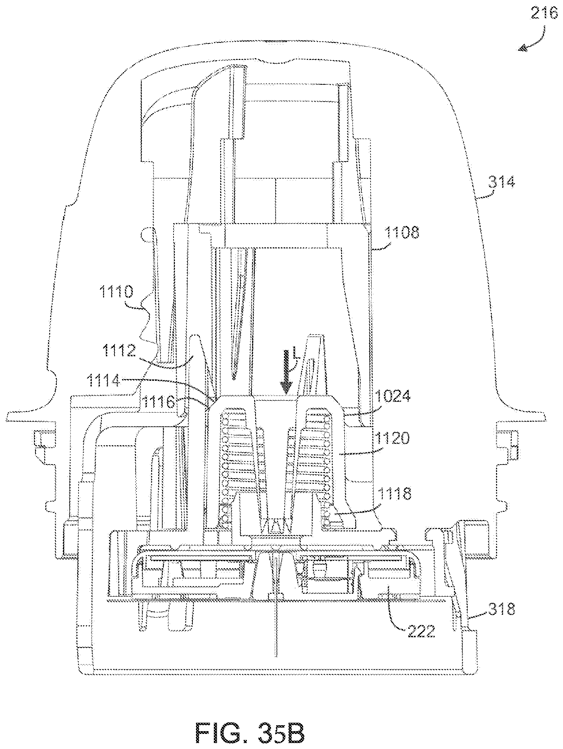

In FIG. 35B, user force is applied to overcome or override detent features 1110 and sheath 318 collapses into housing 314 driving the sensor control device 222 (with associated parts) to translate down as indicated by the arrow L along the longitudinal axis. An inner diameter of the upper guide section 1108 of the sheath 318 constrains the position of carrier arms 1112 through the full stroke of the sensor/sharp insertion process. The retention of the stop surfaces 1114 of carrier arms 1112 against the complimentary faces 1116 of the sharp retraction assembly 1024 maintains the position of the members with return spring 1118 fully energized.

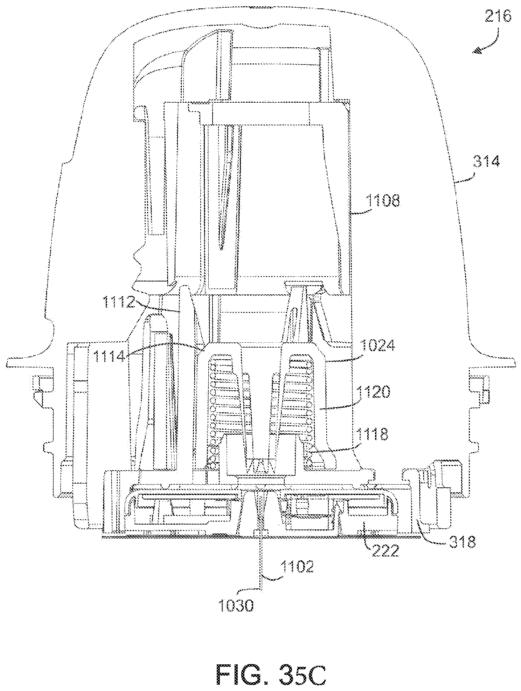

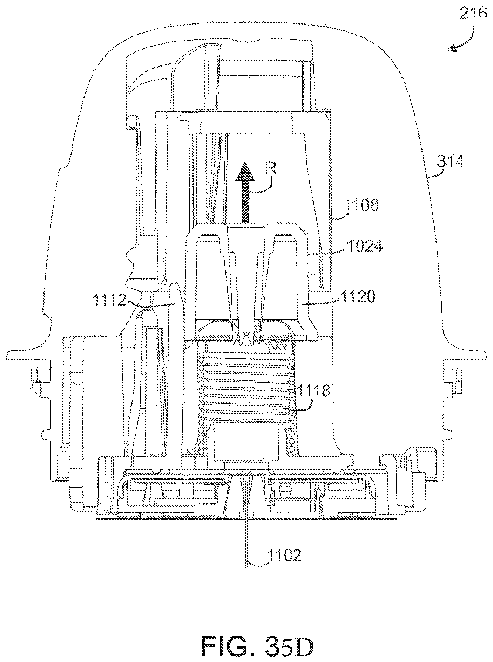

In FIG. 35C, sensor 1102 and sharp 1030 have reached full insertion depth. In so doing, the carrier arms 1112 clear the upper guide section 1108 inner diameter. Then, the compressed force of the coil return spring 1118 drives angled stop surfaces 1114 radially outward, releasing force to drive the sharp carrier 1102 of the sharp retraction assembly 1024 to pull the (slotted or otherwise configured) sharp 1030 out of the user and off of the sensor 1102 as indicated by the arrow R in FIG. 35D.

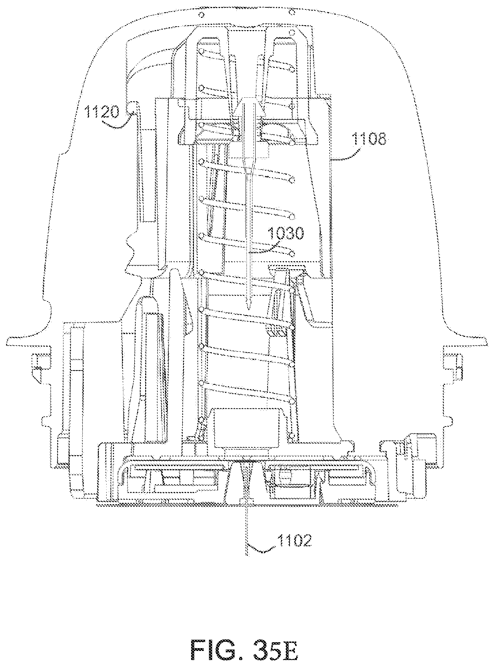

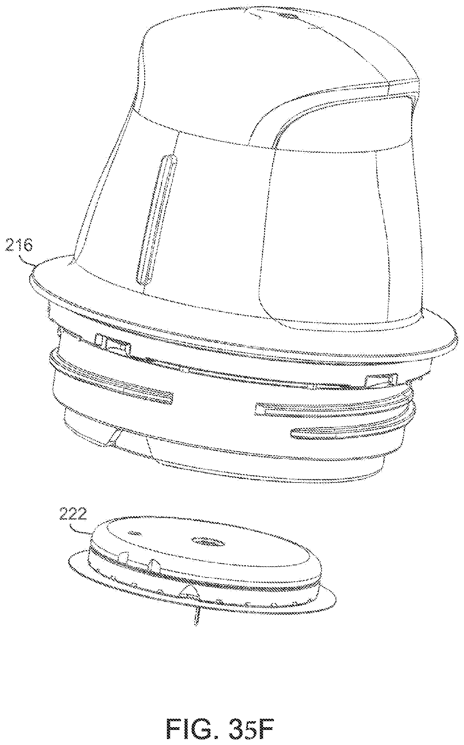

With the sharp 1030 fully retracted as shown in FIG. 35E, the upper guide section 1108 of the sheath 318 is set with a final locking feature 1120. As shown in FIG. 35F, the spent applicator assembly 216 is removed from the insertion site, leaving behind the sensor control device 222, and with the sharp 1030 secured safely inside the applicator assembly 216. The spent applicator assembly 216 is now ready for disposal.

Operation of the applicator 216 when applying the sensor control device 222 is designed to provide the user with a sensation that both the insertion and retraction of the sharp 1030 is performed automatically by the internal mechanisms of the applicator 216. In other words, the present invention avoids the user experiencing the sensation that he is manually driving the sharp 1030 into his skin. Thus, once the user applies sufficient force to overcome the resistance from the detent features of the applicator 216, the resulting actions of the applicator 216 are perceived to be an automated response to the applicator being "triggered." The user does not perceive that he is supplying additional force to drive the sharp 1030 to pierce his skin despite that all the driving force is provided by the user and no additional biasing/driving means are used to insert the sharp 1030. As shown above above in FIG. 35C, the retraction of the sharp 1030 is automated by the coil return spring 1118 of the applicator 216.

With respect to any of the applicator embodiments described herein, as well as any of the components thereof, including but not limited to the sharp, sharp module and sensor module embodiments, those of skill in the art will understand that said embodiments can be dimensioned and configured for use with sensors configured to sense an analyte level in a bodily fluid in the epidermis, dermis, or subcutaneous tissue of a subject. In some embodiments, for example, sharps and distal portions of analyte sensors disclosed herein can both be dimensioned and configured to be positioned at a particular end-depth (i.e., the furthest point of penetration in a tissue or layer of the subject's body, e.g., in the epidermis, dermis, or subcutaneous tissue). With respect to some applicator embodiments, those of skill in the art will appreciate that certain embodiments of sharps can be dimensioned and configured to be positioned at a different end-depth in the subject's body relative to the final end-depth of the analyte sensor. In some embodiments, for example, a sharp can be positioned at a first end-depth in the subject's epidermis prior to retraction, while a distal portion of an analyte sensor can be positioned at a second end-depth in the subject's dermis. In other embodiments, a sharp can be positioned at a first end-depth in the subject's dermis prior to retraction, while a distal portion of an analyte sensor can be positioned at a second end-depth in the subject's subcutaneous tissue. In still other embodiments, a sharp can be positioned at a first end-depth prior to retraction and the analyte sensor can be positioned at a second end-depth, wherein the first end-depth and second end-depths are both in the same layer or tissue of the subject's body.

Additionally, with respect to any of the applicator embodiments described herein, those of skill in the art will understand that an analyte sensor, as well as one or more structural components coupled thereto, including but not limited to one or more spring-mechanisms, can be disposed within the applicator in an off-center position relative to one or more axes of the applicator. In some applicator embodiments, for example, an analyte sensor and a spring mechanism can be disposed in a first off-center position relative to an axis of the applicator on a first side of the applicator, and the sensor electronics can be disposed in a second off-center position relative to the axis of the applicator on a second side of the applicator. In other applicator embodiments, the analyte sensor, spring mechanism, and sensor electronics can be disposed in an off-center position relative to an axis of the applicator on the same side. Those of skill in the art will appreciate that other permutations and configurations in which any or all of the analyte sensor, spring mechanism, sensor electronics, and other components of the applicator are disposed in a centered or off-centered position relative to one or more axes of the applicator are possible and fully within the scope of the present disclosure.

A number of deflectable structures are described herein, including but not limited to deflectable detent snaps 1402, deflectable locking arms 1412, sharp carrier lock arms 1524, sharp retention arms 1618, and module snaps 2202. These deflectable structures are composed of a resilient material such as plastic or metal (or others) and operate in a manner well known to those of ordinary skill in the art. The deflectable structures each has a resting state or position that the resilient material is biased towards. If a force is applied that causes the structure to deflect or move from this resting state or position, then the bias of the resilient material will cause the structure to return to the resting state or position once the force is removed (or lessened). In many instances these structures are configured as arms with detents, or snaps, but other structures or configurations can be used that retain the same characteristics of deflectability and ability to return to a resting position, including but not limited to a leg, a clip, a catch, an abutment on a deflectable member, and the like.

In summary, a system is described for measurement of an analyte level including an analyte sensor having an in vivo portion in contact with the interstitial fluid of a user and an ex vivo portion. The sensor further includes at least one working electrode and a reference electrode located on the in vivo portion, and a first substrate. The at least one working electrode and reference electrode generate signals associated with a measured analyte level. Further, the ex vivo portion includes a plurality of electronic components mounted thereon, and at least one of the electronic components are configured to receive the generated signals associated with the measured analyte level. The electronic components are mounted to the ex vivo portion using photonic soldering.

Additional details of suitable devices, systems, methods, components and the operation thereof along with related features are set forth in International Publication No. WO2018/136898 to Rao et. al., International Publication No. WO2019/236850 to Thomas et. al., International Publication No. WO2019/236859 to Thomas et. al., International Publication No. WO2019/236876 to Thomas et. al., and U.S. Patent Publication No. 2020/0196919. Further details regarding embodiments of applicators, their components, and variants thereof, are described in U.S. Patent Publication Nos. 2013/0150691, 2016/0331283, and 2018/0235520. Further details regarding embodiments of sharp modules, sharps, their components, and variants thereof, are described in U.S. Patent Publication No. 2014/0171771.

It should be noted that all features, elements, components, functions, and steps described with respect to any embodiment provided herein are intended to be freely combinable and substitutable with those from any other embodiment. If a certain feature, element, component, function, or step is described with respect to only one embodiment, then it should be understood that that feature, element, component, function, or step can be used with every other embodiment described herein unless explicitly stated otherwise. This paragraph therefore serves as antecedent basis and written support for the introduction of claims, at any time, that combine features, elements, components, functions, and steps from different embodiments, or that substitute features, elements, components, functions, and steps from one embodiment with those of another, even if the following description does not explicitly state, in a particular instance, that such combinations or substitutions are possible. Thus, the foregoing description of specific embodiments of the disclosed subject matter has been presented for purposes of illustration and description. It is explicitly acknowledged that express recitation of every possible combination and substitution is overly burdensome, especially given that the permissibility of each and every such combination and substitution will be readily recognized by those of ordinary skill in the art.

While the embodiments are susceptible to various modifications and alternative forms, specific examples thereof have been shown in the drawings and are herein described in detail. It will be apparent to those skilled in the art that various modifications and variations can be made in the method and system of the disclosed subject matter without departing from the spirit or scope of the disclosed subject matter. Thus, it is intended that the disclosed subject matter include modifications and variations that are within the scope of the appended claims and their equivalents. Furthermore, any features, functions, steps, or elements of the embodiments may be recited in or added to the claims, as well as negative limitations that define the inventive scope of the claims by features, functions, steps, or elements that are not within that scope.

## Claims

1. A system (100) for measurement of an analyte level, comprising:
an on-body unit including a shell (4006) and a mount (4008), the mount (4008) including an aperture; and
an analyte sensor (104A, 11900A) having an in vivo portion (4002) configured to be positioned in contact with an interstitial fluid of a user and an ex vivo portion (4004) disposed within the on-body unit, wherein the in vivo portion (4002) extends through the aperture, the analyte sensor having
a first substrate;
at least one working electrode located on the in vivo portion;
a reference electrode located on the in vivo portion; and
a plurality of electronic components (2418A) mounted on the ex vivo portion;
wherein the at least one working electrode is configured to sense an analyte level in the interstitial fluid of the user, and at least one of the plurality of electronic components being configured to receive the generated signals associated with the analyte level, wherein the electronic components are mounted directly on the ex vivo portion (4004) using photonic soldering.

2. The system (100) of claim 1, wherein the at least one working electrode is configured to sense at least one of lactate, glucose, and ketone.

3. The system (100) of any of claims 1 or 2 , further comprising:
an applicator (102, 216) for delivery of the analyte sensor including:
a housing (208, 702, 1402, 9504) including a sensor carrier (1022, 5602) configured to secure the on-body unit within an interior of the applicator;
and
an applicator cap (210, 708, 1404, 9506) removably coupled to the housing to seal the interior of the applicator.

4. The system (100) of any of claims 1 to 3, wherein the plurality of electronic components (2418A) are electrically coupled to the at least one working electrode and the reference electrode.

5. A method of assembling a system (100) for measurement of an analyte level, comprising:
providing an on-body unit including a shell (4006) and a mount (4008), the mount (4008) including an aperture;
providing an analyte sensor having an in vivo portion (4002) configured to be positioned in contact with an interstitial fluid of a user and an ex vivo portion (4004) and disposing the ex vivo portion within the on-body unit and extending the in vivo portion (4004) through the aperture, the analyte sensor having:
a first substrate,
at least one working electrode located on the in vivo portion, and
a reference electrode located on the in vivo portion,
wherein the at least one working electrode is configured to sense an analyte level in the interstitial fluid of the user; and
mounting a plurality of electronic components (2418A) to the ex vivo portion using photonic soldering, at least one of the plurality of electronic components being configured to receive the generated signals associated with the analyte level.

6. The method of claim 5, wherein providing the analyte sensor includes printing the at least one working electrode and the reference electrode on the substrate.

7. The method of claim 6, further comprising:
masking a portion of the first substrate prior to photonic soldering, and/or
coating the first substrate with a reflective coating prior to photonic soldering, and/or
providing a vacuum to prevent the first substrate from warping during the photonic soldering process.

8. The system (100) of any of claims 1 to 4 or method of claims 5 to 7, wherein the plurality of electronic components (2418A) are further configured to transmit the signals associated with the analyte level to a remote device (120) having a display screen (122), optionally wherein the remote device is at least one of a hand-held analyte monitoring device, a mobile phone, or a wrist-mounted device.

9. The system (100) of any of claims 1 to 4 and 8 or method of any of claims 5 to 8, wherein the first substrate is a flexible monolithic unit.

10. The system (100) of any of claims 1 to 4, 8 and 9 or method of any of claims 5 to 9, wherein the plurality of electronic components (2418A) comprise one or more processors and a battery, optionally, wherein the battery includes a printed battery.

11. The system (100) or method of claim 10, wherein the plurality of electronic components further comprise at least one antenna.

12. The system (100) of any of claims 1 to 4 and 8 to 11 or method of claims 10 or 11, wherein the analyte sensor further comprises a second substrate having at least one antenna, optionally wherein the at least one antenna includes a Wi-Fi antenna, NFC antenna, Bluetooth antenna, BTLE antenna, or GPS antenna.

13. The system (100) of any of claims 1 to 4 and 8 to 12 or method of any of claims 5 to 12, wherein the first substrate is one of polyamide or polyethylene terephthalate.

14. The system (100) of any of claims 1 to 4 and 8 to 13 or method of claims 5 to 13, wherein the ex vivo portion (4004) comprises a first layer.

15. The system (100) or method of claim 14, wherein the first layer comprises a gradient mix of materials, optionally,
wherein the gradient mix of materials comprises fiberglass and/or
wherein the gradient mix of materials comprises approximately 10% fiberglass and the in vivo portion comprises PET.

16. The system (100) or method of claim 14, wherein the ex vivo portion (4004) comprises a second layer, optionally wherein each of the first layer and the second layer comprise a gradient mix of materials.

17. The system (100) of any of claims 1 to 4 and 8 to 16, wherein the analyte sensor further comprises a membrane configured to regulate analyte influx disposed on the in vivo portion.

## Patentansprüche

1. System (100) zum Messen eines Analytspiegels, Folgendes umfassend:
eine Am-Körper-Einheit, die eine Hülle (4006) und eine Halterung (4008) beinhaltet, wobei die Halterung (4008) eine Öffnung beinhaltet; und
einen Analytsensor (104A, 11900A) mit einem In-vivo-Abschnitt (4002), der dazu konfiguriert ist, in Kontakt mit einem interstitiellen Fluid eines Benutzers positioniert zu sein, und einem Ex-vivo-Abschnitt (4004), der in der Am-Körper-Einheit angeordnet ist, wobei sich der In-vivo-Abschnitt (4002) durch die Öffnung erstreckt, wobei der Analytsensor Folgendes aufweist:
ein erstes Substrat;
mindestens eine Arbeitselektrode, die sich an dem In-vivo-Abschnitt befindet;
eine Referenzelektrode, die sich an dem In-vivo-Abschnitt befindet; und
mehrere elektronische Komponenten (2418A), die an dem Ex-vivo-Abschnitt befestigt sind;
wobei die mindestens eine Arbeitselektrode dazu konfiguriert ist, einen Analytspiegel in dem interstitiellen Fluid des Benutzers abzutasten, und mindestens eine der mehrere elektronischen Komponenten dazu konfiguriert ist, die erzeugten Signale zu empfangen, die dem Analytspiegel zugeordnet sind, wobei die elektronischen Komponenten mittels Laserlöten direkt an dem Ex-vivo-Abschnitt (4004) befestigt sind.

2. System (100) nach Anspruch 1, wobei die mindestens eine Arbeitselektrode dazu konfiguriert ist, mindestens eines von Lactat, Glucose und Keton abzutasten.

3. System (100) nach einem der Ansprüche 1 bis 2, ferner Folgendes umfassend:
einen Applikator (102, 216) zur Abgabe des Analytsensors, Folgendes beinhaltend:
ein Gehäuse (208, 702, 1402, 9504), das einen Sensorträger (1022, 5602) beinhaltet, der dazu konfiguriert ist, die Am-Körper-Einheit in einem Innenraum des Applikators zu sichern; und
eine Applikatorkappe (210, 708, 1404, 9506), die abnehmbar mit dem Gehäuse gekoppelt ist, um das Innere des Applikators abzudichten.

4. System (100) nach einem der Ansprüche 1 bis 3, wobei die mehreren elektronischen Komponenten (2418A) elektrisch mit der mindestens einen Arbeitselektrode und der Referenzelektrode gekoppelt sind.

5. Verfahren zum Zusammensetzen eines Systems (100) zum Messen eines Analytspiegels, Folgendes umfassend:
Bereitstellen einer Am-Körper-Einheit, die einer Hülle (4006) und eine Halterung (4008) beinhaltet, wobei die Halterung (4008) eine Öffnung beinhaltet;
Bereitstellen eines Analytsensors mit einem In-vivo-Abschnitt (4002), der dazu konfiguriert ist, in Kontakt mit einem interstitiellen Fluid eines Benutzers positioniert zu sein, und einem Ex-vivo-Abschnitt (4004) und Anordnen des Ex-vivo-Abschnitts in der Am-Körper-Einheit und Führen des In-vivo-Abschnitts (4004) durch die Öffnung, wobei der Analytsensor Folgendes aufweist:
ein erstes Substrat,
mindestens eine Arbeitselektrode, die sich an dem In-vivo-Abschnitt befindet, und
eine Referenzelektrode, die sich an dem In-vivo-Abschnitt befindet,
wobei die mindestens eine Arbeitselektrode dazu konfiguriert ist, einen Analytspiegel in dem interstitiellen Fluid des Benutzers abzutasten; und
Befestigen mehrerer elektronischer Komponenten (2418A) an dem Ex-vivo-Abschnitt mittels Laserlöten, wobei mindestens eine der mehreren elektronischen Komponenten dazu konfiguriert ist, die erzeugten Signale zu empfangen, die dem Analytspiegel zugeordnet sind.

6. Verfahren nach Anspruch 5, wobei das Bereitstellen des Analytsensors das Drucken der mindestens einen Arbeitselektrode und der Referenzelektrode auf das Substrat umfasst.

7. Verfahren nach Anspruch 6, ferner Folgendes umfassend:
Maskieren eines Abschnitts des ersten Substrats vor dem Laserlöten, und/oder
Beschichten des ersten Substrats mit einer reflektierenden Beschichtung vor dem Laserlöten, und/oder
Bereitstellen eines Unterdrucks, um zu verhindern, dass sich das erste Substrat während des Laserlötens aufwirft.

8. System (100) nach einem der Ansprüche 1 bis 4 oder Verfahren nach den Ansprüchen 5 bis 7, wobei die mehreren elektronischen Komponenten (2418A) ferner dazu konfiguriert sind, die Signale, die dem Analytspiegel zugeordnet sind, an eine ferne Vorrichtung (120) zu übertragen, die einen Anzeigebildschirm (122) aufweist, wobei optional die ferne Vorrichtung mindestens eines von einer handgeführten Analytüberwachungsvorrichtung, einem Mobiltelefon oder einer am Handgelenk befestigten Vorrichtung ist.

9. System (100) nach einem der Ansprüche 1 bis 4 und 8 oder Verfahren nach einem der Ansprüche 5 bis 8, wobei das erste Substrat eine flexible monolithische Einheit ist.

10. System (100) nach einem der Ansprüche 1 bis 4, 8 und 9 oder Verfahren nach einem der Ansprüche 5 bis 9, wobei die mehreren elektronischen Komponenten (2418A) einen oder mehrere Prozessoren und eine Batterie umfassen, wobei die Batterie optional eine gedruckte Batterie beinhaltet.

11. System (100) oder Verfahren nach Anspruch 10, wobei die mehreren elektronischen Komponenten ferner mindestens eine Antenne umfassen.

12. System (100) nach einem der Ansprüche 1 bis 4 und 8 bis 11 oder Verfahren nach Anspruch 10 oder 11, wobei der Analytsensor ferner ein zweites Substrat mit mindestens einer Antenne umfasst, wobei optional die mindestens eine Antenne eine Wi-Fi-Antenne, eine NFC-Antenne, eine Bluetooth-Antenne, eine BTLE-Antenne oder eine GPS-Antenne beinhaltet.

13. System (100) nach einem der Ansprüche 1 bis 4 und 8 bis 12 oder Verfahren nach einem der Ansprüche 5 bis 12, wobei das erste Substrat eines von Polyamid oder Polyethylenterephthalat ist.

14. System (100) nach einem der Ansprüche 1 bis 4 und 8 bis 13 oder Verfahren nach einem der Ansprüche 5 bis 13, wobei der Ex-vivo-Abschnitt (4004) eine erste Schicht umfasst.

15. System (100) oder Verfahren nach Anspruch 14, wobei die erste Schicht eine Gradientenmaterialmischung umfasst, optional
wobei die Gradientenmaterialmischung Glasfaser umfasst und/oder
wobei die Gradientenmaterialmischung ungefähr 10 % Glasfaser umfasst und der In-vivo-Abschnitt PET umfasst.

16. System (100) oder Verfahren nach Anspruch 14, wobei der Ex-vivo-Abschnitt (4004) eine zweite Schicht umfasst, wobei optional die erste und die zweite Schicht jeweils eine Gradientenmaterialmischung umfassen.

17. System (100) nach einem der Ansprüche 1 bis 4 und 8 bis 16, wobei der Analytsensor ferner eine Membran umfasst, die dazu konfiguriert ist, einen Analytzufluss zu regeln, und an dem In-vivo-Abschnitt angeordnet ist.

## Revendications

1. Système (100) de mesure d'un niveau d'analyte, comprenant :
une unité sur le corps incluant une coque (4006) et une monture(4008), la monture (4008) incluant une ouverture ; et
un capteur d'analyte (104A, 11900A) ayant une partie in vivo (4002) configurée pour être positionnée en contact avec un fluide interstitiel d'un utilisateur et une partie ex vivo (4004) disposée au sein de l'unité sur le corps, dans lequel la partie in vivo (4002) s'étend à travers l'ouverture, le capteur d'analyte ayant
un premier substrat ;
au moins une électrode de travail située sur la partie in vivo ;
une électrode de référence située sur la partie in vivo ; et
une pluralité de composants électroniques (2418A) montés sur la partie ex vivo ;
dans lequel l'au moins une électrode de travail est configurée pour détecter un niveau d'analyte dans le fluide interstitiel de l'utilisateur, et au moins l'un de la pluralité de composants électroniques étant configuré pour recevoir les signaux générés associés au niveau d'analyte, dans lequel les composants électroniques sont montés directement sur la partie ex vivo (4004) à l'aide d'un soudage photonique.

2. Système (100) selon la revendication 1, dans lequel l'au moins une électrode de travail est configurée pour détecter au moins un élément parmi le lactate, le glucose, et la cétone.

3. Système (100) selon l'une quelconque des revendications 1 ou 2, comprenant en outre :
un applicateur (102, 216) destiné à la délivrance du capteur d'analyte incluant :
un logement (208, 702, 1402, 9504) incluant un support de capteur (1022, 5602) configuré pour fixer l'unité sur le corps au sein d'un intérieur de l'applicateur ; et
un capuchon d'applicateur (210, 708, 1404, 9506) couplé de manière amovible au logement pour assurer l'étanchéité de l'intérieur de l'applicateur.

4. Système (100) selon l'une quelconque des revendications 1 à 3, dans lequel la pluralité de composants électroniques (2418A) sont couplés électriquement à l'au moins une électrode de travail et à l'électrode de référence.

5. Procédé d'assemblage d'un système (100) de mesure d'un niveau d'analyte, comprenant :
la fourniture d'une unité sur le corps incluant une coque (4006) et une monture (4008), la monture (4008) incluant une ouverture ;
la fourniture d'un capteur d'analyte ayant une partie in vivo (4002) configurée pour être positionnée en contact avec un fluide interstitiel d'un utilisateur et une partie ex vivo (4004) et la disposition de la partie ex vivo au sein de l'unité sur le corps et l'extension de la partie in vivo (4004) à travers l'ouverture, le capteur d'analyte ayant :
un premier substrat,
au moins une électrode de travail située sur la partie in vivo, et
une électrode de référence située sur la partie in vivo,
dans lequel l'au moins une électrode de travail est configurée pour détecter un niveau d'analyte dans le fluide interstitiel de l'utilisateur ; et
le montage d'une pluralité de composants électroniques (2418A) sur la partie ex vivo à l'aide d'un soudage photonique, au moins l'un de la pluralité de composants électroniques étant configuré pour recevoir les signaux générés associés au niveau d'analyte.

6. Procédé selon la revendication 5, dans lequel la fourniture du capteur d'analyte inclut l'impression de l'au moins une électrode de travail et de l'électrode de référence sur le substrat.

7. Procédé selon la revendication 6, comprenant en outre :
le masquage d'une partie du premier substrat avant le soudage photonique, et/ou
le revêtement du premier substrat avec un revêtement réfléchissant avant le soudage photonique, et/ou
la fourniture d'un vide pour empêcher le premier substrat de se déformer pendant le processus de soudage photonique.

8. Système (100) selon l'une quelconque des revendications 1 à 4 ou procédé selon les revendications 5 à 7, dans lequel la pluralité de composants électroniques (2418A) sont configurés en outre pour émettre les signaux associés au niveau d'analyte vers un dispositif à distance (120) ayant un écran d'affichage (122), facultativement dans lequel le dispositif à distance est au moins l'un dispositif de surveillance d'analyte portatif, un téléphone portable, ou un dispositif monté sur le poignet.

9. Système (100) selon l'une quelconque des revendications 1 à 4 et 8 ou procédé selon l'une quelconque des revendications 5 à 8, dans lequel le premier substrat est une unité monolithique flexible.

10. Système (100) selon l'une quelconque des revendications 1 à 4, 8 et 9 ou procédé selon l'une quelconque des revendications 5 à 9, dans lequel la pluralité de composants électroniques (2418A) comprennent un ou plusieurs processeurs et une batterie, facultativement, dans lequel la batterie inclut une batterie imprimée.

11. Système (100) ou procédé selon la revendication 10, dans lequel la pluralité de composants électroniques comprennent au moins une antenne.

12. Système (100) selon l'une quelconque des revendications 1 à 4 et 8 à 11 ou procédé selon les revendications 10 ou 11, dans lequel le capteur d'analyte comprend en outre un deuxième substrat ayant au moins une antenne, facultativement dans lequel l'au moins une antenne inclut une antenne Wi-Fi, une antenne NFC, une antenne Bluetooth, une antenne BTLE, ou une antenne GPS.

13. Système (100) selon l'une quelconque des revendications 1 à 4 et 8 à 12 ou procédé selon l'une quelconque des revendications 5 à 12, dans lequel le premier substrat est l'un d'un polyamide ou d'un poly(téréphtalate d'éthylène).

14. Système (100) selon l'une quelconque des revendications 1 à 4 et 8 à 13 ou procédé selon les revendications 5 à 13, dans lequel la partie ex vivo (4004) comprend une première couche.

15. Système (100) ou procédé selon la revendication 14, dans lequel la première couche comprend un mélange par gradient de matériaux, facultativement,
dans lequel le mélange par gradient de matériaux comprend de la fibre de verre et/ou
dans lequel le mélange par gradient de matériaux comprend approximativement 10 % de fibre de verre et la partie in vivo comprend du PET.

16. Système (100) ou procédé selon la revendication 14, dans lequel la partie ex vivo (4004) comprend une deuxième couche, facultativement dans lequel chacune de la première couche et de la deuxième couche comprend un mélange par gradient de matériaux.

17. Système (100) selon l'une quelconque des revendications 1 à 4 et 8 à 16, dans lequel le capteur d'analyte comprend en outre une membrane configurée pour réguler un flux entrant d'analyte disposée sur la partie in vivo.
